# EUROPEAN PATENT SPECIFICATION

(11) **EP 4 490 176 B1**
(45) Date of publication and mention of the grant of the patent: **30.09.2026**
(21) Application number: 23710892.3
(22) Date of filing: 10.03.2023
(51) Int. Cl.: C07K 14/82, A61P 3/10, A61P 9/00, A61P 19/02, A61P 25/28, A61P 35/00, A61P 37/00, A61K 38/00, C07K 19/00

(54) **C-JUN ANTAGONIST PEPTIDES**
C-JUN ANTAGONISTISCHE PEPTIDE
PEPTIDES ANTAGONISTES DE C-JUN

(30) Priority: 11.03.2022 GB 202203399
(43) Date of publication of application: 15.01.2025
(73) Proprietor: The University of Bath, Bath Somerset BA2 7AY (GB)
(72) Inventor: MASON, Jody Michael, Bath Somerset BA2 7AY (GB); BRENNAN, Andrew, Bath Somerset BA2 7AY (GB)
(74) Representative: Mewburn Ellis LLP
(86) International application number: PCT/EP2023/056243
(87) International publication number: WO 2023/170302

(56) References cited:
- WO-A1-2006/017913
- BAXTER DANIEL ET AL: "Exploiting Overlapping Advantages of In Vitro and In Cellulo Selection Systems to Isolate a Novel High-Affinity cJun Antagonist", ACS CHEMICAL BIOLOGY, vol. 12, no. 10, 20 October 2017 (2017-10-20), pages 2579 - 2588, XP093052321, ISSN: 1554-8929, DOI: 10.1021/acschembio.7b00693
- BAXTER D ET AL: "Supporting Information- Exploiting Overlapping Advantages of In Vitro and In Cellulo Selection Systems to Isolate a Novel High-Affinity cJun Antagonist", 20 October 2017 (2017-10-20), XP093052325, Retrieved from the Internet <URL:https://pubs.acs.org/doi/abs/10.1021/acschembio.7b00693> [retrieved on 20230606]
- NIKOLAEV YAROSLAV ET AL: "The Leucine Zipper Domains of the Transcription Factors GCN4 and c-Jun Have Ribonuclease Activity", PLOS ONE, vol. 5, no. 5, 21 May 2010 (2010-05-21), pages e10765, XP093051961, DOI: 10.1371/journal.pone.0010765
- BRENNAN ANDREW ET AL: "Selective antagonism of cJun for cancer therapy", JOURNAL OF EXPERIMENTAL AND CLINICAL CANCER RESEARCH, vol. 39, no. 1, 1 December 2020 (2020-12-01), IT, XP055802224, ISSN: 0392-9078, DOI: 10.1186/s13046-020-01686-9

## Description

### Field of the Invention

The present invention relates to peptides that antagonise c-Jun, nucleic acids encoding peptides that antagonise c-Jun, pharmaceutical preparations comprising peptides that antagonise c-Jun, and the use of the antagonist peptides in the treatment of c-Jun-mediated diseases.

### Background

Transcription factors (TFs) play crucial roles in the determination of cell function and fate. A range of upstream signals converge upon TFs, converting vital cell signalling processes into transcriptional outputs via specific DNA site recognition. Consequently, of the ~1600 TFs in the human genome, >300 are associated with a disease phenotype. TF dysfunction leads to a range of detrimental outcomes including cancer, diabetes, and cardiovascular disease (Lee et al., 2013; Lambert et al., 2018). Selective TF antagonism is therefore a compelling therapeutic route for the treatment of these diseases.

c-Jun is a transcription factor that is implicated in a range of human diseases (Eferl et al., 2003; Yung et al., 2010; Shiozawa et al., 2009). c-Jun is a member of the activator protein-1 (AP-1) family of dimeric transcription factors. AP-1 proteins bind to DNA recognition elements via their basic-leucine zipper (bZIP) domain which consists of a leucine zipper (LZ) to facilitate dimerisation and a DNA-binding domain (DBD) to facilitate DNA sequence recognition (Glover et al., 1995; Risse et al., 1989). c-Jun binds to 12-O-tetradecanoylphorbol-13-acetate response elements (TREs), directly influencing cellular processes such as differentiation, proliferation, and survival (Shaulian et al., 2001; Eferl et al., 2003; Eckert et al., 2013; Alani et al., 1991). Dysregulation of these functions therefore promotes hallmark cancer cell behaviour, rendering cJun a focal point for cancer therapy.

TF function relies on protein-protein interactions (PPIs) and protein-DNA interactions which form many points of contact over their large surfaces. Small molecules (SMs) typically fail to abrogate these types of interactions due to the lack of tractable pockets.

While the flat protein-protein interactions are inaccessible to many pharmaceuticals, including small molecules, peptides have the potential to excel as high-affinity and selective inhibitors when designed to complement the broad target surface. Various methodologies have produced peptide c-Jun antagonists that target the broad LZ binding interface (Boysen et al., 2002; Mason et al., 2006; Kaplan et al., 2014; Baxter et al., 2017; Lathbridge et al., 2018). However, it is difficult to predict if LZ binding will translate into functional antagonism as the cJun DBD remains unbound and capable of binding TRE DNA (Seldeen et al., 2008; Szalóki et al., 2015). A rationally designed peptide has been shown to target the c-Jun DBD but exhibits lower potency than LZ antagonists, with concerns over specificity due to high sequence similarity across the AP-1 family DBDs (Tsuchida et al., 2004).

One approach to circumvent the potential downsides of existing methods is to utilise longer peptides that target the full c-Jun bZIP domain with a selective yet high affinity interaction, simultaneously blocking both DNA binding and LZ dimerisation. Olive et al. took this approach to produce A-Fos, which combined the wild-type (WT) cFos LZ (known to heterodimerise with c-Jun) and a rationally designed Glu-rich acidic extension (Olive et al., 1997). The A-Fos design principle postulated that the LZ interaction is extended N-terminally generating a DBD-acidic extension interaction facilitated by the incorporation of Leu residues into putative d positions in the acidic extension.

For peptides to be able to act as functionally active c-Jun antagonists they must not only bind to c-Jun but target binding must also result in ablation of function. One recently described approach to identify functional antagonists of transcription factors is the Transcription Block Survival (TBS) assay described in WO2020128015.

There remains a need for c-Jun antagonists that act as functional antagonists, especially those that exhibit desirable pharmacokinetic properties for therapeutic use.

The present invention has been devised in light of the above considerations.

### Summary of the Invention

The present inventors have identified novel peptide inhibitors that are demonstrated to bind c-Jun and antagonise its DNA-binding function.

In work leading up to the present invention, the inventors used a library-based approach in which the hinge region that straddles the acidic extension and leucine zipper region of a c-Jun antagonist was semi-randomised. A peptide library was produced upon this scaffold and was randomised across a central tract of residues and tested using the TBS screening platform. This led to the identification of a recombinantly produced functional c-Jun antagonist termed 'HingeW', from a library of ~130,000 peptides. The HingeW peptide is demonstrated in the examples to bind to c-Jun preferentially and with a higher affinity compared to the c-Jun antagonist from which HingeW was derived, and effectively antagonises the c-Jun/TRE DNA interaction. The nature of the broad, shallow helical binding surface of c-Jun supports the use of longer peptides such as the one identified by TBS.

The binding epitope of bZIP antagonists is presented on one side of a single α-helix and as such target binding requires the peptide to adopt this secondary structure. Recognising that a fundamental step in the development of therapeutic peptides is downsizing of the peptide towards the smallest functional unit required for effective binding, the inventors introduced iterative truncations in the identified antagonist peptide. Downsizing tends to reduce the α-helicity of the peptide as both the interaction interface and extended internal hydrogen bonding network becomes reduced and water competes for these interactions, shifting the folding equilibrium towards a random coil. Downsizing peptides to increase drug-like characteristics such as stability and membrane permeability therefore needs to be balanced against reduced affinity resulting from a reduction in the α-helicity of the peptide. As demonstrated herein, various optimised and truncated forms of the HingeW peptide were developed that retain functional activity, while improving on the peptide's drug-like characteristics.

In a first aspect, the present invention provides a c-Jun antagonist comprising an extended hinge region having an amino acid sequence of LV[X₁]EE[X₂][X₃]LE[X₄]E (SEQ ID NO: 1); and a leucine zipper (LZ) region C-terminal to the extended hinge region,
wherein
X₁ is selected from V, D, K, C and R,
X₂ is selected from D, K, C and R,
X₃ is selected from V, D, C, K and R, and
X₄ is selected from E, D, C, K and R.

In some embodiments:
X₁ is selected from V, D, K and C,
X₂ is selected from D, K and C,
X₃ is selected from V, D and C, and
X₄ is selected from E, D and C.

In some embodiments, the extended hinge region further comprises an N-terminal acidic extension having an amino acid sequence of EA[X₅][X₆] (SEQ ID NO: 2),
wherein
X₅ is selected from E, K or C, and
X₆ is selected from E or D.

In some embodiments, the acidic extension has an amino acid sequence of EAEE (SEQ ID NO: 3).

In some embodiments, X₁ is V. In some embodiments, X₃ is V. In some embodiments, X₁ and X₃ are V.

In some embodiments:
(i) X₁ is V, X₂ is D, X₃ is V and X₄ is E;
(ii) X₁ is V, X₂ is K, X₃ is V and X₄ is D; or
(iii) X₁ is V, X₂ is C, X₃ is V and X₄ is C.

In some embodiments the LZ region comprises amino acid sequence
IEQLEERNYALR[X7]E[X8]K[X9]L[X10]D[X11] (SEQ ID NO: 29) or
IEQLEERNYALR[X7]E[X8]C[X9]L[X10]C[X11] (SEQ ID NO: 30) wherein
   [X7] is K, L, S, W, P, Q, R, M, T, V, A, E, or G;
   [X8] is I, V, or L;
   [X9] and [X10] are any amino acid residue.
   [X11] is Q, E, or K.

In some embodiments, the LZ region comprises an amino acid sequence selected from the group consisting of:
IEQLEERNYALRSEICSLQCQ (SEQ ID NO: 66); or
IEQLEERNYALRKEICELSCQ (SEQ ID NO: 67); or
IEQLEERNYALRAEICNLSCQ (SEQ ID NO: 68); or
IEQLEERNYALRTEICSLMCK (SEQ ID NO: 69); or
IEQLEERNYALRAEICSLQCQ (SEQ ID NO: 70),
or a variant thereof comprising 1, 2, or 3 amino acid modifications.

In some embodiments, the LZ region comprises an amino acid sequence selected from the group consisting of:
IEQLEERNYALRKEIEDLQKQ (SEQ ID NO: 4);
IEQLEEKNKALKDEIEDLQKQ (SEQ ID NO: 5);
IKQLEDRNYALRKEIEDLQKQ (SEQ ID NO: 6);
IEQLEERNYALRKEIKDLQDQ (SEQ ID NO: 7);
IEQLEEKNKALKDEIEDLY (SEQ ID NO: 8);
IEQLEERNYALRKEIEDLQ (SEQ ID NO: 9); and
IEQLEERNYALRKEICDLQCQ (SEQ ID NO: 27),or a variant thereof comprising 1, 2 or 3 amino acid modifications.

In some embodiments, the LZ region comprises an amino acid sequence of IEQLEERNYALRKEIKDLQDQ (SEQ ID NO: 7), or a variant thereof comprising 1, 2 or 3 amino acid modifications,
optionally wherein amino acid residues at positions corresponding to position 16 (position b in the heptad) and position 20 (position *f* in the heptad) of SEQ ID NO: 7 in the variant are K and D amino acid residues, respectively.

In some embodiments, the LZ region comprises an amino acid sequence of IEQLEERNYALRKEICDLQCQ (SEQ ID NO: 27), or a variant thereof comprising 1, 2 or 3 amino acid modifications,
optionally wherein amino acid residues at positions corresponding to position 16 (position *b* in the heptad) and position 20 (position *f* in the heptad) of SEQ ID NO: 27 in the variant are both C amino acid residues.

In some embodiments, the c-Jun antagonist has a length of between 30 and 70 amino acids, between 30 and 60 amino acids, between 30 and 50 amino acids, or between 30 and 40 amino acids. In particular embodiments, the c-Jun antagonists have a length of 36 amino acids.

In some embodiments, the c-Jun antagonist has the amino acid sequence of
EAEELVVEEDVLEEEIEQLEERNYALRKEIKDLQDQ (SEQ ID NO:10);
EAEELVVEEKVLEDEIEQLEERNYALRKEIKDLQDQ (SEQ ID NO: 11); or
EAEELVVEEDVLEEEIEQLEERNYALRKEICDLQCQ (SEQ ID NO: 28); or
EAEELVVEEDVLEEEIEQLEERNYALRSEICSLQCQ (SEQ ID NO: 37); or
EAEELVVEEDVLEEEIEQLEERNYALRKEICELSCQ (SEQ ID NO: 38); or
EAEELVVEEDVLEEEIEQLEERNYALRAEICNLSCQ (SEQ ID NO: 39); or
EAEELVVEEDVLEEEIEQLEERNYALRTEICSLMCK (SEQ ID NO: 40); or
EAEELVVEEDVLEEEIEQLEERNYALRAEICSLQCQ (SEQ ID NO: 41)., or a variant thereof
comprising 1, 2 or 3 amino acid modifications.

In some embodiments, the c-Jun antagonist comprises at least one covalent amino acid residue cross-linker. Such peptides may be referred to herein as 'helix constrained c-Jun antagonists'. In some embodiments, the c-Jun antagonist peptide comprises at least one covalent *i to i+4* or *i to i+7* amino acid residue cross-linker. As demonstrated herein, introducing a covalent amino acid residue cross-linker increases helicity and can increase antagonist activity of the peptide. This is beneficial, as it can be used to derive functionally active peptide antagonists that have similar binding affinity and function as parental proteins, with the same amino acid sequences that confer specificity, while retaining stability and solubility akin to small molecule therapeutics. While this is demonstrated for K to D lactam bridge as cross-linkers, similar results are expected if other cross-linkers are used, such as alkyl cross-links formed between two C residues via cystine alkylation, such as DBMB.

In some embodiments, the c-Jun antagonist peptide comprises at least one covalent *i to i+ 4* amino acid residue cross-linker. For example, the c-Jun antagonist peptide may comprise two covalent *i to i+4* amino acid cross-linkers. Preferably, the covalent *i to i+4* cross-linker(s) are present at heptad locations b-to-f or f-to-c. Preferably, the covalent *i to i+4* amino acid cross-linker(s) are K to D lactam bridge(s), or an alkyl cross-link formed between two C residues (cysteine alkylation).

In a second aspect, the invention provides a nucleic acid encoding the c-Jun antagonist peptide according to the first aspect of the invention.

In a third aspect, the invention provides a conjugate comprising the c-Jun antagonist peptide according to the first aspect of the invention conjugated to a lipid, a polymer, or a second peptide.

In a fourth aspect, the invention provides a pharmaceutical composition comprising the c-Jun antagonist according to the first aspect of the invention, nucleic acid according to the second aspect of the invention, or conjugate according to the third aspect of the invention in combination with a physiologically acceptable vehicle or carrier.

In a fifth aspect, the invention provides the c-Jun antagonist peptide according to the first aspect of the invention, a nucleic acid according to the second aspect of the invention, conjugate according to the third aspect of the invention, or a pharmaceutical composition according to the fourth aspect of the invention for use as a medicament.

In a sixth aspect, the invention provides a method of inhibiting c-Jun comprising a peptide according to the first aspect of the invention, a nucleic acid according to the second aspect of the invention, or conjugate according to the third aspect of the invention, *in vitro* to a cell comprising or expressing c-Jun peptide.

Also provided herein are methods of producing the c-Jun antagonist peptide according to the first aspect of the invention. A method of producing a c-Jun antagonist peptide may comprise synthesising the c-Jun antagonist peptide using solid or liquid phase peptide synthesis, or may comprise producing the c-Jun antagonist peptide by recombinant expression. The method may further comprise contacting the c-Jun antagonist peptide with a cross-linker to produce a helix constrained c-Jun antagonist peptide. Additionally provided herein are methods of producing a helix constrained c-Jun antagonist peptide, comprising contacting the c-Jun antagonist peptide according to the first aspect of the invention with a cross-linker.

The invention includes the combination of the aspects and preferred features described except where such a combination is clearly impermissible or expressly avoided.

These and other aspects of the invention are described in more detail below.

### Summary of the Figures

Embodiments and experiments illustrating the principles of the invention will now be discussed with reference to the accompanying figures in which:

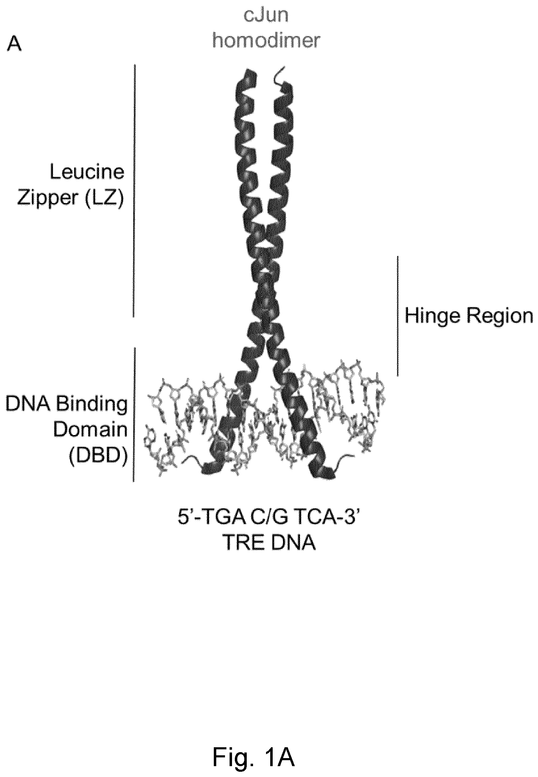

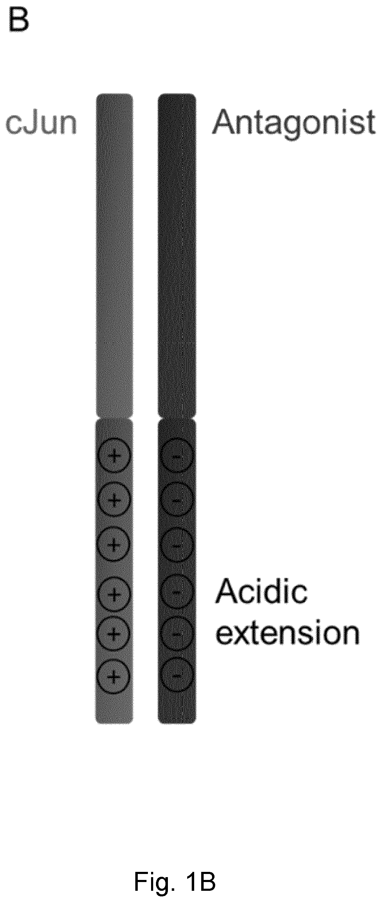

**Figure 1** **- TRE DNA-bound cJun structure and cJun antagonist design. (A)** The DNA bound-cJun homodimer crystal structure (PDB: 2H7H) is shown to highlight LZ and DBD components required for 6imerization and DNA binding. **(B)** Schematic illustrating the acidic extension design principle (A-FosW, HingeW). This utilises a region known to bind to the cJun LZ, to which a Glu-rich, extension is appended to interact with the cJun DBD.

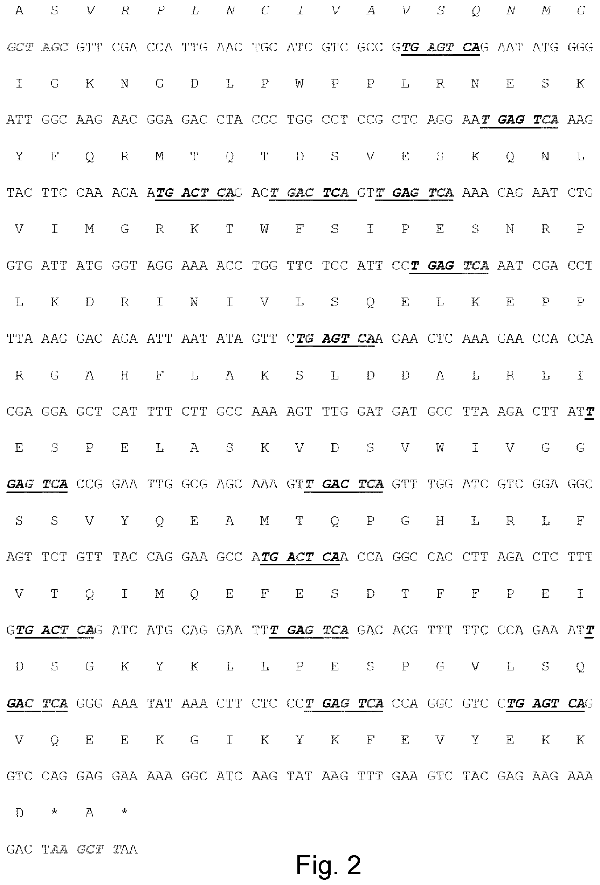

**Figure 2** - **Protein and DNA sequence of TRE-mDHFR showing the introduction of 15 TRE sites into the gene.** Amino acids and DNA bases mutated from the WT murine protein are shown in red, and the TRE DNA sites these mutations have added are emboldened and underlined. Shown in green are the Nhel and Hindlll sites used for subcloning the gene into the pES300d vector.

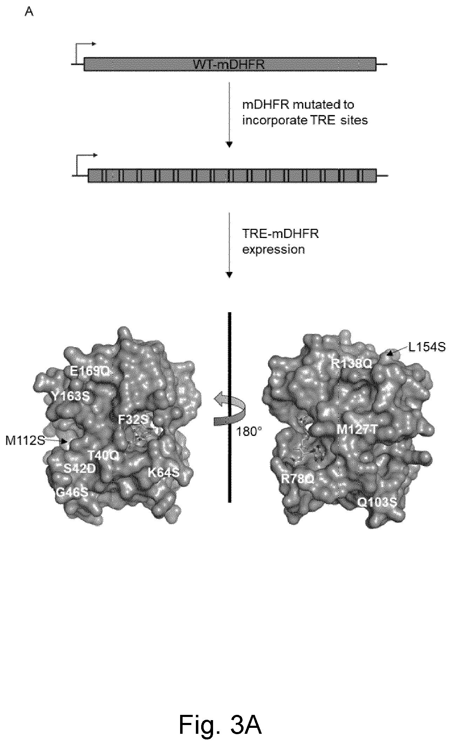

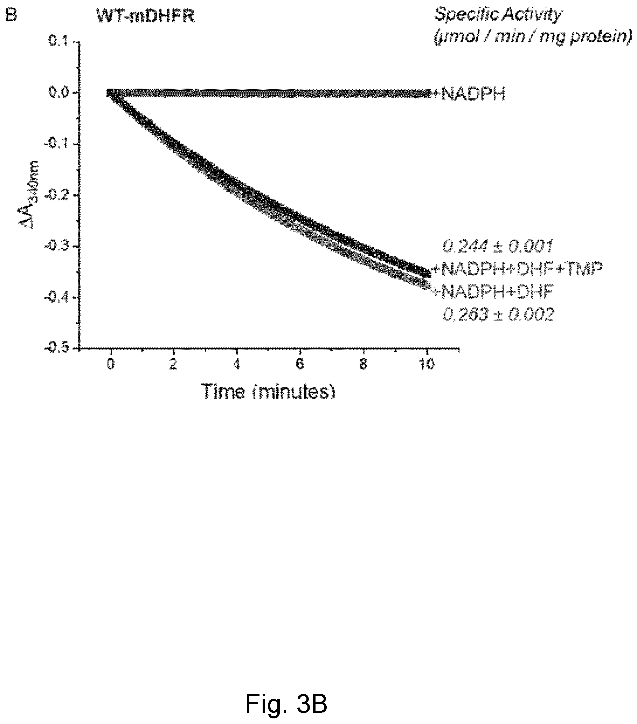

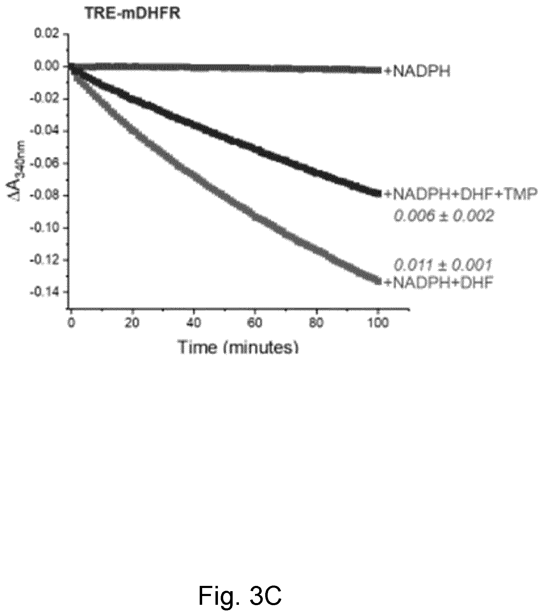

**Figure 3** - **mDHFR retains activity upon introduction of TRE sites. (A)** Fifteen TRE sites were introduced into the mDHFR gene (two silent and thirteen substitutions) to allow for a cJun-induced transcriptional block. Substitutions are mapped (green) on the mDHFR structure (PDB code: 1U72) demonstrating surface exposure at positions distal from the active site where the substrate DHF (shown is competitive inhibitor methotrexate (MTX) bound in the DHF binding site) and cofactor NADPH are bound. Change in absorbance at 340 nm was measured to determine the rate of NADPH turnover by **(B)** WT-mDHFR and **(C)** TRE-mDHFR with or without the substrate DHF. Also shown are reactions repeated in the presence of TMP, demonstrating that activity is retained for both enzymes, with TRE-mDHFR partially inhibited as expected. Specific activity was calculated from the linear initial rate (first 2.5 minutes for WT-mDHFR, first 10 minutes for TRE-mDHFR; +NADPH only reaction blank subtracted). Data are averages from triplicate experiments with errors shown as one standard deviation. MTX exhibits broader inhibition than TMP, inhibiting both eukaryotic and prokaryotic DHFR enzymes, and therefore inhibited both WT- and TRE-mDHFR.

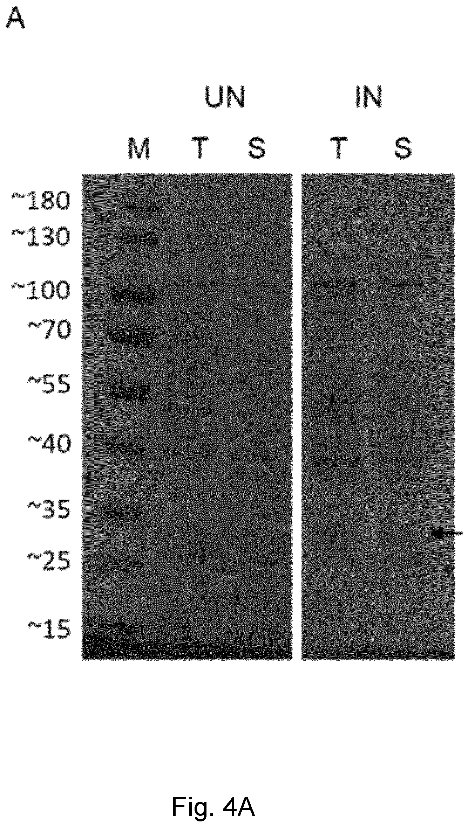

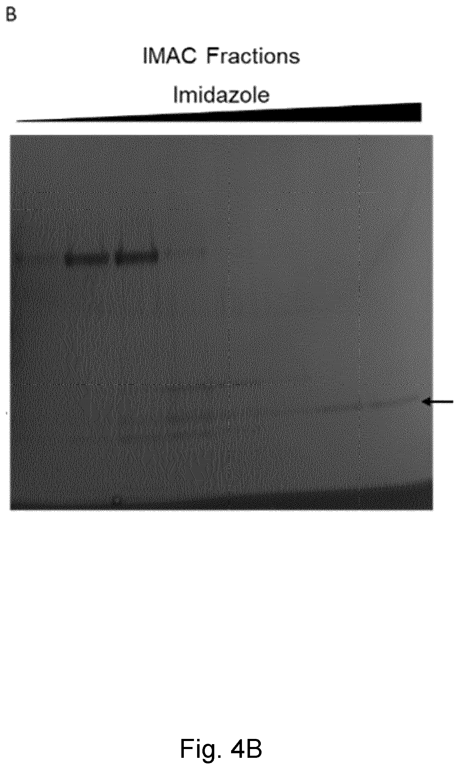

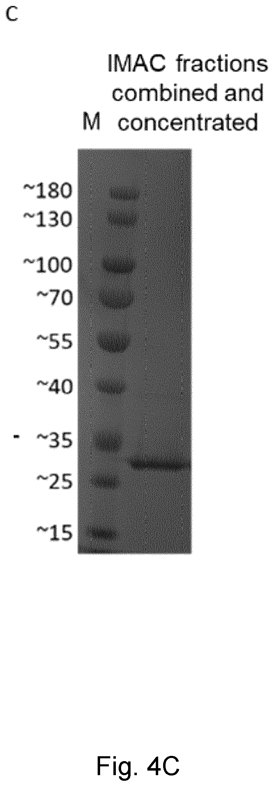

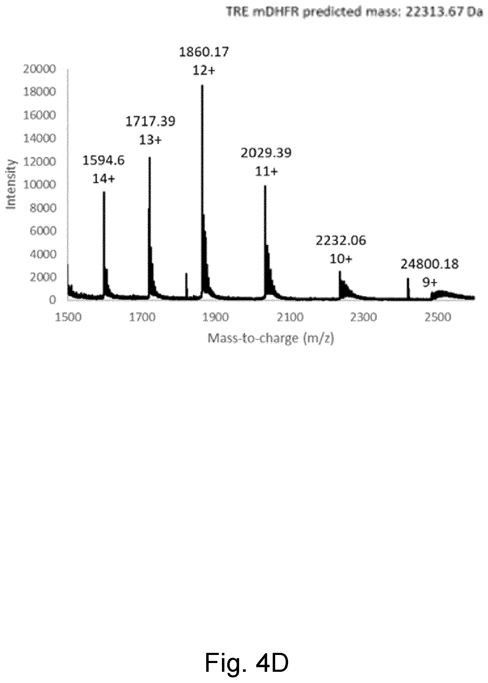

**Figure 4** - **TRE-mDHFR is expressed in the soluble fraction and can be purified for further study. (A)** SDS-PAGE analysis of *E*. *coli* cell lysate from cells before and after the induction of TRE-mDHFR plasmid expression with IPTG (1 mM, 18 hours, 30°C). A total (T) sample is taken directly after lysis and a soluble (S) sample is taken after the lysate is centrifuged. This shows the appearance of a protein band in the induced samples with equal band intensity in the T and S fractions, indicating the protein is folded and soluble. TRE-mDHFR can be bound to an immobilised metal affinity chromatography column due to its 6xHis-tag and subsequently eluted by an imidazole gradient **(B)** to give pure protein as determined by **(C)** SDS-PAGE of the combined and concentrated fractions shown to contain the induced protein band. TRE-mDHFR did not migrate through the polyacrylamide gel as predicted by the protein marker lane (M), running at an apparently higher molecular weight but its identity was confirmed by **(D)** electrospray ionisation mass spectrometry.

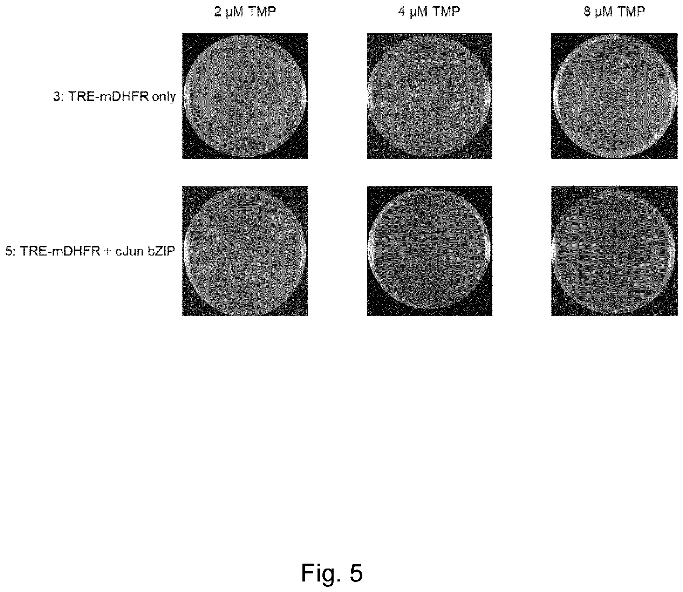

**Figure 5** - **Optimisation of TMP concentration required to produce selectivity between *E. coli* expressing TRE-mDHFR and *E. coli* with TRE-mDHFR expression transcriptionally-blocked by cJun bZIP.** Controlled numbers of *E*. *coli* cells expressing the indicated proteins were plated on selective media at varying TMP concentration. 4 µM TMP produces the optimum differential in colony numbers between the TRE-mDHFR only and TRE-mDHFR + cJun bZIP plates.

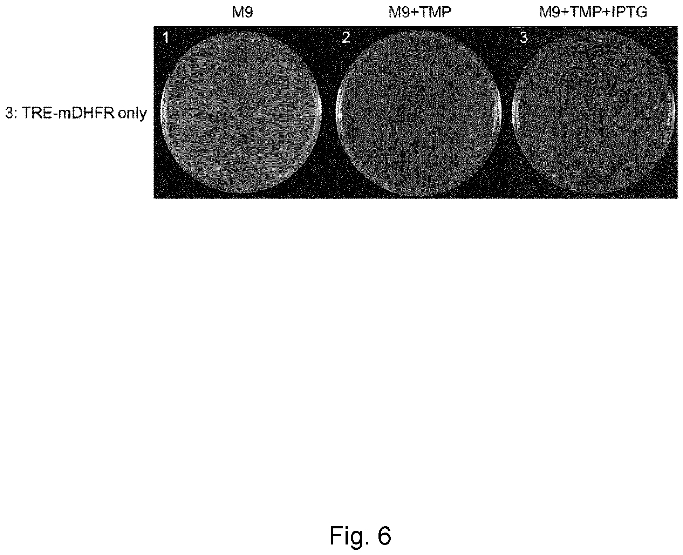

**Figure 6** - **Bacterial DHFR can be inhibited by TMP and have its activity replaced by the induction of TRE-mDHFR expression.** *E*. *coli* cells containing the plasmid for TRE-mDHFR only grow differentially on different agar media, following the design principles of the TBS assay. In M9 agar (1), a lawn of colonies is produced as the bacteria grow freely; upon addition of TMP (2) to the media the bacterial DHFR is inhibited and cells cannot grow; and further addition of IPTG (3) leads to expression of the TRE-mDHFR which restores cell survival to a degree.

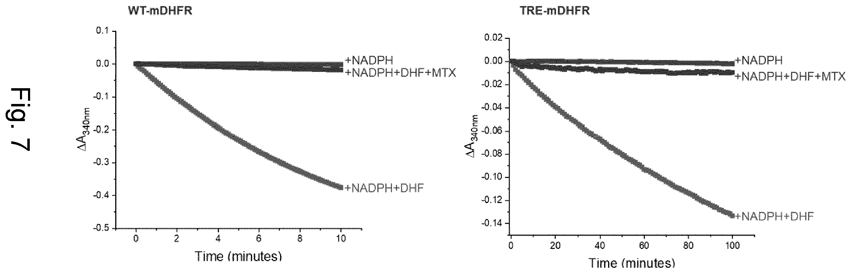

**Figure 7** - **Both WT- and TRE-mDHFR are inhibited by the broad DHFR inhibitor MTX.** The change in absorbance at 340 nm was measured to determine the rate of NADPH turnover by WT-mDHFR and TRE-mDHFR, with and without the substrate DHF. Also shown are the reactions repeated in the presence of MTX which shows clear inhibition of the reaction, as expected and indicative of DHFR activity.

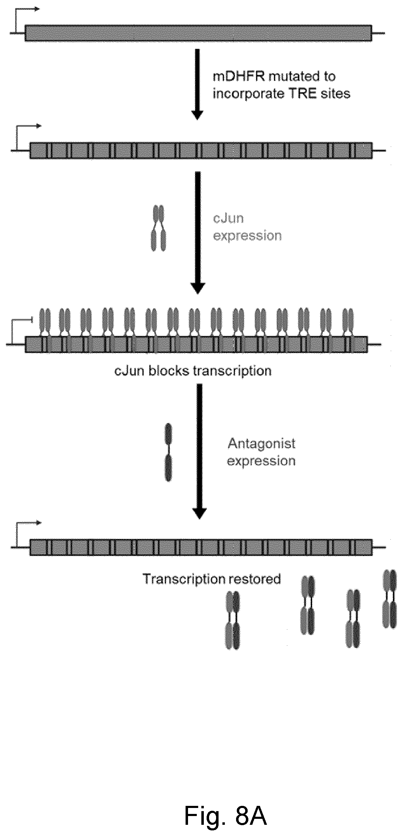

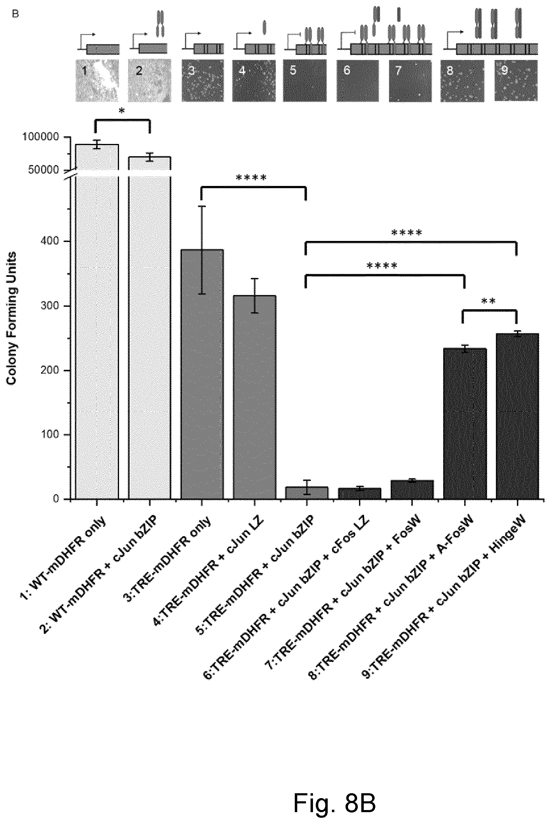

**Figure 8** **- Transcription Block Survival (TBS) assay to derive functionally active cJun inhibitors. (A)** Schematic illustrating the design and operation of TBS. **(B)** Controlled numbers of E. coli expressing the indicated proteins were plated on selective media and growth rates were calculated by counting colony forming units. **(1)** WT-mDHFR expression can replace ecDHFR and is uninhibited by TMP producing significant growth. **(2)** A small effect on colony numbers is observed when cJun bZIP is additionally expressed. **(3)** TRE-mDHFR can replace the inhibited ecDHFR with colony count lower than for WT as expected. **(4)** The cJun LZ domain (lacking DBD) does not affect TRE-mDHFR transcription and colony formation, however, **(5)** the cJun bZIP domain (with DBD) binds TRE sites to block transcription of TRE-mDHFR leading to reduced bacterial survival. Although **(6)** cFos LZ and (7) FosW are known cJun-binders, they are unable to effectively dissociate the cJun bZIP from TRE DNA. However, **(8)** A-FosW and the TBS-derived hit **(9)** HingeW remove TRE-mDHFR transcriptional blocks to restore cell survival. Bar charts represent averages of three experimental repeats. Errors are shown as one standard deviation. Selected P values from a t-test are indicated (* P ≤ 0.05; ** P ≤ 0.01; **** P ≤ 0.0001) with values for all possible comparisons within the bar chart reported in Figure S7. Serial dilutions were used to quantify colony numbers where required. Also shown are representative plate images and schematics to illustrate the effect upon TRE-mDHFR transcription.

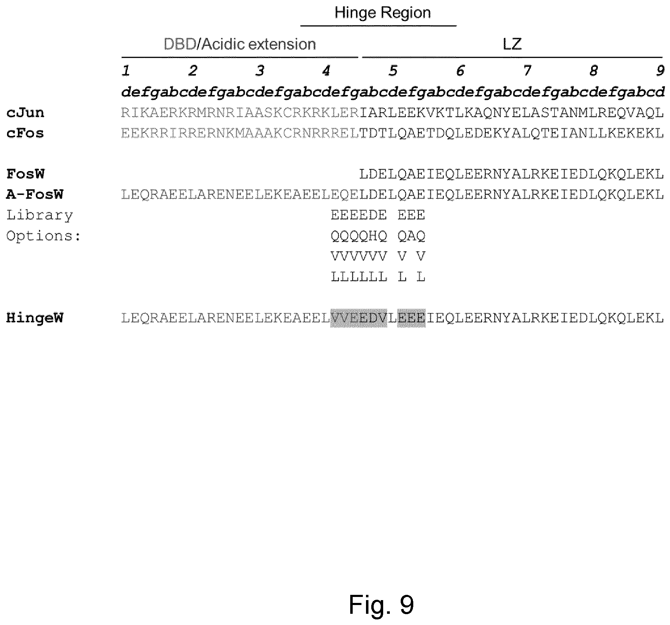

**Figure 9** **- Target and antagonist peptide sequences, and TBS library design.** The cJun target sequence is shown and compared to related off-target cFos. To facilitate optimisation of cJun binding, nine residues within a ten-residue tract **(e4** to **g5)** in the A-FosW sequence were selected for variation within the library, providing acidic, polar and hydrophobic options, resulting in a 131,072-member library. Screening using TBS produced the 'HingeW' sequence. DBD and acidic extension regions are shown in blue or red respectively, with the selected library options in the winner peptide highlighted in green. Residues are named according to the heptad numbering and position within given heptad repeat.

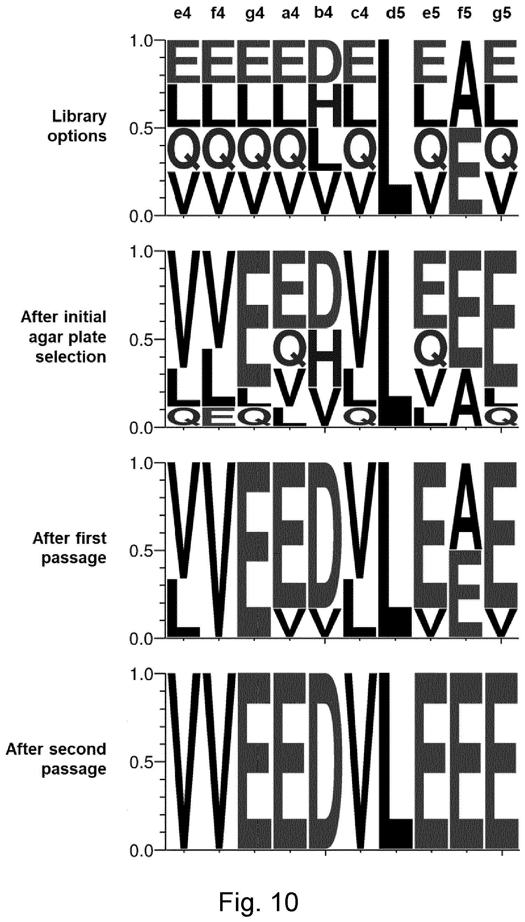

**Figure 10** **- TBS selection pressure shifts the representation of library members in the DNA pool sequencing towards the selection of HingeW as an assay winning sequence.** Sequence logos showing the relative abundances of the amino acids on the initial selection plate (nine colonies sequenced), the first passage (six colonies sequenced) and the final winning sequence (HingeW; only sequence present in the DNA pool and in five colonies).

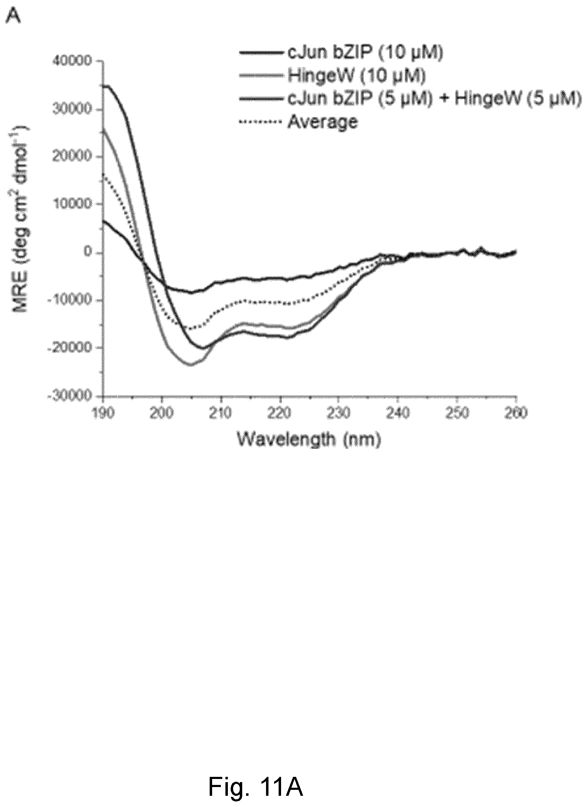

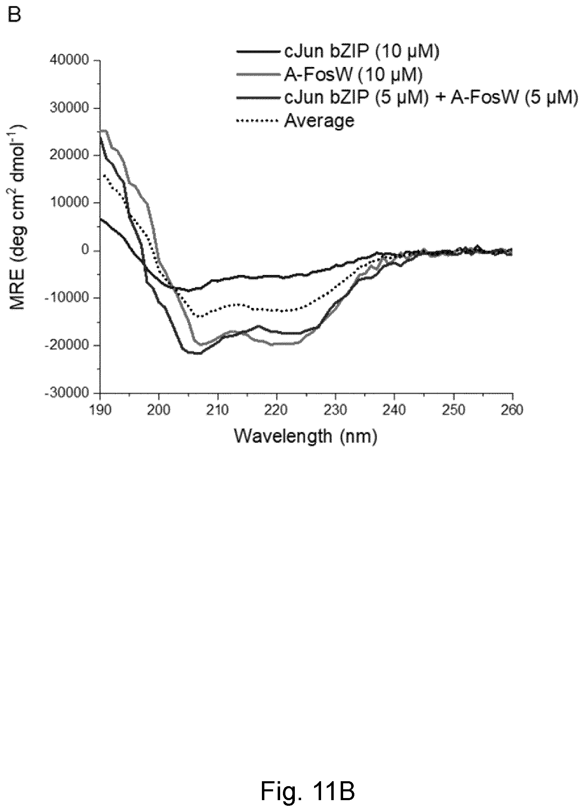

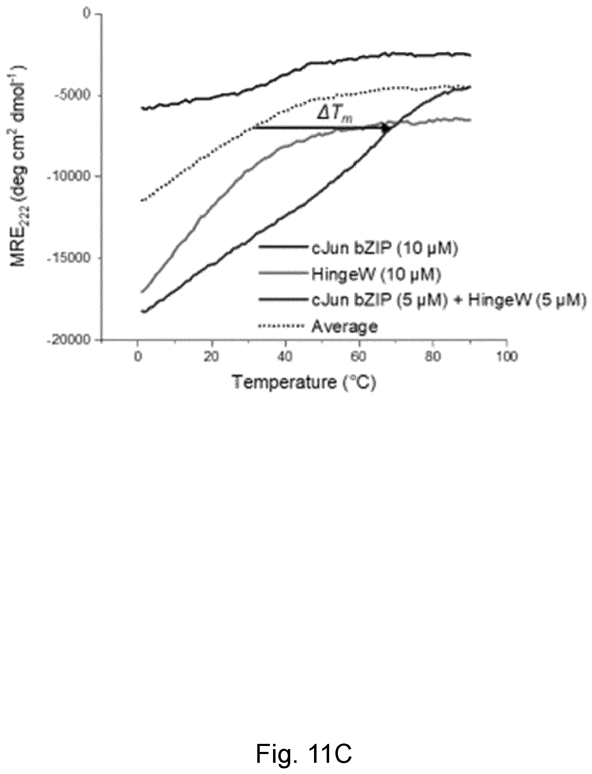

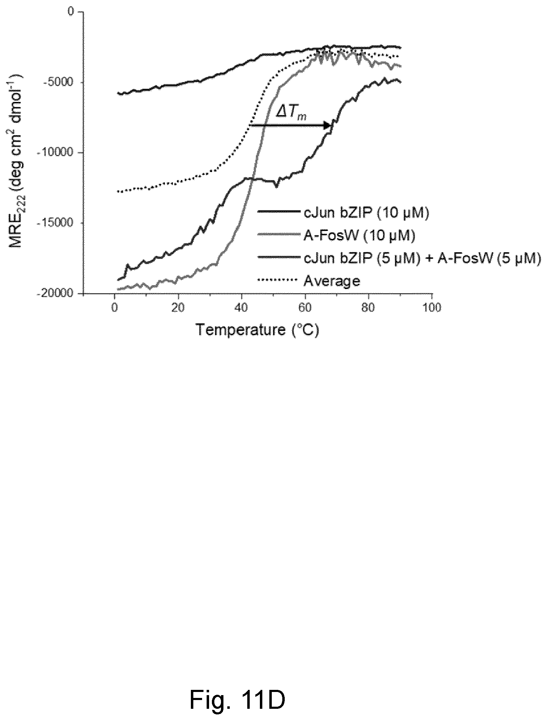

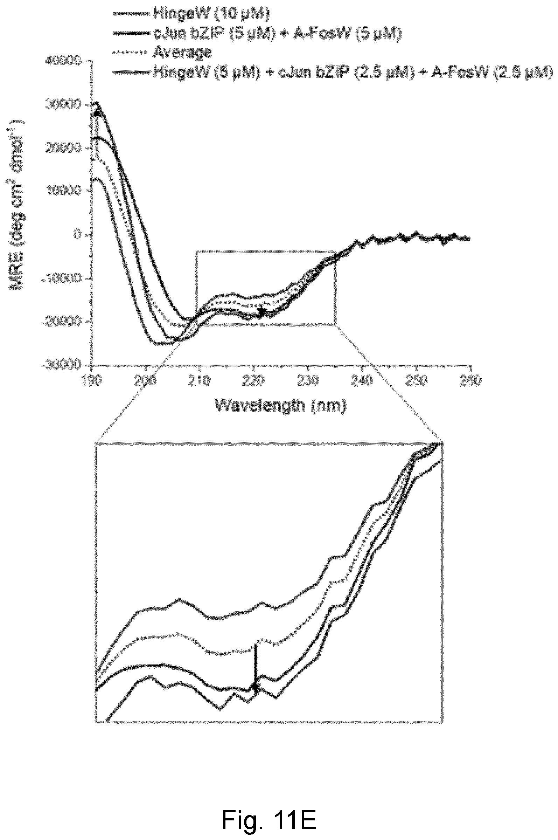

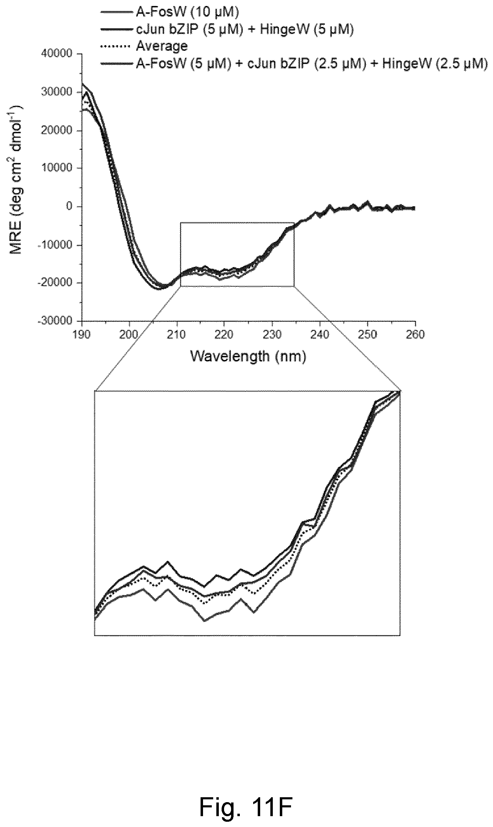

**Figure 11** **- TBS winner peptide HingeW binds cJun preferentially over A-FosW.** CD spectra (20°C) show binding of cJun to either **(A)** HingeW or **(B)** A-FosW. In both cases the heterodimeric spectrum shows increased α-helical character relative to the average of the component peptides. However, the effect is larger for HingeW, indicating a greater increase in peptide helicity. Similarly, the thermal denaturation of cJun bound to either **(C)** HingeW or **(D)** A-FosW is right-shifted from the average of the component peptide denaturation profiles. HingeW/cJun displays a larger ΔTₘ of binding than A-FosW/cJun due to the lower Tₘ of the HingeW homodimer (indicated by arrows). CD dimer exchange spectra show **(E)** an increase in helicity when HingeW is mixed with the A-FosW/cJun heterodimer as the HingeW exchanges with the A-FosW due to the binding preference of cJun for HingeW and **(F)** no shift from the average is observed when A-FosW is mixed with the HingeW/cJun heterodimer, indicating no change in dimer populations. Arrows are shown to highlight the shift from the average at 190 and 222 nm. In all experiments, the total sample peptide concentration was fixed to 10 µM using equimolar concentrations of each component peptide to remove concentration dependent effects.

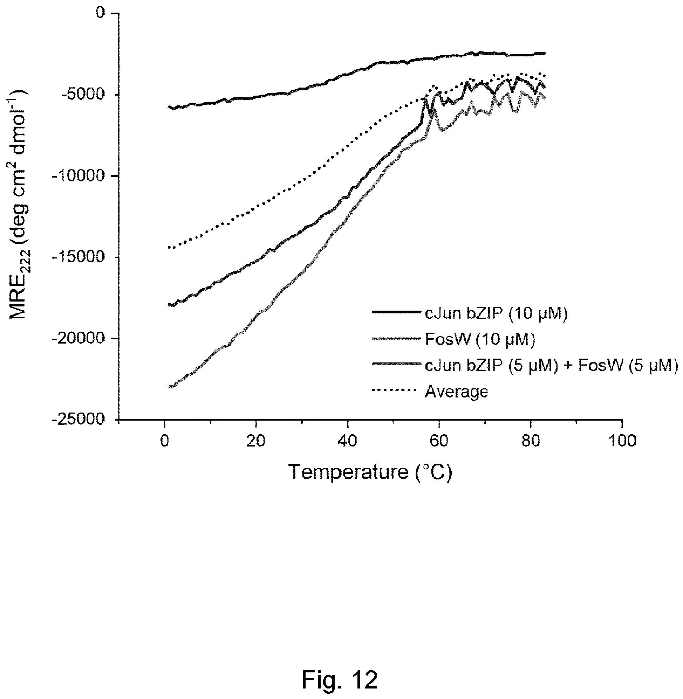

**Figure 12** - **CD thermal denaturation profiles showing the interaction of FosW with the cJun bZIP.** The thermal denaturation profile of the FosW/cJun bZIP heterodimer is shifted from the average of the two component peptide curves. This shows an increased helicity and *Tₘ* value (54°C for the heterodimer), indicative of a binding interaction.

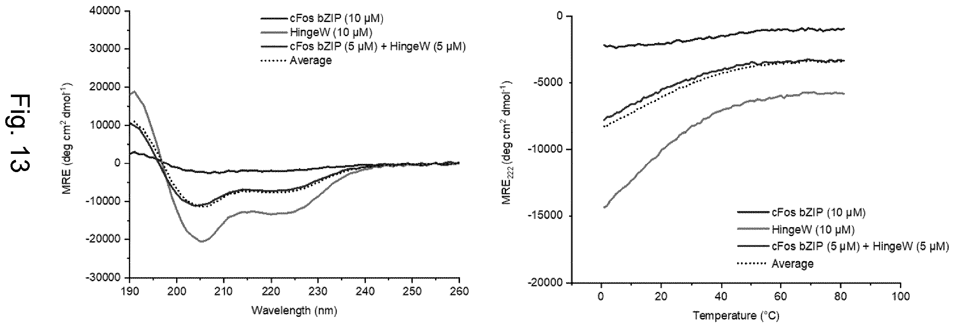

**Figure 13** - **TBS winner peptide HingeW does not interact with cFos.** CD spectra and thermal denaturation curves showing no interaction between HingeW and cFos as the measured heterodimer spectrum/thermal denaturation curve overlays with the average of the individual component spectra.

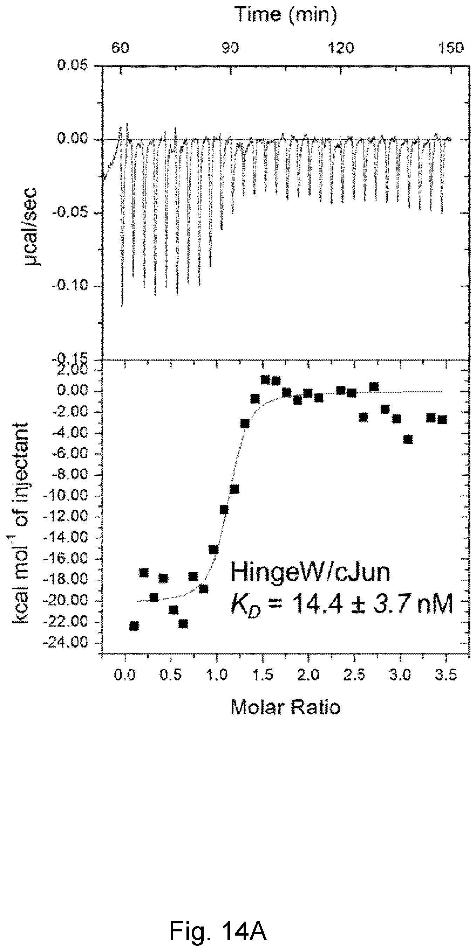

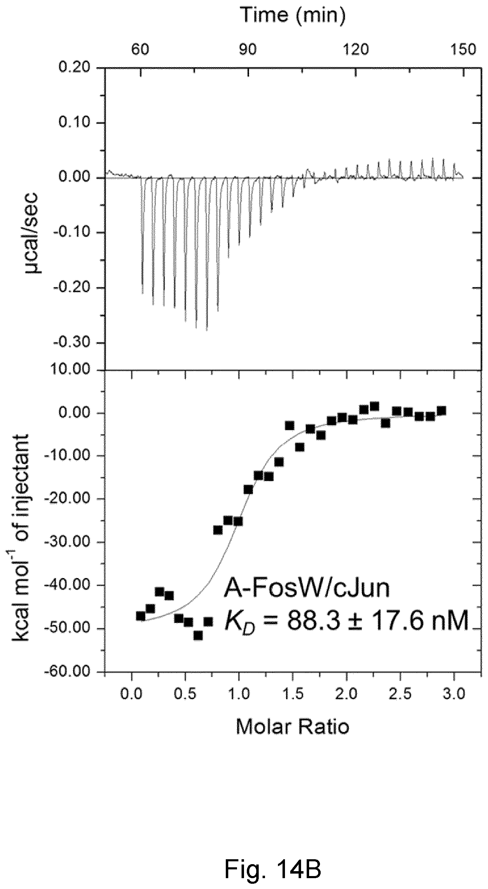

**Figure 14** - **Isothermal titration calorimetry data demonstrate a six-fold higher affinity for HingeW/cJun relative to A-FosW/cJun.** ITC analysis profiles for cJun binding to **(A)** HingeW and **(B)** A-FosW show the raw power compensation plot throughout the titration in the upper graph and the integrated data points and single site model fit (MicroCal ORIGIN software) in the lower graph.

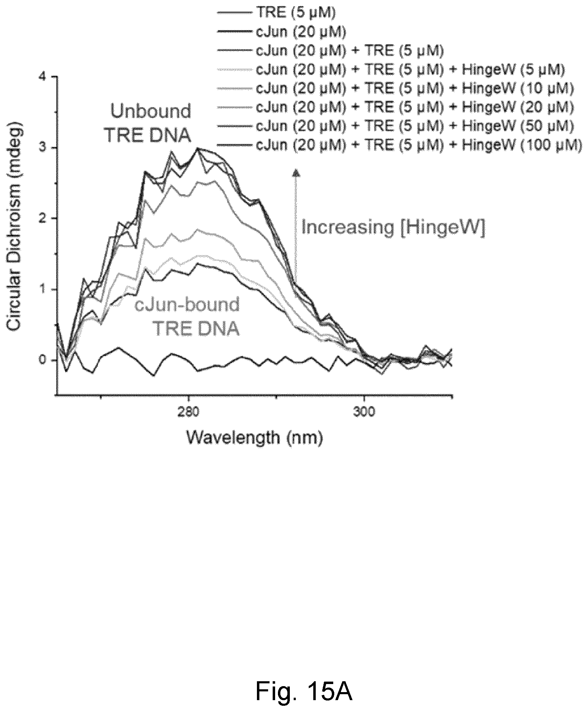

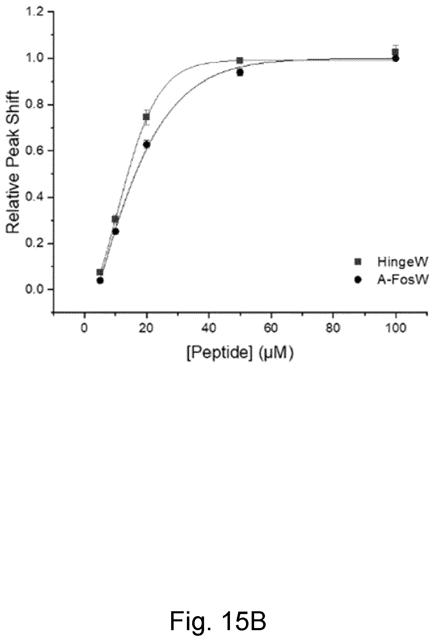

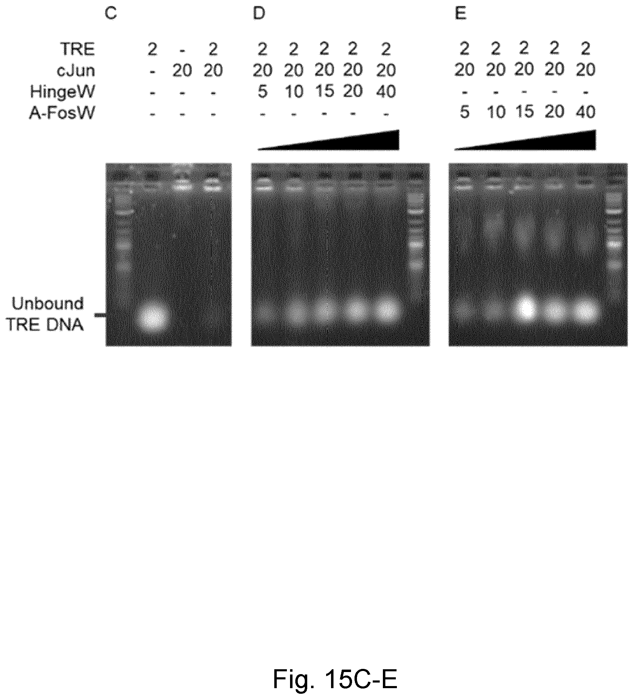

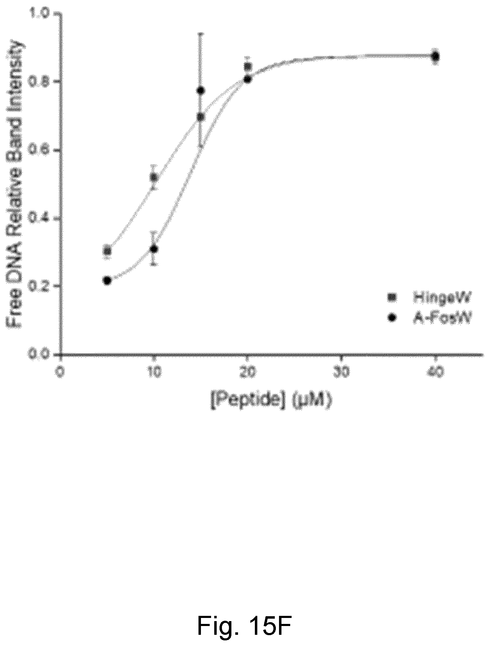

**Figure 15** - **HingeW antagonises cJun/TRE DNA interaction more effectively than A-FosW. (A)** CD spectra showing a shift in the TRE DNA peak at ~281 nm upon addition of cJun which is reversed by the titration of HingeW into the sample, as HingeW sequesters the cJun in a non-functional heterodimer. **(B)** The relative peak shift from bound to free TRE is plotted for varying concentrations of HingeW and A-FosW showing greater cJun/TRE DNA inhibition for HingeW across all concentrations. EMSA showing the **(C)** unbound TRE DNA band shift upon addition of cJun and the subsequent restoration of the unbound DNA band intensity upon titration of either **(D)** HingeW or **(E)** A-FosW. **(F).** For both CD and EMSA, data was averaged from three independent experiments and the plotted error bars indicate one standard deviation.

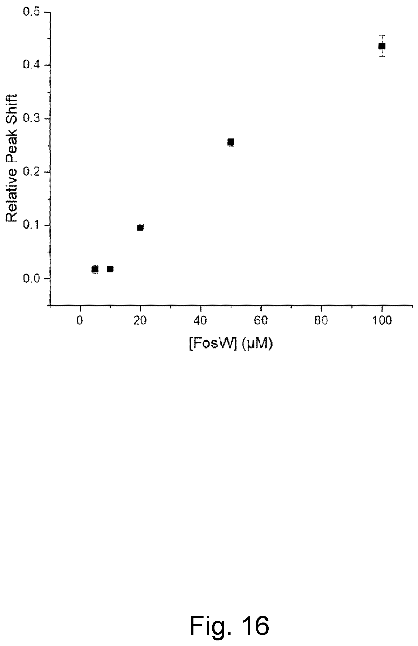

**Figure 16** - **CD antagonism data showing the shift in the DNA spectrum upon addition of FosW to the cJun-bound DNA.** The relative shift from the cJun-bound TRE DNA peak to the free TRE DNA peak is monitored at 281 nm as FosW is sequentially added. Data averaged from three independent experiments.

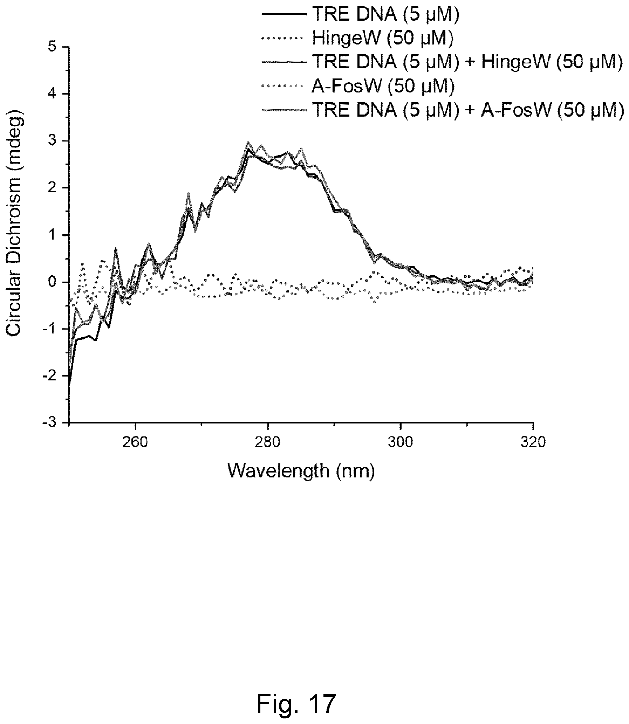

**Figure 17** - **HingeW and A-FosW do not interact with TRE DNA.** CD spectra showing no interaction between either HingeW or A-FosW with TRE DNA. Proteins do not absorb in this wavelength range, so the CD signal observed occurs due to the DNA structure, which is not perturbed upon addition of either protein.

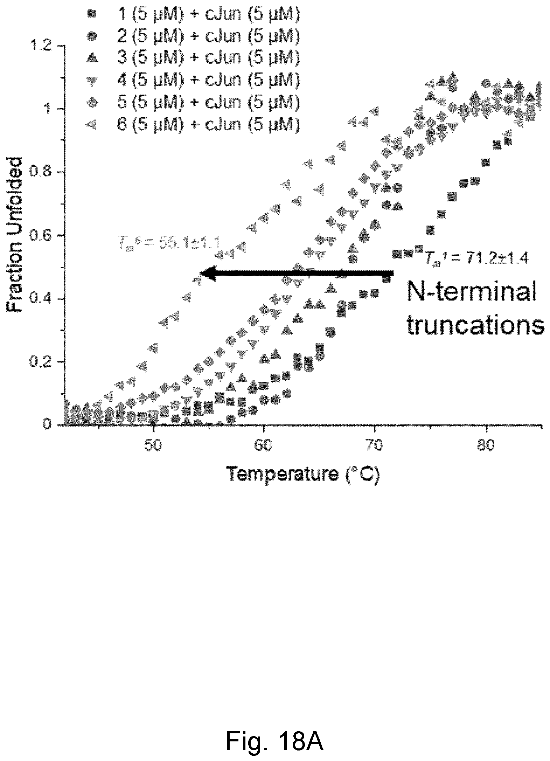

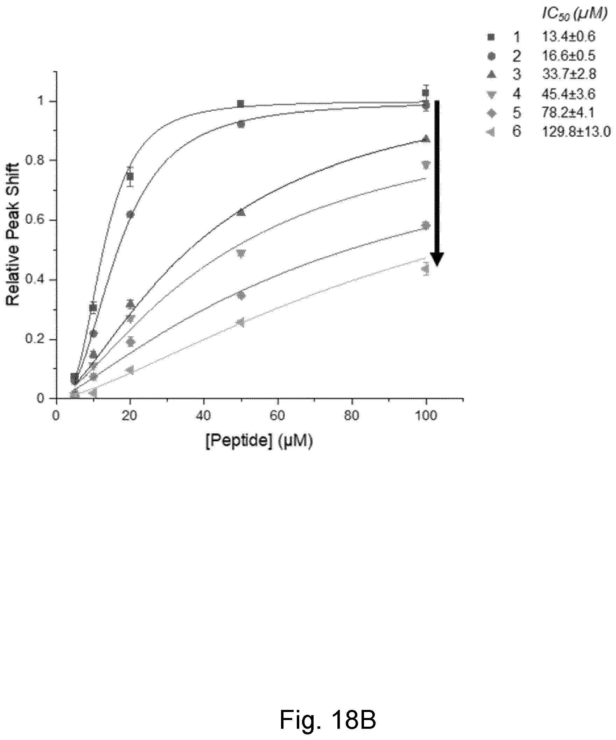

**Figure 18** - **Iterative N-terminal truncation of HingeW reduces c-Jun binding and antagonism. (A)** Thermal denaturation profiles for iteratively truncated peptide (5 µM)/c-Jun (5 µM) heterodimer samples. **(B)** CD antagonism data produced by monitoring a shift in a DNA specific peak, to provide a direct readout of cJun-induced DNA binding.

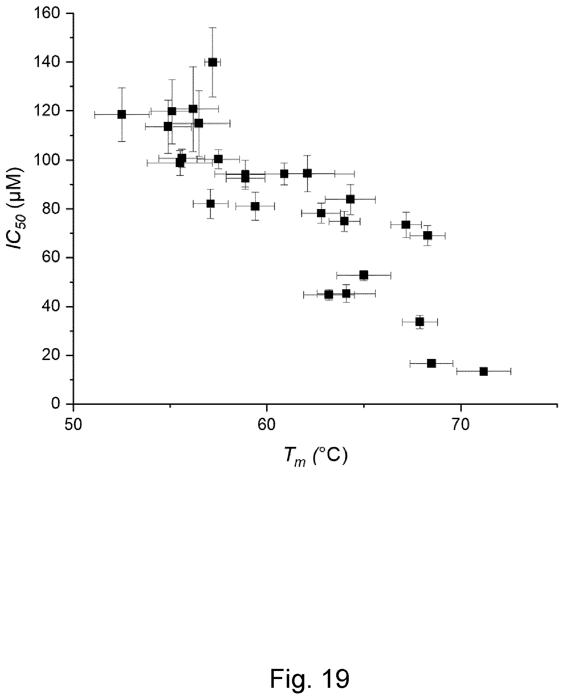

**Figure 19** - ***Tₘ* values broadly correlate with *IC₅₀* values.** As the peptide-cJun heterodimer (i.e the formation of a binary complex) *Tₘ* increases a direct correlation is observed with improved cJun/TRE DNA antagonism (antagonism of DNA binding in which formation of the ternary complex is blocked) as indicated by the lower *IC₅₀* value.

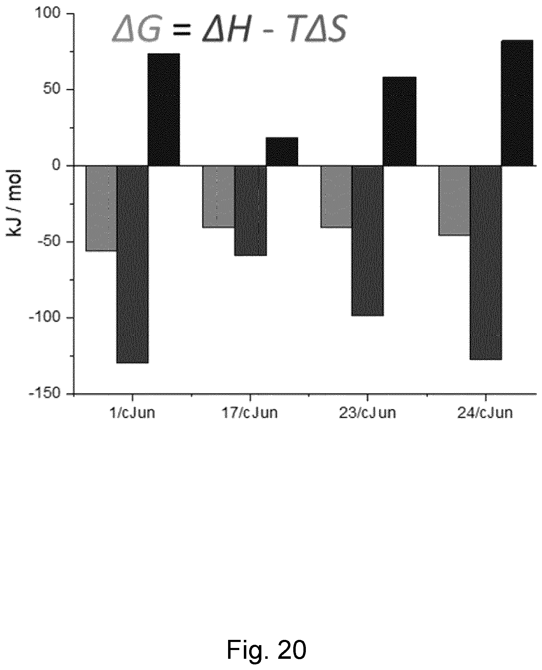

**Figure 20** **- ITC data showing thermodynamic parameters c-Jun peptide interactions**

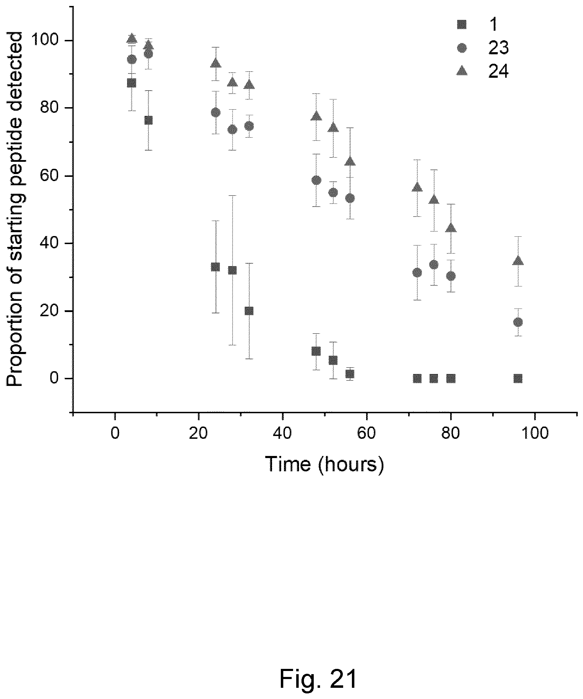

**Figure 21** - **Lactamisation results in enhanced serum stability.** The amount of peptide detected by LC-MS is plotted compared to the starting point and shows that linear peptides degrade faster than lactamised, with the double lactamised 24 showing the highest stability.

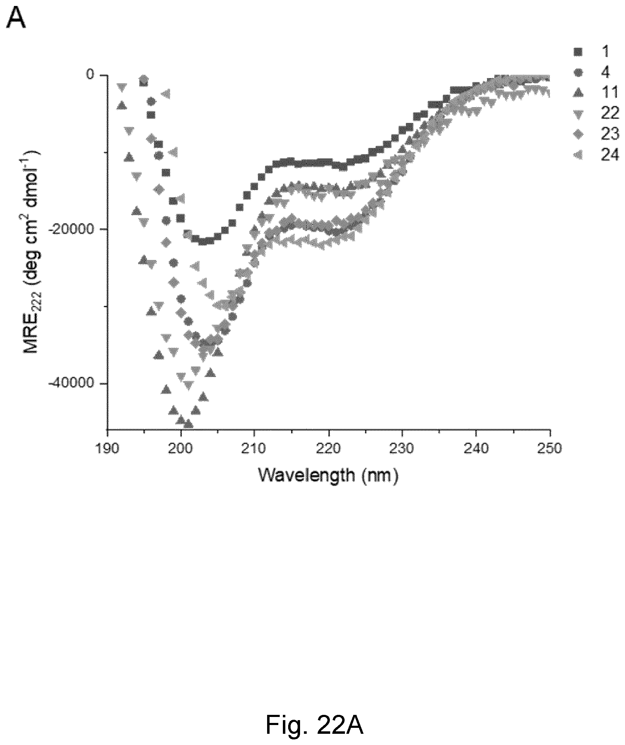

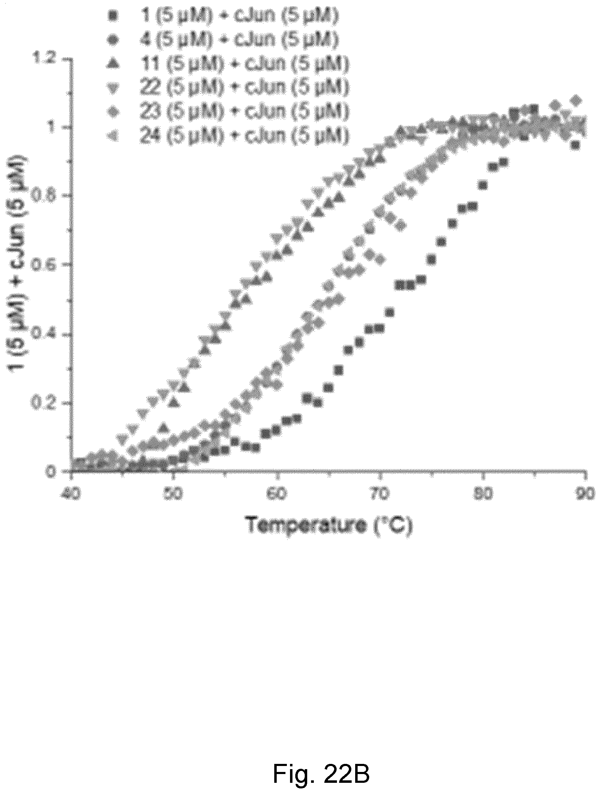

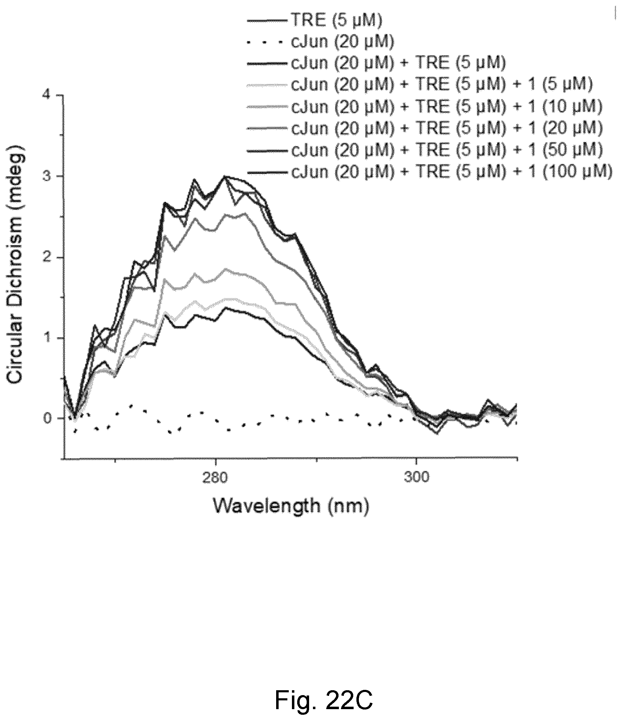

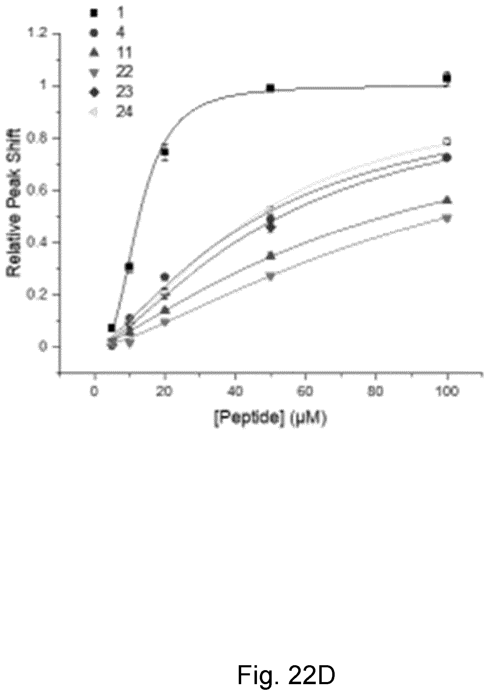

**Figure 22** - **Peptide optimisation quantified by CD to determine peptide helicity, c-Jun target binding and c-Jun/TRE DNA antagonism.** The biophysical characterisation of selected peptides are shown to illustrate exemplar data and the effects observed throughout the optimisation process. **(A)** Spectra of selected peptides (10 µM) which shows the increase in helicity from truncating at the N-terminus, the decrease in helicity from truncating at the C-terminus and the increase in helicity due to lactamisation. **(B)** Thermal denaturation profiles for selected antagonist (5 µM)/cJun (5 µM) heterodimer samples. **(C)** CD spectra showing a shift in the TRE DNA peak at ~281 nm upon addition of cJun which is reversed by the titration of HingeW into the sample, as HingeW sequesters the cJun in a non-functional heterodimer. **(D)** The relative peak shift from bound to free TRE is plotted for varying peptide concentrations.

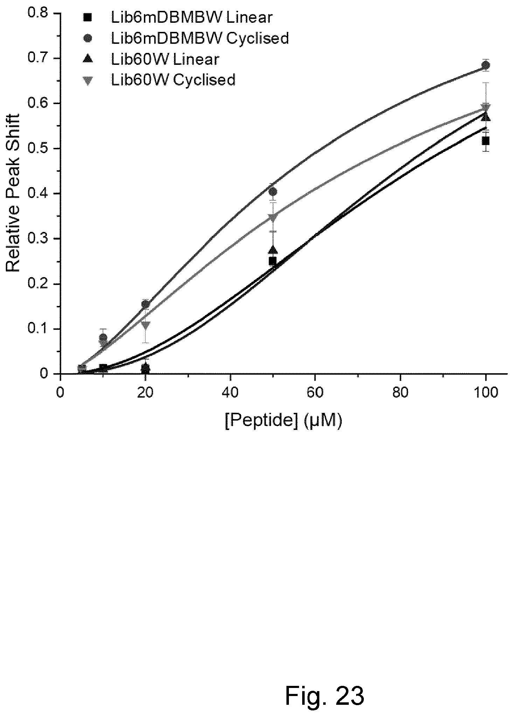

**Figure 23** - **Optimisation of bisalkylated HingeW peptide variants.** CD antagonism data produced by monitoring a shift from bound to free TRE, to provide a direct readout of cJun-induced DNA binding of the tested cyclised (mDBMBW cyclised and 0W cyclised) versus linear HingeW (mDBMBW Linear and 0W linear) variants.

### Detailed Description of the Invention

Aspects and embodiments of the present invention will now be discussed with reference to the accompanying figures. Further aspects and embodiments will be apparent to those skilled in the art.

The c-Jun antagonist described herein typically comprises a hinge region of V[X₁]EE[X₂][X₃]LE[X₄]E, more preferably an extended hinge region having an amino acid sequence of LV[X₁]EE[X₂][X₃]LE[X₄]E (SEQ ID NO: 1); and a leucine zipper (LZ) region C-terminal to the extended hinge region, wherein X₁ is V, D, K, C, or R, X₂ is K, D, C or R, X₃ is V, D, C, K, or R and X₄ is selected from E, D, C, K or R. The c-Jun antagonists are also referred to herein as 'c-Jun antagonist peptides.

### Extended hinge region

In this specification the term "hinge region" is intended to mean a ten-residue amino acid tract. The amino acid residues in the tract may be acidic (D/E) and hydrophobic residues (V), and, optionally, a K residue may be present. The "hinge region" of the c-Jun antagonist is so named because it corresponds to the "hinge" forming parts of both the DBD domain and LZ domain of c-Jun, and therefore is capable of interacting with both these domains in c-Jun. The presence of a hinge region provides a peptide with the ability of binding c-Jun as well as antagonising its DNA-binding activity. The dominant negative charge of the hinge region is also believed to result in a favourable interaction with the positive charge within the c-Jun DNA-Binding Domain (DBD).

The extended hinge region comprises the hinge region as well as an L residue at its N-terminal portion. The extended hinge region may have an amino acid sequence of LV[X₁]EE[X₂][X₃]LE[X₃]E (SEQ ID NO: 1) wherein X₁ is selected from V, D, K, C and R, X₂ is selected from D, K, C and R, X₃ is selected from V, D, C, K and R, and X₄ is selected from E, D, C, K and R. In some embodiments, X₁ is selected from V, D, K and C, X₂ is selected from K, D and C, X₃ is selected from V, D, or C, and X₄ is selected from E, D, C, or R. The extra negatively charged amino acid residue is believed to favour the interaction with the positively charged DBD of c-Jun. Also contemplated are variants of the extended hinge region sequences described herein, wherein the variant contains 1, 2 or 3 amino acid modifications.

In some embodiments, X₁ is V, and/or X₃ is V. In some embodiments, X₂ is D and X₄ is E. For example, the extended hinge region may comprise an amino acid sequence of LVVEEDVLEEE (SEQ ID NO: 31)

In other embodiments, X₂ is K and X₄ is D. For example, the extended hinge region may comprise an amino acid sequence of LVVEEKVLEDE (SEQ ID NO: 32). Such amino acid sequences can be used, for example, to introduce an *i to i+*4 K to D lactam bridge in the *b-to-f* heptad positions of the hinge region of the antagonist.

In other embodiments, X₁ is K and X₃ is D. For example, the extended hinge region may comprise an amino acid sequence of LVKEEDDLEEE (SEQ ID NO: 33). Such amino acid sequences can be used, for example, to introduce an *i to i+*4 K to D lactam bridge in the in the *f-to-c* heptad positions of the hinge region of the antagonist.

In other embodiments, X₂ is C and X₄ is C. For example, the extended hinge region may comprise an amino acid sequence of LVVEECVLECE (SEQ ID NO: 34). Such amino acid sequences can be used, for example, to introduce an *i to i*+4 alkyl cross-link in the in the *b-to-f* heptad positions of the hinge region of the antagonist.

In other embodiments, X₁ is C and X₃ is C. For example, the extended hinge region may comprise an amino acid sequence of LVCEEDCLEEE (SEQ ID NO: 35). Such amino acid sequences can be used, for example, to introduce an *i to i+*4 alkyl cross-link in the in the *f-to-c* heptad positions of the hinge region of the antagonist.

In other embodiments, X₁ is C and X₄ is C. For example, the extended hinge region may comprise an amino acid sequence of LVCEEDVLECE (SEQ ID NO: 36). Such amino acid sequences can be used, for example, to introduce an *i to i+*7 alkyl cross-link in the in the *f-to-f* heptad positions of the hinge region of the antagonist.

In some embodiments, one or more of X₁, X₂, X₃, and X₄ is an R or a K. In one embodiment, X⁴ is R or K. For example, the extended hinge region may comprise an amino acid sequence of LVVEEKVLERE (SEQ ID NO: 71). As explained below, introducing an arginine or lysine at solvent exposed positions can increase cell permeability of the peptide.

### Acidic extension

According to the present invention the extended hinge region may further comprise an N-terminal acidic extension having an amino acid sequence of EA[X₅][X₆] (SEQ ID NO: 2), wherein X₅ is selected from E, K and C, and X₆ is selected from E, D and C. In some embodiments, X6 is selected from E and D (e.g. X₆ is E).

The N-terminal acidic extension is believed to produce electrostatic repulsion, advantageously reducing the tendency of the peptide to homodimerize thereby making more peptide antagonist available for heterodimerisation with c-Jun. The negative charge throughout the N-terminal domain of the acidic extension acts favourably with the positive charge of the c-Jun DBD.

In some embodiments, X₁ (in the extended hinge region) is D and X₅ (in the acidic extension) is K. Such amino acid sequences can be used, for example, to introduce an *i to i+4* K to D lactam bridge that spans the acidic extension (heptad position *b*) and hinge region (heptad position *f*) of the antagonist.

In some embodiments, X₁ (in the extended hinge region) is C and X₅ (in the acidic extension) is C. Such amino acid sequences can be used, for example, to introduce an *i to i*+4 alkyl cross-link that spans the acidic extension (heptad position *b*) and hinge region (heptad position *f*) of the antagonist.

According to the present invention, the acidic extension may have an amino acid sequence of EAEE (SEQ ID NO:3). This amino acid sequence is believed to induces helicity and stabilises the dipole of the molecule. A further advantage of the EAEE sequence is that its two central residues, AE, occur at positions corresponding to interaction with DNA on c-Jun, thereby forming a direct block between c-Jun and DNA.

### LZ region

The LZ region of the antagonist of the invention is located C-terminal to the hinge region and is capable of interacting with the leucine zipper of c-Jun.

According to the present invention the LZ region may comprise or consist of an amino acid sequence selected from the group consisting of:
IEQLEERNYALRKEIEDLQKQ (SEQ ID NO: 4);
IEQLEEKNKALKDEIEDLQKQ (SEQ ID NO: 5);
IKQLEDRNYALRKEIEDLQKQ (SEQ ID NO: 6);
IEQLEERNYALRKEIKDLQDQ (SEQ ID NO: 7);
IEQLEEKNKALKDEIEDLY (SEQ ID NO: 8); and
IEQLEERNYALRKEIEDLQ (SEQ ID NO: 9),
or a variant thereof. The variant may comprise one or more amino acid modifications. For example, the variant may comprise 1, 2, 3, 4, or 5 amino acid modifications. For example, the variant comprises 1, 2 or 3 amino acid modifications.

In some embodiments, the LZ region comprises or consists of an amino acid sequence of IEQLEERNYALRKEIKDLQDQ (SEQ ID NO: 7), or a variant comprising 1, 2, or 3 modifications. In some embodiments, amino acid residues at positions corresponding to position 16 (position *b* in a heptad) and position 20 (position *f* in the same heptad) of SEQ ID NO: 7 in the variant are K and D amino acid residues, respectively (i.e. the amino acid modification(s) are at positions other than the positions corresponding to 16 and 20 of SEQ ID NO: 7).

In some embodiments, the LZ region comprises or consists of an amino acid sequence of IEQLEERNYALRKEICDLQCQ (SEQ ID NO: 27), or a variant comprising 1, 2, or 3 modifications. In some embodiments, amino acid residues at positions corresponding to position 16 (position *b* in a heptad) and position 20 (position *f* in the same heptad) of SEQ ID NO: 27 in the variant are both C amino acid residues (i.e. the amino acid modification(s) are at positions other than the positions corresponding to 16 and 20 of SEQ ID NO: 27).

In some embodiments, the LZ region comprises:
IEQLEERNYALR[X₇]E[X₈]K[X₉]L[X₁₀]D[X₁₁] (SEQ ID NO: 29) or
IEQLEERNYALR[X₇]E[X₈]C[X₉]L[X₁₀]C[X₁₁] (SEQ ID NO: 30),
wherein [X₇] is K, L, S, W, P, Q, R, M, T, V, A, E, or G;
[X₈] is I, V, or L;
[X₉] and [X₁₀] are any amino acid residue;
[X₁₁] is Q, E, or K.

In some embodiments, the LZ region comprises:
IEQLEERNYALRSEICSLQCQ (SEQ ID NO: 66); or
IEQLEERNYALRKEICELSCQ (SEQ ID NO: 67); or
IEQLEERNYALRAEICNLSCQ (SEQ ID NO: 68); or
IEQLEERNYALRTEICSLMCK (SEQ ID NO: 69); or
IEQLEERNYALRAEICSLQCQ (SEQ ID NO: 70).

Such LZ regions are suitable for bisalkylation, as explained in more detail below.

In some embodiments, the LZ region comprises one or more lysine(s) and/or arginine(s) at heptad positions *b, c*, and/or *f* in the LZ region. In some embodiments, the lysine(s) or arginine(s) are located at positions other than the positions being used to introduce the cross-link (e.g. lactam bridge or bisalkylation). As explained in more detail below, the introduction of positively charged amino acids at a solvent exposed face of an α-helical peptide may improve cell penetrance.

Accordingly, in some embodiments, the LZ region comprises or consists of an amino acid sequence of IRRLERRNRALRKEIKDLQDQ (SEQ ID NO: 74), or a variant comprising 1, 2, or 3 modifications. In some embodiments, amino acid residues at positions corresponding to position 16 (position *b* in a heptad) and position 20 (position *f* in the same heptad) of SEQ ID NO: 74 in the variant are K and D amino acid residues, respectively (i.e. the amino acid modification(s) are at positions other than the positions corresponding to 16 and 20 of SEQ ID NO: 74). In some embodiments, amino acid residues at positions corresponding to position 2 (position b in a heptad) and position 3 (position *c* in a heptad) and position 9 (position *b* a heptad) of SEQ ID NO: 74 in the variant are K or R (optionally R) (i.e. the amino acid modification(s) are at positions other than the positions corresponding to 2, 3, and 9 of SEQ ID NO: 74). In some embodiments, the amino acid modification(s) are at positions other than the positions corresponding to positions 2, 3, 9, 16 and 20 of SEQ ID NO: 74.

In other embodiments, the LZ region comprises or consists of an amino acid sequence of IERLERRNYRLRREIKDLQDQ (SEQ ID NO: 75), or a variant comprising 1, 2, or 3 modifications. In some embodiments, amino acid residues at positions corresponding to position 16 (position b in a heptad) and position 20 (position *f* in the same heptad) of SEQ ID NO: 75 in the variant are K and D amino acid residues, respectively (i.e. the amino acid modification(s) are at positions other than the positions corresponding to 16 and 20 of SEQ ID NO: 75). In some embodiments, amino acid residues at positions corresponding to position 3 (position *c* in a heptad), position 10 (position *c* in a heptad) and position 13 (position *f* in a heptad) of SEQ ID NO: 75 in the variant are K or R (optionally R) (i.e. the amino acid modification(s) are at positions other than the positions corresponding to 3, 10 and 13 of SEQ ID NO: 75). In some embodiments, the amino acid modification(s) are at positions other than the positions corresponding to positions 3, 10, 13, 16 and 20 of SEQ ID NO: 75.

### Antagonists and properties

The c-Jun antagonist as described herein is peptidic and may be in the D- or L-form. "Peptidic" as used herein includes compounds that are composed of or comprise a linear chain of amino acids linked by peptide bonds and may be any peptide, polypeptide or protein. The amino acid residues that form the peptidic antagonists may be comprised of D- or L-form amino acid residues, or a mixture of both. In this specification, the peptidic compounds are typically referred to as peptides.

A c-Jun antagonist as described herein may be isolated, in the sense of being free from contaminants, such as other polypeptides and/or cellular components.

The c-Jun antagonist as described herein may be in the free form, or any pharmacologically acceptable salt form, for example, a form of acid salt, metal salt, alkaline earth metal salt, or amine salt.

The c-Jun antagonist may be between 10 and 100 amino acid residues long. The c-Jun antagonist may be less than 70, preferably less than 60, more preferably less than 55, even more preferably less than 50, yet more preferably less than 45, still more preferably less than 40 amino acids long. The c-Jun antagonist may be between 30 and 70, 30 and 60, 30 and 50, or 30 and 40 amino acid residues long. For example, the c-Jun antagonist may have a length of length of 30, 31, 32, 33, 34, 35, 36, 37, 38, 39, 40, 41, 42, 43, 44, 45, 46, 47, 48, 49, 50, 51, 52, 53, 54, 55, 56, 57 amino acids. In certain embodiments, the c-Jun antagonist has a length of 36 amino acids.

The c-Jun antagonist may be the HingeW peptide, or a variant thereof. The HingeW peptide comprises an amino acid sequence of LEQRAEELARENEELEKEAEELVVEEDVLEEEIEQLEERNYALRKEIEDLQKQLEKL (SEQ ID NO: 12).

The HingeW peptide may further comprise one or more of the following: MAS at the N-terminus, GAP at the C-terminus, and a 6xHis tag (HHHHHH) (SEQ ID NO: 53) at the C-terminus. As described herein, the HingeW peptide was demonstrated to bind to the target c-Jun protein with a high affinity and antagonise the DNA-binding function of c-Jun, and hence is demonstrated to be a functional antagonist of c-Jun. The c-Jun antagonist may be a truncated form of the HingeW peptide, or a variant thereof. As described herein, various truncated HingeW peptides were developed and demonstrated to be functional antagonists of HingeW. Although the functional antagonism of these truncated forms was reduced compared to the HingeW peptide, the truncated peptides are believed to exhibit more drug-like characteristics compared to the full-length HingeW peptide, suggesting that these truncated forms also represent effective therapeutic candidates for antagonising c-Jun function.

As used herein, a 'functional antagonist' of c-Jun is a peptidic compound that is capable of binding to c-Jun and inhibit its DNA-binding activity. Methods for identifying functional antagonist peptides include the Transcription-Block Survival (TBS) assay described in Example 1. Briefly, in TBS the coding region for the essential gene dihydrofolate reductase (DHFR) is mutated to incorporate TRE sites so that introduction of c-Jun to this gene inside *E*. *coli* produces a transcriptional block that abrogates cell proliferation. The TRE site-bound c-Jun molecules sterically prevent RNA polymerase transcribing the essential gene and this can only be restored upon introduction of an effective c-Jun/TRE antagonist. Consequently, the survival of a particular cell is controlled by the ability of a peptide library member to remove the c-Jun transcriptional block and therefore to restore DHFR activity. TBS thus facilitates the identification of therapeutically valuable sequences. Further details for the TBS assay are provided in WO2020/128015.

Other methods of determining antagonism of c-Jun includes a circular dichroism (CD) assay, as described in Example 2. Briefly, this assay involves preparing a sample containing the peptide and a TRE-DNA construct (GTCAGTCAGTGACTCAATCGGTCA) (SEQ ID NO: 51) and measuring the signal between 265-320 nm. The TRE-DNA construct produces a positive CD peak at ~281nm, which decreases in intensity upon c-Jun binding. If peptide is capable of antagonising c-Jun DNA binding activity, increasing concentrations of the peptide will shift the peak back to the free TRE-DNA peak. Hence, peak shift can be used to quantify the ability of the peptide to antagonise c-Jun DNA binding. This method allows for the calculation of an *IC₅₀* value by fitting the titration data to a Hill equation.

In some embodiments, the c-Jun antagonist is able to inhibit the DNA-binding activity of c-Jun within 10-fold of the ability of HingeW to inhibit the DNA-binding activity of c-Jun (e.g. the c-Jun antagonist has a reduced ability to inhibit the DNA-binding activity of c-Jun that is within 10-fold of that determined for HingeW). In some embodiments, the c-Jun antagonist is able to inhibit the DNA-binding activity of c-Jun within 9-fold, preferably within 8-fold, more preferably within 7-fold, even more preferably within 6-fold, yet more preferably within 5-fold of the ability of HingeW to inhibit the DNA-binding activity of c-Jun. The ability of the c-Jun antagonist and HingeW to inhibit the DNA-binding activity may be measured using the TBS assay (e.g. by quantifying the number of colonies) or by determining the IC₅₀ using a circular dichroism assay described herein. Optionally, the c-Jun antagonist may have this activity when cross-linked. Methods for cross-linking peptides are described in more detail below.

In some embodiments, the c-Jun antagonist comprises or consists of any one of the following amino acid sequences:
LEQRAEELARENEELEKEAEELVVEEDVLEEEIEQLEERNYALRKEIEDLQKQLEKL (SEQ ID NO: 12);
LARENEELEKEAEELVVEEDVLEEEIEQLEERNYALRKEIEDLQKQLEKL (SEQ ID NO: 13);
LEKEAEELVVEEDVLEEEIEQLEERNYALRKEIEDLQKQLEKL (SEQ ID NO: 14);
EAEELVVEEDVLEEEIEQLEERNYALRKEIEDLQKQLEKL (SEQ ID NO: 15);
LVVEEDVLEEEIEQLEEKNKALKDEIEDLQKQLEKLY (SEQ ID NO: 16);
LVVEEDVLEEEIEQLEERNYALRKEIEDLQKQLEDL (SEQ ID NO: 17);
EAEELVVEEDVLEEEIEQLEERNYALRKEIEDLQKQ (SEQ ID NO: 18);
KEAEDLVVEEDVLEEEIEQLEERNYALRKEIKDLQDQ (SEQ ID NO: 19);
EAKELVDEEDVLEEEIEQLEERNYALRKEIEDLQKQ (SEQ ID NO: 20);
EAEELVVEEKVLEDEIEQLEERNYALRKEIEDLQKQ (SEQ ID NO: 21);
EAEELVVEEDVLEEEIKQLEDRNYALRKEIEDLQKQ (SEQ ID NO: 22);
EAEELVVEEDVLEEEIEQLEEKNKALKDEIEDLQKQY (SEQ ID NO: 23);
EAEELVVEEDVLEEEIEQLEERNYALRKEIKDLQDQ (SEQ ID NO: 10);
EAEELVVEEKVLEDEIEQLEERNYALRKEIKDLQDQ (SEQ ID NO: 11);
EAEELVVEEDVLEEEIEQLEERNYALRKEIEDL (SEQ ID NO: 24);
EAEELVVEEDVLEEEIEQLEEKNKALKDEIEDLY (SEQ ID NO: 25);
EAEELVVEEDVLEEEIEQLEERNYALRKEIEDLQ (SEQ ID NO: 26);
EAEELVVEEDVLEEEIEQLEERNYALRKEICDLQCQ (SEQ ID NO: 28);
EAEELVVEEDVLEEEIEQLEERNYALRSEICSLQCQ (SEQ ID NO: 37);
EAEELVVEEDVLEEEIEQLEERNYALRKEICELSCQ (SEQ ID NO: 38);
EAEELVVEEDVLEEEIEQLEERNYALRAEICNLSCQ (SEQ ID NO: 39);
EAEELVVEEDVLEEEIEQLEERNYALRTEICSLMCK (SEQ ID NO: 40);
or EAEELVVEEDVLEEEIEQLEERNYALRAEICSLQCQ (SEQ ID NO: 41);
EAEELVVEEKVLEREIRRLERRNRALRKEIKDLQDQ (SEQ ID NO:72); or
EAEELVVEEKVLEDEIERLERRNYRLRREIKDLQDQ (SEQ ID NO: 73),
or a variant thereof comprising 1, 2 or 3 amino acid modifications, optionally wherein the 1, 2 or 3 amino acid modifications are present in the LZ region.

As described herein, the c-Jun antagonist amino acid sequence may be modified in order to introduce covalent *i to i+4* cross-linker(s) or *i to i+7* cross-linker(s) into the c-Jun antagonist. The covalent *i to i+*4 amino acid cross-linker(s) may be K to D lactam bridge(s), or an alkyl cross-link formed between two C residues (cysteine alkylation). Preferably, *i to i+*4 amino acid residue cross-links are introduced at solvent exposed *b*-to-*f* (in one heptad) or *f*-to-*c* (spanning two heptads) heptad positions in order to prevent disruption of the binding surface of the helix. In this specification, heptad numbering refers to the positioning of a specific amino acid residue within a heptad repeat, which is a structural motif that consists of a repeating pattern of seven amino acids. The positions of the heptad repeat are commonly denoted by the lowercase letters *a* to *g*, typically *abcdefg.* Table 5 below illustrates how heptad number corresponds to the HingeW amino acid sequence.

Hence, provided herein is a c-Jun antagonist comprising a modified version of the amino acid sequence EAEELVVEEDVLEEEIEQLEERNYALRKEIEDLQKQ (SEQ ID NO: 18), wherein the modifications include one or more (e.g. one or two) of the following:
(i) amino acid residues at positions corresponding to position 3 (position *b* in a heptad) and position 7 (position *f* in the same heptad) of SEQ ID NO: 18 are K and D amino acid residues, respectively, or both are C amino acid residues;
(ii) amino acid residues at positions corresponding to position 10 (position *b* in a heptad) and position 14 (position *f* in the same heptad) of SEQ ID NO: 18 are K and D amino acid residues, respectively, or both are C amino acid residues;
(iii) amino acid residues at positions corresponding to position 17 (position *b* in a heptad) and position 21 (position *f* in the same heptad) of SEQ ID NO: 18 are K and D amino acid residues, respectively, or both are C amino acid residues;
(iv) amino acid residues at positions corresponding to position 24 (position *b* in a heptad) and position 28 (position *f* in the same heptad) of SEQ ID NO: 18 are K and D amino acid residues, respectively, or both are C amino acid residues; and
(v) amino acid residues at positions corresponding to position 31 (position *b* in a heptad) and position 35 (position *f* in the same heptad) of SEQ ID NO: 18 are K and D amino acid residues, respectively, or both are C amino acid residues,
or a variant thereof comprising 1, 2, 3 or 4 amino acid modifications outside of the stated positions (i.e. outside of the positions *b* and *f* in each heptad).

Also provided herein is a c-Jun antagonist comprising a modified version of the amino acid sequence EAEELVVEEDVLEEEIEQLEERNYALRKEIEDLQKQ (SEQ ID NO: 18), wherein the modifications include one or more (e.g. one or two) of the following:
(i) amino acid residues at positions corresponding to position 7 (position *f* in a heptad) and position 11 (position c in the subsequent heptad) of SEQ ID NO: 18 are K and D amino acid residues, respectively, or both are C amino acid residues;
(ii) amino acid residues at positions corresponding to position 14 (position *f* in a heptad) and position 18 (position *c* in the subsequent heptad) of SEQ ID NO: 18 are K and D amino acid residues, respectively, or both are C amino acid residues;
(iii) amino acid residues at positions corresponding to position 21 (position *f* in a heptad) and position 25 (position c in the subsequent heptad) of SEQ ID NO: 18 are K and D amino acid residues, respectively, or both are C amino acid residues; and
(iv) amino acid residues at positions corresponding to position 28 (position *f* in a heptad) and position 32 (position *c* in the subsequent heptad) of SEQ ID NO: 18 are K and D amino acid residues, respectively, or both are C amino acid residues,
or a variant thereof comprising 1, 2, 3 or 4 amino acid modifications outside of the stated positions (i.e. outside of the positions *f* in one heptad and *c* in the subsequent heptad).

Also provided are c-Jun antagonists where the amino acid sequence has been modified in order to introduce *i to i+7* cross-linker(s) into the c-Jun antagonist. The covalent *i to i+7* amino acid cross-linker(s) may be alkyl cross-link formed between two C residues (cysteine alkylation). Preferably, *i to i*+7amino acid residue cross-links are introduced at solvent exposed *b*-to-*b*, *c*-to-*c* or *f*-to-*f* (spanning two heptads) heptad positions.

Hence, provided herein is a c-Jun antagonist comprising a modified version of the amino acid sequence EAEELVVEEDVLEEEIEQLEERNYALRKEIEDLQKQ (SEQ ID NO: 18), wherein the modifications include one or more (e.g. one or two) of the following:
(i) amino acid residues at positions corresponding to position 3 (position *b* in a heptad) and position 10 (position *b* in the subsequent heptad) of SEQ ID NO: 18 are both C amino acid residues;
(ii) amino acid residues at positions corresponding to position 10 (position *b* in a heptad) and position 17 (position *b* in the subsequent heptad) of SEQ ID NO: 18 are both C amino acid residues;
(iii) amino acid residues at positions corresponding to position 17 (position *b* in a heptad) and position 24 (position *b* in the subsequent heptad) of SEQ ID NO: 7 are both C amino acid residues;
(iv) amino acid residues at positions corresponding to position 24 (position *b* in a heptad) and position 31 (position *b* in the subsequent heptad) of SEQ ID NO: 7 are both C amino acid residues;
(v) amino acid residues at positions corresponding to position 4 (position *c* in a heptad) and position 11 (position *c* in the subsequent heptad) of SEQ ID NO: 18 are both C amino acid residues;
(vi) amino acid residues at positions corresponding to position 11 (position *c* in a heptad) and position 18 (position *c* in the subsequent heptad) of SEQ ID NO: 18 are both C amino acid residues;
(vii) amino acid residues at positions corresponding to position 18 (position *c* in a heptad) and position 25 (position *c* in the subsequent heptad) of SEQ ID NO: 18 are both C amino acid residues;
(viii) amino acid residues at positions corresponding to position 25 (position *c* in a heptad) and position 32 (position *c* in the subsequent heptad) of SEQ ID NO: 18 are both C amino acid residues;
(ix) amino acid residues at positions corresponding to position 7 (position *f* in a heptad) and position 14 (position *f* in the subsequent heptad) of SEQ ID NO: 18 are both C amino acid residues;
(x) amino acid residues at positions corresponding to position 14 (position *f* in a heptad) and position 21 (position *f* in the subsequent heptad) of SEQ ID NO: 18 are both C amino acid residues;
(xi) amino acid residues at positions corresponding to position 21 (position *f* in a heptad) and position 28 (position *f* in the subsequent heptad) of SEQ ID NO: 18 are both C amino acid residues; and
(xii) amino acid residues at positions corresponding to position 28 (position *f* in a heptad) and position 35 (position *f* in the subsequent heptad) of SEQ ID NO: 18 are both C amino acid residues,
or a variant thereof comprising 1, 2, 3 or 4 amino acid modifications outside of the stated positions (i.e. outside of the positions *b, c* or *f*, accordingly).

In preferred embodiments, the c-Jun antagonist comprises or consists of the amino acid sequence of:
EAEELVVEEDVLEEEIEQLEERNYALRKEIEDLQKQLEKL (SEQ ID NO:15);
EAEELVVEEDVLEEEIEQLEERNYALRKEIEDLQKQ (SEQ ID NO:18);
EAEELVVEEDVLEEEIEQLEERNYALRKEIKDLQDQ (SEQ ID NO:10);
EAEELVVEEKVLEDEIEQLEERNYALRKEIKDLQDQ (SEQ ID NO: 11); or
EAEELVVEEDVLEEEIEQLEERNYALRKEICDLQCQ (SEQ ID NO: 28),
or a variant thereof comprising 1, 2 or 3 amino acid modifications, optionally wherein the 1, 2 or 3 amino acid modifications are present in the LZ region.

In more preferred embodiments, the c-Jun antagonist comprises or consists of the amino acid sequence of:
EAEELVVEEDVLEEEIEQLEERNYALRKEIKDLQDQ (SEQ ID NO:10);
EAEELVVEEKVLEDEIEQLEERNYALRKEIKDLQDQ (SEQ ID NO: 11); or
EAEELVVEEDVLEEEIEQLEERNYALRKEICDLQCQ (SEQ ID NO: 28),
or a variant thereof comprising 1, 2 or 3 amino acid modifications, optionally wherein the 1, 2 or 3 amino acid modifications are present in the LZ region.

For example, in embodiments where the c-Jun antagonist comprises a K to D lactam bridge, the c-Jun antagonist may comprise or consist of the amino acid sequence of:
EAEELVVEEDVLEEEIEQLEERNYALRKEIKDLQDQ (SEQ ID NO:10),
or a variant thereof comprising 1, 2 or 3 amino acid modifications, optionally wherein the 1, 2 or 3 amino acid modifications are present in the LZ region, further optionally wherein amino acid residues at positions corresponding to position 31 (position b in the heptad) and position 35 (position *f* in the heptad) of SEQ ID NO: 10 in the variant are K and D amino acid residues, respectively.

As another example, in embodiments where the c-Jun antagonist comprises two K to D lactam bridges, the c-Jun antagonist may comprise or consist of the amino acid sequence of:
EAEELVVEEKVLEDEIEQLEERNYALRKEIKDLQDQ (SEQ ID NO: 11),
or a variant thereof comprising 1, 2 or 3 amino acid modifications, optionally wherein the 1, 2 or 3 amino acid modifications are present in the LZ region,
further optionally wherein amino acid residues at positions corresponding to position 10 (position *b* in a first heptad) and position 14 (position *f* in the first heptad) of SEQ ID NO: 11 in the variant are K and D amino acid residues, respectively, and wherein amino acid residues at positions corresponding to position 31 (position b in a second heptad) and position 35 (position *f* in the second heptad) of SEQ ID NO: 11 in the variant are K and D amino acid residues, respectively.

As another example, in embodiments where the c-Jun antagonist comprises an alkyl cross-link, the c-Jun antagonist may comprise or consist of the amino acid sequence of EAEELVVEEDVLEEEIEQLEERNYALRKEICDLQCQ (SEQ ID NO:28),
or a variant thereof comprising 1, 2 or 3 amino acid modifications, optionally wherein the 1, 2 or 3 amino acid modifications are present in the LZ region, further optionally wherein amino acid residues at positions corresponding to position 31 (position *b* in a heptad) and position 35 (position *f* in the same heptad) of SEQ ID NO: 28 in the variant are both C amino acid residues.

As another example, in embodiments where the c-Jun antagonist comprises an alkyl cross-link the c-Jun antagonist may comprise or consist of the amino acid sequence of
EAEELVVEEDVLEEEIEQLEERNYALRSEICSLQCQ (SEQ ID NO: 37); or
EAEELVVEEDVLEEEIEQLEERNYALRKEICELSCQ (SEQ ID NO: 38); or
EAEELVVEEDVLEEEIEQLEERNYALRAEICNLSCQ (SEQ ID NO: 39); or
EAEELVVEEDVLEEEIEQLEERNYALRTEICSLMCK (SEQ ID NO: 40); or
EAEELVVEEDVLEEEIEQLEERNYALRAEICSLQCQ (SEQ ID NO: 41) or a variant thereof comprising 1, 2, or 3 amino acid modifications, optionally wherein the amino acid modifications are present in the LZ region.

An amino acid modification may be an insertion, a substitution, or a deletion. In some embodiments, the amino acid modification is a substitution of an amino acid residue to any other amino acid residue. The substituted amino acid residue may be in the D- or L-form and may be a naturally occurring amino acid residue or a non-naturally occurring amino acid residue.

Naturally occurring residues may be divided into classes based on common side chain properties:
1) nonpolar, aliphatic (hydrophobic): glycine (G), methionine (M), alanine (A), valine (V), leucine (L), isoleucine (I);
2) polar, uncharged: cysteine (C), serine (S), threonine (T), asparagine (N), glutamine (Q), proline (P);
3) acidic (negatively charged): aspartic acid (D), glutamic acid (E);
4) basic (positively charged): histidine (H), lysine (K), arginine I;
5) aromatic: tryptophan (W), tyrosine (Y), phenylalanine (F).

The amino acid substitution may be a conservative amino acid substitution. Conservative amino acid substitutions may involve exchange of a member of one of these classes with another member of the same class. For example, a conservative amino acid substitution may be a substitution of the acidic amino acid glutamic acid (E) for the acidic amino acid aspartic acid (D).

Amino acid substitutions (e.g. conservative amino acid substitutions) may encompass non-naturally occurring amino acid residues, which are typically incorporated by chemical peptide synthesis rather than by synthesis in biological systems. Suitable non-natural amino acids include 3-Cyclohexylalanine (Cha), Norleucine (NLe) and Ornithine (Orn). Other examples of non-natural amino acids include citrulline (Cit), hydroxyproline (Hyp), 3-nitrotyrosine, nitroarginine naphtylalanine (Nal), Abu, DAB, methionine sulfoxide and methionine sulfone.

In some embodiments, the amino acid modifications result in the introduction of hydrophobic and charged surface patches in the peptide. Hydrophobic and charged surface patches can be introduced by inserting clusters of amino acid residues (e.g. at least 3 contiguous residues) that are hydrophobic and/or positively charged, as described for example in Perry et al., 2018. For example, the amino acid modifications described herein may produce a c-Jun antagonist that contains at least 3 contiguous amino acid residues that are either lysine or leucine (e.g. in the extended hinge region and/or leucine region).

For c-Jun antagonists that are cross-linked, any amino acid modifications (e.g. substitutions) are typically located outside of the relevant positions that are being used for cross-linking. That is, for antagonists comprising *b*-to-*f* (in one heptad) amino acid residue cross-links, the amino acid modification(s) may be at positions *a*, *c, d, e,* or *g* in that heptad. Similarly, for antagonists comprising *f*-to-*c* (spanning two heptads) amino acid residue cross-links, the amino acid modification(s) may be at any of positions *a, b, c, d or e* in the first heptad and *a, b, d, e*, *f* or *g*, in the second heptad.

It is known in the art that the introduction of positively charged amino acids at a solvent exposed face of an α-helical peptide improves cell penetrance (see for example, Smith et al., 2008 and Perry et al., 2018). This may be achieved by the introduction of arginine residues at specific positions in order to generate an arginine substitution pattern known to promote cell permeability as described in Smith et al., 2008. Accordingly, in some embodiments, the peptides described herein comprise one or more arginine or lysine substitutions. In some embodiments, the extended hinge region and/or LZ region comprises one or more arginine or lysine modifications, i.e. an arginine or lysine substitutions pattern may be introduced in the peptides described herein. In some embodiments, these arginine modifications are located at heptad positions *b, c*, and/or *f*, *i.e.* on the solvent exposed face of an α-helical peptide.

In some embodiments, the c-Jun antagonist peptide described herein comprises a modified version of the amino acid sequence according to SEQ ID NO:11, wherein modifications include one or more (e.g one or two) of the following:
(i) amino acid residues at positions corresponding to positions 14 (position *f* in a heptad), 17 (position b in a heptad), 18 (position c in a heptad) 24 (position *b* in a heptad) are K or R residues (optionally R). For example, the c-Jun antagonist peptide may have the amino acid sequence EAEELVVEEKVLEREIRRLERRNRALRKEIKDLQDQ (SEQ ID NO:72); or
(ii) amino acid residues at positions corresponding to position 18 (position *c* in a heptad), position 25 (position *c* in a heptad), position 28 (position f in a heptad) are K or R residues (optionally R).

For example, the c-Jun antagonist may have the amino acid sequence
EAEELVVEEKVLEDEIERLERRNYRLRREIKDLQDQ (SEQ ID NO: 73)
or a variant thereof comprising 1, 2, 3 or 4 amino acid modifications outside of the stated positions. Optionally, the amino acid residues at positions corresponding to position 31 (position *b* in a heptad) and position 35 (position *f* in a heptad) of SEQ ID NO: 72 or 73 in the variant are K and D amino acid residues, respectively (i.e. the amino acid modification(s) are at positions other than positions corresponding to positions 14, 17, 18, 31 and 35 of SEQ ID NO: 72, or at positions other than positions corresponding to positions 18, 25, 28, 31 and 35 of SEQ ID NO: 73.

Alternatively or additionally, a c-Jun antagonist may have an amino acid sequence having a specified degree of sequence identity to one of SEQ ID Nos 12 to 26. The specified degree of sequence identity may be from at least 60% to 100% sequence identity. More preferably, the specified degree of sequence identity may be one of at least 65%, 70%, 75%, 80%, 85%, 86%, 87%, 88%, 89%, 90%, 91%, 92%, 93%, 94%, 95%, 96%, 97%, 98% or 99% identity.

### Production of antagonists

A c-Jun antagonist peptide as described herein may be provided using synthetic or recombinant techniques which are standard in the art. Conveniently, a c-Jun peptide as described herein may be produced by solid phase synthesis. Peptides are typically synthesized by solid phase synthesis in a stepwise fashion from the C terminus to the N terminus. In an initial step, an N protected amino acid is covalently attached to an insoluble solid support via its carbonyl group. Suitable groups for N protecting the amino acid include 9-fluorenylmethyloxycarbonyl group (Fmoc) and t-butyloxycarbonyl (Boc). Following covalent attachment of the N protected amino acid, the N protecting group is removed and the deprotected NH₂ group of the attached amino acid is reacted with the carboxylic acid group of the next N protected amino acid to generate a nascent peptide comprising 2 amino acids that is covalently attached to the solid phase. This process is repeated until the complete peptide sequence is built up on the solid phase. In some embodiments, protecting groups may be employed to prevent functional groups in the side chains of amino acids from reacting with an incoming N protected amino acids. These side chain protecting groups may be present throughout the synthesis of the peptide and may be removed in a final deprotection step.

A method of producing a c-Jun antagonist peptide may comprise synthesising a peptide comprising SEQ ID NO:1 by solid or liquid phase peptide synthesis.

Methods of solid phase peptide synthesis are well-established in the art (see for example Coin et al Nature Protocols 2, 3247-3256 (2007) Stawikowski (2002) Curr Protoc Protein Sci. 2002 Unit-18.1. oi:10.1002/0471140864. ps1801s26; Chan and White; Fmoc Solid Phase Peptide Synthesis - A Practical Approach. Oxford University Press, 2000; Stewart, J. M.; Young, J. D. Solid-Phase Peptide Synthesis (2nd ed.), Pierce Chemical Co., Rockford, IL, 1984; Atherton, E.; Sheppard, R. C., Solid-Phase Peptide Synthesis: A Practical Approach. Oxford University Press: New York City, 1989; M. Bodanzsky and A.

Bodanzsky, The Practice of Peptide Synthesis, Springer Verlag, New York (1984); J. H. Jones, The Chemical Synthesis of Peptides. Oxford University Press, Oxford 1991; in Applied Biosystems 430A User's Manual, ABI Inc., Foster City, California; G. A. Grant, (Ed.) Synthetic Peptides, A User's Guide. W. H. Freeman & Co., New York 1992, and G.B. Fields, (Ed.) Solid-Phase Peptide Synthesis (Methods in Enzymology Vol. 289). Academic Press, New York and London 1997); Merrifield, J. Amer. Chem. Soc. 85:2149-54(1963)). Methods of liquid phase peptide synthesis are also well-established in the art (U.S. Pat. No. 5,516,891).

A c-Jun antagonist peptide as described herein may be produced using recombinant expression. Recombinant techniques for producing peptides are standard in the art, for example as described in Sambrook, J., Russel, D.W. Molecular Cloning, A Laboratory Manual. 3 ed. 2001, Cold Spring Harbor, New York: Cold Spring Harbor Laboratory Press. The c-Jun antagonist peptide may be capped, for example it may be capped at the N-terminus with MAS residues and at the C-terminus with GAP residues. The c-Jun antagonist peptide as described herein may further also be His-tagged (e.g. 6xHis-tagged).

### c-Jun

An antagonist peptide described herein antagonises c-Jun. C-Jun is involved in a number of cellular processes including differentiation, proliferation, and survival (Shaulian et al., 2001; Eferl et al., 2003; Eckert et al., 2013; Alani et al., 1991). Human c-Jun has been well-characterised in the art and may, for example, have the amino acid sequence of (UniProt accession P05412, version 2.).

### Peptide cross-linkers

The residues present on the surface of a protein that are responsible for PPIs are associated with protein secondary structure motifs, such as alpha-helix, beta-sheets and beta-turns. Of note, alpha-helices are thought to comprise approximately 60% of all secondary structures in protein complexes (Jochim and Arora, 2010). Additionally, alpha-helices have been shown to mediate a large number of key therapeutically relevant PPI interfaces, of which 60% bind to one face of the helix (Raj et al., 2013). Alpha-helices contain a hydrogen bond between the carbonyl group (C=O) of a given amino acid and the amino group (NH) of an amino acid three or four residues away.

Constraining peptides in a helical conformation using a cross-linker has been reported to confer benefits that include enhancing protease resistance, stability in cells, increases cellular uptake, enhanced biophysical properties and are anticipated to bind their targets with higher potency in comparison to wild-type peptide sequences (Azzarito et al. 2013). As a result, peptides that contain constrained alpha-helices (also termed "helix-constrained peptides") have been of great interest for identifying PPI inhibitors (Robertson and Spring, 2018).

Thus, in some embodiments, the c-Jun antagonist peptide compound is a helix-constrained peptide.

The term "helix-constrained peptide" is intended to mean a peptide having at least one chemical modification that results in an intramolecular cross-link between two amino acids in order to produce a stabilised alpha-helix. Generally, the cross-link extends across the length of one or two helical turns (i.e. about 3-3.6 or about 7 amino acids). Accordingly, amino acids positioned at *i* and one of: *i+3, i+4,* and *i+7* are ideal candidates for cross-linking. Thus, for example, where a peptide has the sequence . . . N1, N2, N3, N4, N5, N6, N7, N8, N9 . . . , and the amino acid N is independently selected for each position, cross-links between N1 and N4, or between N1 and N5, or between N1 and N8 are useful as are cross-links between N2 and N5, or between N2 and N6, or between N2 and N9, etc. The use of multiple cross-links (e.g., 2, 3, 4 or more) is also contemplated. Hence, as used herein, a helix-constrained peptide comprises at least one cross-linker between two amino acid residues.

Chemical modification includes a chemical modification to incorporate a molecular tether, such as a hydrocarbon staple, and a chemical modification to promote the formation of a disulphide bridge. The cross-link can be an ionic, covalent or hydrogen bond that links the two residues together, preferably the cross-link is a covalent bond.

The presence of a stabilised alpha-helix can be determined using methods such as circular dichroism spectroscopy for an alpha-helix, for example as described in Jo et al. (2012) as in the examples herein. Circular dichroism be used to measure a helicity increase, i.e. linear to cyclic. In situations where the cross-linking occurs through the formation of a disulphide bridge between two thiol groups, such as between two cysteine residues, the presence of a stabilised alpha-helix can also be determined using an assay that determining if thiols in the sample are free or conjugated. For example, free thiols can be assayed via reaction with Ellman's reagent (5,5'-dithiobis(2-nitrobenzoic acid; DNTB) (Sigma)) and monitoring absorbance at 412 nm.

Methods of inducing cross-links between amino acids are well known and include methods that induce cross-links between the peptide backbone, e.g. between the carbonyl group and amino group as in natural alpha-helices, as well as between side-chains of the peptides.

Cross linkers include disulfide bonds (e.g. as described in Leduc et al. (2003)), hydrogen bond surrogates (e.g. as described in Wang et al. (2005)), ring-closing metathesis (e.g. as described in Walensky et al. (2004)), cysteine alkylation using α-haloacetamide derivatives (e.g. as described in Woolley (2005)) or biaryl halides (e.g. as described in Muppidi et al. (2011)), lactam rings (e.g. as described in Fujimoto et al. (2008)), hydrazine linkage (e.g. as described in Cabezas & Satterthwait (1999)), oxime linkage (e.g. as described in Haney et al. (2011)), metal chelation (e.g. as described in Ruan et al. (1990)), and "click" chemistry (e.g. as described in Holland-Nell & Meldal (2011)).

The cross-linker may be used to cross-link cysteine residues. Hence, the peptide may comprise a cysteine (C) at positions *i* and *i+4,* or *i* and *i+7,* in its amino acid sequence. As described in Jo et al. (2012), the introduction of cysteine residues at *i* and *i+4* positions is useful because this spacing brings two thioether residues into proximity when in the alpha-helix. Suitable cross-linking agents for stabilising the alpha-helix within the peptide containing a cysteine (C) at position *i* and *i+4* are described in Jo et al. (2012). For example, the cross-linking agent could be a cross-linker selected from the group consisting of an alkyl bromide, an alkyl iodide, a benzyl bromide, an allyl bromide, a maleimide, and an electrophilic difluorobenzene. Suitable cross-linkers are known in the art for crosslinking cysteine (see for example: Fairlie & Dantas de Araujo, 2016 and Jo et al., 2012).

In some embodiments, the cross-linking agent is an m-xylene based, o-xylene based, or *p*-xylene based benzyl bromide, more preferably a m-xylene based benzyl bromide.

In some embodiments, the cross-linker is a compound of formula 1: 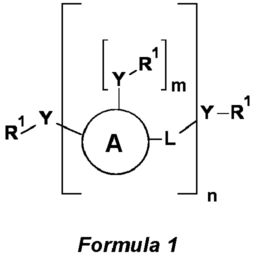 wherein
n is an integer selected from 1 to 3;
m is an integer selected from 0 to 2;
A is selected from C₂₋₆-alkenylene, C₅₋₁₂-arylene and C₅₋₁₂-heteroarylene;
Y is a covalent bond, C₁₋₆alkylene or -N(H)C(=O)CH₂-;
R¹ is selected from CI, Br, I, or F;and
each L is independently selected from -C(=O)-, -C=C-, -N=N-, C₁₋₆alkylene and a covalent bond.

The R¹ groups provide reactive groups (e.g. leaving groups) for reaction with the cysteine. The A groups provide the linkers with structures suitable for conformationally constraining a peptide in a call when cross inked via the two derivatisable amino acid residues. For example, the A group may be conformational constrained into a geometry suitable for linking the two derivatisable amino acid residues. In some embodiments, R¹ is Br. In some embodiments A is selected from C₅₋₁₂-arylene and C₅₋₁₂-heteroarylene. In some embodiments m is 0. In some embodiments Y is methylene. In some embodiments L is a covalent bond.

In some preferred embodiments, the cross-linking agent is 1,3-dibromomethylbenzene (DBMB) having the following chemical formula:

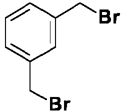

DBMB can be used to react with derivatisable amino acid residues at the *i* and *i*+*3* or *i* and *i+4* in the amino acid sequence of the peptide.

In some preferred embodiments, the cross-linking agent is 4,4'-bisbromomethyl-biphenyl (Bpy) having the following chemical formula:

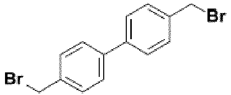

Bpy can be used to react with derivatisable amino acid residues at the *i* and *i+7* in the amino acid sequence of the peptide.

Cross-linking cysteine residues in peptides can be carried out using known methods, such as those described in Timmerman et al., 2005 or WO 2021/260074. Briefly, this method may comprise reacting the cross-linker (e.g. DBMB) with the peptide in the presence of tris(2-carboxyethyl) phosphine (TCEP) and ammonium bicarbonate, and reacted at pH 8.0 and room temperature for 4 to 5 hours in the dark. The method may be carried out *in vitro* or *in cellulo. in cellulo* methods may comprise providing a cell (e.g. a bacterial cell, such as an *E. coli* cell, or a eukaryotic cell, such as a human cell) containing a recombinant peptide, contacting the cell with the cross-linker (e.g. as part of the cell culture media) and culturing the cell in the presence of the cross-linker. The cross-linker may be present at a concentration of between 1 µM and 1 mM (e.g. between 10 µM and 100 µM), and for a period of at least 20 minutes (e.g. between 20 minutes and 10 hours). Further details of a suitable *in cellulo* cross-linking method are provided for example in WO 2021/260074.

The crosslinker forms thioether cross-links with the at least a pair of cysteines such that the c-Jun antagonist may comprise the structure:

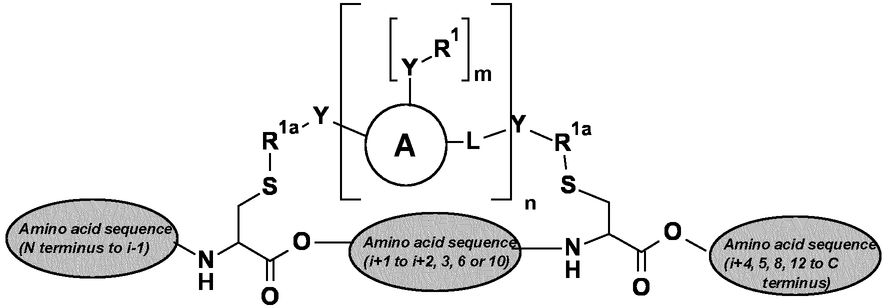

Y, L, R¹, n, m and A are as defined for formula 1. R^{1a} represents a bond or CH2-CH2- linker derived from the appropriate R1 group in formula 1.

In embodiments where the alkyl cross-link formed between two C residues is formed by DBMB, the c-Jun antagonist may comprise the structure:

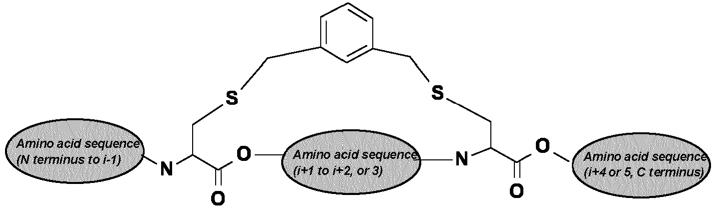

The cross-linker may be used to cross-link lysine (K) and aspartic acid (D) in the peptide. Hence, the peptide may comprise a lysine (K) and aspartic acid (D) at *i* and *i+4* positions in its amino acid sequence. That is, position *i* is a lysine (K) and position *i+4* is an aspartic acid (D), or position *i* is an aspartic acid (D) and position *i*+*4* is a lysine (K). For example, the K may be at **b** and the D at **f** in one heptad, or the K may be at **f** in one heptad and the D at **c** in the subsequent heptad.

Lactamisation is useful in terms of biostability since proteases universally recognise β-strands, with the constraint providing a further steric block, denying access to the backbone (Tyndall JD et al., 2005), and potentially bioavailability and membrane permeability owing to the lipophilic nature of the constraint. A lactam bridge in a peptide refers to the side chain of lysine (K) forming an amide bond with the side chain of glutamic acid (E) or aspartic acid (D), typically aspartic acid (D). Methods of carrying out K-D lactamisation are described in the examples herein and, for example, in de Araujo et al. (2014).

As noted above, the cross-link may be formed between amino acids at positions *i* and *i+3, i* and *i+4,* or *i* and *i*+7 in the amino acid sequence of the peptide. In some embodiments, the cross-link is between cysteine (C) residues located at these positions. In other embodiments, the cross-link is between lysine (K) and aspartic acid (D) residues at these positions. Preferably, the cross-link is formed between amino acids at positions *i* and *i+4.*

### Nucleic acids

In this specification, a nucleic acid encoding a c-Jun antagonist peptide may be any nucleic acid (DNA or RNA).

### Conjugates

In some embodiments, the c-Jun antagonist may be conjugated, optionally through a linker, to another moiety, such as a fatty acid or other lipid, a polymer, or another peptide sequence (e.g. a cell penetrating peptides (CPPs). Such conjugates retain the functional antagonist property of the c-Jun antagonist, and may have one or more improved properties, such as stability, in vivo half-life, or potency, or cell penetrance relative to unconjugated c-Jun antagonist. The moiety may be conjugated to the c-Jun antagonist through the N- or C-terminus, or any other site of the peptide.

In some embodiments, the peptide may be conjugated to a cell penetrating peptides (CPP). CPPs are a class of peptides capable of penetrating the plasma membrane of mammalian cells and of transporting compounds of many types and molecular weights across the membrane. When CPPs are chemically linked or fused to other proteins, the resulting polypeptides are able to enter cells. The linkage to the CPP may be direct (e.g. as part of a fusion protein), or may be via a linker (e.g. a short peptide linker).

CPPs are generally peptides of less than 30 amino acids, derived from natural or unnatural protein or chimeric sequences. Examples of CPPs include tat (PGRKKRRQRRPPQ) (SEQ ID NO: 54), penetratin (RQIKIWFQNRRMKWKK) (SEQ ID NO: 55), transportan (GWTLNSAGYLLGKINLKALAALAKKIL) (SEQ ID NO: 56), VP-22 (DAATATRGRSAASRPTERPRAPARSASRPRRPVD) (SEQ ID NO: 57), Pep-1 (KETWWETWWTEWSQPKKKRKV) (SEQ ID NO: 58), MAP (KALAKALAKALA) (SEQ ID NO: 59), SAP (VRLPPPVRLPPPVRLPPP) (SEQ ID NO: 60), oligoarginine (RRRRRRRR (SEQ ID NO: 61) or RRRRRRRRR (SEQ ID NO: 62)), calcitonin (LGTYTQDFNKTFPQTAIGVGAP) (SEQ ID NO: 63), SynB (RGGRLSYSRRRFSTSTGR (SEQ ID NO: 64)), and Pvec (LLIILRRRIRKQAHAHSK (SEQ ID NO: 65)). These and other suitable CPPs are described in Heitz et al. 2009.

In some embodiments, the peptide may be conjugated to a lipid. Peptide lipidation is an effective strategy to modify the pharmacokinetic, pharmacodynamic and cell penetrance properties of peptide therapeutics and has proven to be successful with several therapeutic peptides. Cholesterol and fatty acids of various chain lengths such as C8-caprylic, C12-lauric, and C16-palmitic are often utilized as lipid motifs that are covalently attached to a peptide inhibitor via ester, ether, amide or carbamate bonds. Examples of peptide lipidation are described in Kowalczyk et al. 2017.

### Pharmaceutical compositions

Functionally active antagonists of c-Jun of the present invention may be useful in inhibiting c-Jun in a therapeutic setting. Thus, the c-Jun antagonist peptides of the invention may be formulated in a pharmaceutical composition.

A pharmaceutical composition is a formulation comprising one or more active agents (e.g. the c-Jun antagonist peptides or conjugates described herein) and one or more pharmaceutically acceptable excipients. The pharmaceutical composition may be capable of eliciting a therapeutic effect.

A pharmaceutical composition may comprise the c-Jun antagonist peptide or conjugate of the invention and a pharmaceutically acceptable excipient or carrier.

A method of making a pharmaceutical composition may comprise; admixing a c-Jun antagonist peptide or conjugate as described above with a pharmaceutically acceptable excipient.

The term "pharmaceutically acceptable" relates to compounds, materials, compositions, and/or dosage forms which are, within the scope of sound veterinary or medical judgement, suitable for use in contact with the tissues of a subject (e.g. human or other mammal) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable excipients and carriers include, without limitation, water, saline, buffered saline, phosphate buffer, alcoholic/aqueous solutions, emulsions or suspensions. Other conventionally employed diluents, adjuvants, and excipients may be added in accordance with conventional techniques. Such carriers can include ethanol, polyols, and suitable mixtures thereof, vegetable oils, and injectable organic esters. Buffers and pH-adjusting agents may also be employed, and include, without limitation, salts prepared from an organic acid or base. Representative buffers include, without limitation, organic acid salts, such as salts of citric acid (e.g., citrates), ascorbic acid, gluconic acid, carbonic acid, tartaric acid, succinic acid, acetic acid, phthalic acid, Tris, trimethylamine hydrochloride, or phosphate buffers. Parenteral carriers can include sodium chloride solution, Ringer's dextrose, dextrose, trehalose, sucrose, lactated Ringer's, or fixed oils. Intravenous carriers can include fluid and nutrient replenishers, electrolyte replenishers, such as those based on Ringer's dextrose, and the like. Preservatives and other additives such as, for example, antimicrobials, antioxidants, chelating agents (e.g., EGTA; EDTA), inert gases, and the like may also be provided in the pharmaceutical carriers. The pharmaceutical compositions described herein are not limited by the selection of the carrier. The preparation of these pharmaceutically-acceptable compositions, from the above-described components, having appropriate pH, isotonicity, stability and other conventional characteristics, is within the skill of the art.

Suitable carriers, excipients, etc. may be found in standard pharmaceutical texts, for example, Remington's Pharmaceutical Sciences and The Handbook of Pharmaceutical Excipients, 4th edit., eds. R. C. Rowe et al, APhA Publications, 2003.

The term "carrier" refers to diluents, binders, lubricants and disintegrants. Those with skill in the art are familiar with such pharmaceutical carriers and methods of compounding pharmaceutical compositions using such carriers.

A pharmaceutical composition may conveniently be presented in unit dosage form and may be prepared by any methods well-known in the art of pharmacy. Such methods include the step of bringing the peptide into association with a carrier or excipient as described above which may constitute one or more accessory ingredients. In general, the formulations are prepared by uniformly and intimately bringing into association the active compound with liquid carriers or finely divided solid carriers or both.

Pharmaceutical compositions described herein may be produced in various forms, depending upon the route of administration. The pharmaceutical compositions may be prepared for administration to subjects in the form of, for example, liquids, powders, aerosols, tablets, capsules, enteric-coated tablets or capsules, or suppositories. Pharmaceutical compositions may also be in the form of suspensions, solutions, emulsions in oily or aqueous vehicles, pastes, and implantable sustained-release or biodegradable formulations. Compositions for sustained release or implantation may comprise pharmaceutically acceptable polymeric or hydrophobic materials, such as an emulsion, an ion exchange resin, a sparingly soluble polymer, or a sparingly soluble salt. Pharmaceutical compositions may be made in the form of sterile aqueous solutions or dispersions, suitable for injectable use, or made in lyophilized forms using freeze-drying techniques. Lyophilized pharmaceutical compositions are typically maintained at about 4°C, and can be reconstituted in a stabilizing solution, e.g., saline or HEPES, with or without adjuvant. Pharmaceutical compositions can also be made in the form of suspensions or emulsions.

Pharmaceutical compositions may be presented in unit-dose or multi-dose sealed containers, for example, ampoules and vials, and may be stored in a freeze-dried (lyophilised) condition requiring only the addition of the sterile liquid carrier, for example water for injections immediately prior to use.

The pharmaceutical composition may be administered to a subject by any convenient route of administration. In some embodiments, administration is by systemic routes, including oral, or more preferably parenteral routes. For example, the pharmaceutical composition may be administered by intravenous, intraperitoneal or subcutaneous injection.

### Treatment of diseases

c-Jun plays a role in many cellular processes such as differentiation, proliferation, and survival and dysregulation of this transcription factor can therefore lead to a range of human diseases. Accordingly, a c-Jun antagonist peptide, nucleic acid, conjugate, or pharmaceutical composition as described herein may be for use in a method of treatment of the animal or human body, for example a c-Jun-mediated disease in an individual in need thereof.

An individual with a c-Jun-mediated disease may display at least one identifiable sign, symptom, or laboratory finding that is sufficient to make a diagnosis of a c-Jun-mediated disorder in accordance with clinical standards known in the art. Examples of such clinical standards can be found in textbooks of medicine such as Harrison's Principles of Internal Medicine, 15th Ed., Fauci AS et al., eds., McGraw-Hill, New York, 2001. In some embodiments, the individual may have been previously identified or diagnosed with a c-Jun-mediated disorder or a method of the invention may comprise identifying or diagnosing the presence of a c-Jun-mediated disorder in the individual, prognosing a c-Jun-mediated disorder or assessing the risk of onset of a c-Jun-mediated disorder in the individual.

Treatment may be any treatment and therapy, whether of a human or an animal (e.g. in veterinary applications), in which some desired therapeutic effect is achieved, for example, the inhibition or delay of the progress of the c-Jun-mediated disease, and includes a reduction in the rate of progress, a halt in the rate of progress, amelioration of the c-Jun-mediated disease, cure or remission (whether partial or total) of the c-Jun-mediated disease, preventing, delaying, abating or arresting one or more symptoms and/or signs of the c-Jun-mediated disease or prolonging survival of a subject or patient beyond that expected in the absence of treatment.

Treatment as a prophylactic measure (i.e. prophylaxis) is also included (e.g. treatment before the onset of a condition in an individual to reduce the risk of the condition occurring in the individual; delay its onset; or reduce its severity after onset). For example, an individual susceptible to or at risk of the occurrence or re-occurrence of a c-Jun-mediated disease, such as cancer may be treated as described herein. Such treatment may prevent or delay the occurrence or re-occurrence of a c-Jun-mediated disease or one or more symptoms thereof in the individual.

The c-Jun antagonist peptide may be used in a method of treatment of any one of the following diseases: cancer, diabetes, cardiovascular disease, autoimmune disease, joint disorders (such as arthritis), and neurodegenerative disease.

A "cancer" can comprise any one or more of the following: acute lymphocytic leukemia (ALL), acute myeloid leukemia (AML), adrenocortical cancer, anal cancer, bladder cancer, blood cancer, bone cancer, brain tumor, breast cancer, cancer of the female genital system, cancer of the male genital system, central nervous system lymphoma, cervical cancer. childhood rhabdomyosarcoma, childhood sarcoma, chronic lymphocytic leukemia (CLL), chronic myeloid leukemia (CML), colon and rectal cancer, colon cancer, endometrial cancer, endometrial sarcoma, esophageal cancer, eye cancer, gallbladder cancer, gastric cancer, gastrointestinal tract cancer, hairy cell leukemia, head and neck cancer, hepatocellular cancer, Hodgkin's disease, hypopharyngeal cancer, Kaposi's sarcoma, kidney cancer, laryngeal cancer, leukemia, leukemia, liver cancer, lung cancer, malignant fibrous histiocytoma, malignant thymoma, melanoma, mesothelioma, multiple myeloma, myeloma, nasal cavity and paranasal sinus cancer, nasopharyngeal cancer, nervous system cancer, neuroblastoma, non-Hodgkin's lymphoma, oral cavity cancer, oropharyngeal cancer, osteosarcoma, ovarian cancer, pancreatic cancer, parathyroid cancer, penile cancer, pharyngeal cancer, pituitary tumor, plasma cell neoplasm, primary CNS lymphoma, prostate cancer, rectal cancer, respiratory system, retinoblastoma, salivary gland cancer, skin cancer, small intestine cancer, soft tissue sarcoma, stomach cancer, stomach cancer, testicular cancer, thyroid cancer, urinary system cancer, uterine sarcoma, vaginal cancer, vascular system, Waldenstrom's macroglobulinemia and Wilms' tumor.

Cancers may be of a particular type. Examples of types of cancer include astrocytoma, carcinoma (e.g. adenocarcinoma, hepatocellular carcinoma, medullary carcinoma, papillary carcinoma, squamous cell carcinoma), glioma, lymphoma, medulloblastoma, melanoma, myeloma, meningioma, neuroblastoma, sarcoma (e.g. angiosarcoma, chrondrosarcoma, osteosarcoma).

In some embodiments, the individual may have minimal residual disease (MRD) after an initial cancer treatment.

Cancer treatment may include inhibiting cancer growth, including complete cancer remission, and/or inhibiting cancer metastasis. Cancer growth generally refers to any one of a number of indices that indicate change within the cancer to a more developed form. Thus, indices for measuring an inhibition of cancer growth include a decrease in cancer cell survival, a decrease in tumour volume or morphology (for example, as determined using computed tomographic (CT), sonography, or other imaging method), a delayed tumour growth, a destruction of tumour vasculature, improved performance in delayed hypersensitivity skin test, an increase in the activity of T cells, and a decrease in levels of tumour-specific antigens.

In some embodiments, a c-Jun antagonist peptide may be useful in inhibiting or reducing the metastasis of a cancer. For example, a method of reducing or inhibiting metastasis in an individual with cancer may comprise administering therapeutically effective amounts of a c-Jun peptide to the individual

An individual suitable for treatment as described above may be a mammal, such as a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orang-utan, gibbon), or a human.

In some preferred embodiments, the individual is a human. In other preferred embodiments, non-human mammals, especially mammals that are conventionally used as models for demonstrating therapeutic efficacy in humans (*e.g*. murine, primate, porcine, canine, or rabbit animals) may be employed.

Methods according to the present invention may be performed, or products may be present, *in vitro, ex vivo,* or *in vivo.* The term *"in vitro"* is intended to encompass experiments with materials, biological substances, cells and/or tissues in laboratory conditions or in culture whereas the term *"in vivo"* is intended to encompass experiments and procedures with intact multi-cellular organisms. *"Ex vivo"* refers to something present or taking place outside an organism, e.g. outside the human or animal body, which may be on tissue (e.g. whole organs) or cells taken from the organism.

Where the method is performed in vitro it may comprise a high throughput screening assay. Test compounds used in the method may be obtained from a synthetic combinatorial peptide library, or may be synthetic peptides or peptide mimetic molecules.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Sequences

| **SEQ ID NO.** | **Description** | **Sequence** | |
|---|---|---|---|
| 1 | Extended hinge region | LV[X₁]EE[X₂][X₃]LE[X₄]E | |
| | | | X₁ is selected from V, D, K, C and R, |
| | | | X₂ is selected from D, K, C, and R, |
| | | | X₃ is selected from V, D, C, K and R, |
| | | | X₄ is selected from E, D, C, K and R. |
| 2 | N terminal acidic extension | EA[X₅][X₆] | |
| | | | X₅ is selected from E, K or C, and |
| | | | X₆ is selected from E or D. |
| 3 | N terminal acidic extension | EAEE | |
| 4 | LZ region | IEQLEERNYALRKEIEDLQKQ | |
| 5 | LZ region | IEQLEEKNKALKDEIEDLQKQ | |
| 6 | LZ region | IKQLEDRNYALRKEIEDLQKQ | |
| 7 | LZ region | IEQLEERNYALRKEIKDLQDQ | |
| 8 | LZ region | IEQLEEKNKALKDEIEDLY | |
| 9 | LZ region | IEQLEERNYALRKEIEDLQ | |
| 10 | HingeW peptide variant 22/23 | EAEELVVEEDVLEEEIEQLEERNYALRKEIKDLQDQ | |
| 11 | HingeW peptide variant 24 (HW30) | EAEELVVEEKVLEDEIEQLEERNYALRKEIKDLQDQ | |
| 12 | HingeW variant | LEQRAEELARENEELEKEAEELVVEEDVLEEEIEQLEERNYA LRKEIEDLQKQLEKL | |
| 13 | HingeW peptide variant 2 | LARENEELEKEAEELVVEEDVLEEEIEQLEERNYALRKEIEDL QKQLEKL | |
| 14 | HingeW peptide variant 3 | LEKEAEELVVEEDVLEEEIEQLEERNYALRKEIEDLQKQLEKL | |
| 15 | HingeW variant peptide 4 | EAEELVVEEDVLEEEIEQLEERNYALRKEIEDLQKQLEKL | |
| 16 | HingeW variant peptide 7/8 | LVVEEDVLEEEIEQLEEKNKALKDEIEDLQKQLEKLY | |
| 17 | HingeW variant peptide 9/10 | LVVEEDVLEEEIEQLEERNYALRKEIEDLQKQLEDL | |
| 18 | HingeW variant peptide 11 | EAEELVVEEDVLEEEIEQLEERNYALRKEIEDLQKQ | |
| 19 | HingeW variant peptide 12/13 | KEAEDLVVEEDVLEEEIEQLEERNYALRKEIKDLQDQ | |
| 20 | HingeW variant peptide 14/15 | EAKELVDEEDVLEEEIEQLEERNYALRKEIEDLQKQ | |
| 21 | HingeW variant peptide 16/17 | EAEELVVEEKVLEDEIEQLEERNYALRKEIEDLQKQ | |
| 22 | HingeW variant peptide 18/19 | EAEELVVEEDVLEEEIKQLEDRNYALRKEIEDLQKQ | |
| 23 | HingeW variant peptide 20/21 | EAEELVVEEDVLEEEIEQLEEKNKALKDEIEDLQKQY | |
| 24 | HingeW variant peptide 25 | EAEELVVEEDVLEEEIEQLEERNYALRKEIEDL | |
| 25 | HingeW variant peptide 26/27 | EAEELVVEEDVLEEEIEQLEEKNKALKDEIEDLY | |
| 26 | HingeW variant peptide 28/29 | EAEEL VVEEDVLEEEI EQLEERNY ALRKEI EDLQ | |
| 27 | LZ region | IEQLEERNYALRKEICDLQCQ | |
| 28 | HingeW variant peptide | EAEEL VVEEDVLEEEI EQLEERNY ALRKEICDLQCQ | |
| 29 | LZ region | IEQLEERNYALR[X₇]E[X₈]K[X₉]L[X₁₀]D[X₁₁] | |
| | | wherein [X₇] is K, L, S, W, P, Q, R, M, T, V, A, E, or G; [X₈] is I, V, or L; [X₉] and [X₁₀] are any amino acid residue; [X₁₁] is Q, E, or K. | |
| 30 | LZ region | | IEQLEERNYALR[X₇]E[X₈]C[X₉]L[X₁₀]C[X₁₁] |
| | | | wherein [X₇] is K, L, S, W, P, Q, R, M, T, V, A, E, or G; |
| | | | [X₈] is I, V, or L; |
| | | | [X₉] and [X₁₀] are any amino acid residue; |
| | | | [X₁₁] is Q, E, or K. |
| 31 | Extended hinge region | | LVVEEDVLEEE |
| 32 | Extended hinge region | | LVVEEKVLEDE |
| 33 | Extended hinge region | | LVKEEDDLEEE |
| 34 | Extended hinge region | | LVVEECVLECE |
| 35 | Extended hinge region | | LVCEEDCLEEE |
| 36 | Extended hinge region | | LVCEEDVLECE |
| 37 | HingeW variant 0W | | EAEEL VVEEDVLEEEI EQLEERNY ALRSEI CSLQCQ |
| 38 | Hinge W variant ortho DBMBW | | EAEELVVEEDVLEEEIEQLEERNYALRKEICELSCQ |
| 39 | HingeW variant meta DBMBW | | EAEELVVEEDVLEEEIEQLEERNYALRAEICNLSCQ |
| 40 | HingeW variant para DBMBW | | EAEELVVEEDVLEEEIEQLEERNYALRTEICSLMCK |
| 41 | tddDBMBW | | EAEEL VVEEDVLEEEI EQLEERNY ALRAEI CSLQCQ |
| 42 | cJun LZ | | MASIARLEEKVKTLKAQNYELASTANMLREQVAQLGAPHHH HHH |
| 43 | cJun bZIP | | RIKAERKRMRNRIAASKCRKRKLERIARLEEKVKTLKAQNYE LASTANMLREQVAQLGAP |
| 44 | cFos LZ | | MASTDTLQAETDQLEDEKYALQTEIANLLKEKEKLGAPHHHH HH |
| 45 | cFos bZIP | | EEKRRIRRERNKMAAAKCRNRRRELTDTLQAETDQLEDEKY ALQTEIANLLKEKEKLGAP |
| 46 | FosW | | MASLDELQAEIEQLEERNYALRKEIEDLQKQLEKLGAPHHHH HH |
| 47 | A-FosW | | MASLEQRAEELARENEELEKEAEELEQELDELQAEIEQLEER NYALRKEIEDLQKQLEKLGAPHHHHHH |
| 48 | HingeW | | MASLEQRAEELARENEELEKEAEELVVEEDVLEEEIEQLEER NYALRKEIEDLQKQLEKLGAPHHHHHH |
| 49 | cJun-Hinge-Lib-F primer | | GAAGAGCTCSWGSWGSWGSWGSWTSWGCTGSWGGMA SWGATTGAACAGCTGGAAGAACGCAACTATGCC |
| 50 | cJun-Hinge-Lib_R primer | | TGAGGCGCGCCCAGTTTCTCCAGCTGTTTCTGGAGGTCTT CGATCTCTTTGCGCAAGGCATAGTTGCGTTC |
| 51 | TRE Primer | | GTCAGTCAGTGACTCAATCGGTCA |
| 52 | Non-TRE primer | | CCTGCGTAGTTCCATAAGGATAGC |
| 53 | His-tag | | HHHHHH |
| 54 | tat | | PGRKKRRQRRPPQ |
| 55 | penetratin | | RQIKIWFQNRRMKWKK |
| 56 | transportan | | GWTLNSAGYLLGKINLKALAALAKKIL |
| 57 | VP-22 | | DAATATRGRSAASRPTERPRAPARSASRPRRPVD |
| 58 | Pep-1 | | KETWWETWWTEWSQPKKKRKV |
| 59 | MAP | | KALAKALAKALA |
| 60 | SAP | | VRLPPPVRLPPPVRLPPP |
| 61 | Oligoarginine | | RRRRRRRR |
| 62 | Oligoarginine | | RRRRRRRRR |
| 63 | calcitonin | | LGTYTQDFNKTFPQTAIGVGAP |
| 64 | SynB | | RGGRLSYSRRRFSTSTGR |
| 65 | Pvec | | LLIILRRRIRKQAHAHSK |
| 66 | LZ region 0W | | IEQLEERNYALRSEICSLQCQ |
| 67 | LZ region ortho DBMBW | | IEQLEERNYALRKEICELSCQ |
| 68 | LZ region meta DBMBW | | IEQLEERNYALRAEICNLSCQ |
| 69 | LZ region para DBMBW | | IEQLEERNYALRTEICSLMCK |
| 70 | LZ region tdd DBMBW | | IEQLEERNYALRAEICSLQCQ |
| 71 | Extended hinge region | | LVVEEKVLERE |
| 72 | c-Jun antagonist peptide variant | | EAEEL VVEEKVLEREI RRLERRNRALRKEI KDLQDQ |
| 73 | c-Jun antagonist peptide variant | | EAEELVVEEKVLEDEIERLERRNYRLRREIKDLQDQ |
| 74 | Modified LZ region | | IRRLERRNRALRKEIKDLQDQ |
| 75 | Modified LZ region | | IERLERRNYRLRREIKDLQDQ |

### Examples

Certain aspects and embodiments will now be illustrated by way of example and with reference to the figures described above.

### EXAMPLE 1 - Identification of functional peptide antagonists

Many rational design approaches, library screens, and selection systems exist and have resulted in the successful identification of molecules capable of binding to given TF targets, but a key challenge remains in ensuring that target binding will translate into ablation of function (Brennan et al., 2020; Baxter et al., 2014). Various methodologies have produced peptide-based c-Jun antagonists that target the broad LZ binding interface (Boysen et al., 2002; Mason et al., 2006; Kaplan et al., 2014; Baxter et al., 2017; Lathbridge et al., 2018). However, it is difficult to predict if LZ binding will translate into functional antagonism as the c-Jun DBD remains unbound and capable of binding TRE DNA (Seldeen et al., 2008; Szalóki N et al., 2015). A rationally designed peptide has been shown to target the cJun DBD but exhibits lower potency than LZ antagonists, with concerns over specificity due to high sequence similarity across the AP-1 family DBDs (Tsuchida et al 2004). Similarly, a range of SMs targeting TRE DNA have been developed (Dai et al., 2004; Fanjul et al., 1994) but these are also lower potency and have the potential to produce off-target effects since multiple TFs typically bind to any given DNA element, with some bZIP/DNA combinations known to promote anti-oncogenic outcomes (Eferl et al., 2003; Rodriguez-Martinez JA et al., 2017). One approach to circumvent the potential downsides of these methods is to utilise longer peptides that target the full c-Jun bZIP domain with a selective yet high affinity interaction, simultaneously blocking both DNA binding and LZ dimerisation. Olive et al. took this approach to produce A-Fos, which combined the wild-type (WT) cFos LZ (known to heterodimerise with c-Jun) and a rationally designed Glu-rich acidic extension (**Figure 1**) (Olive et al., 1997). The A-Fos design principle postulated that the LZ interaction is extended N-terminally generating a DBD-acidic extension interaction facilitated by the incorporation of Leu residues into putative d positions in the acidic extension. An intracellular Transcription Block Survival (TBS) library screening assay was developed and validated to search for functional TF antagonists, where cell survival only occurs when TF activity is abolished. Further, bacterial growth rates are correlated with antagonist efficiency allowing for comparison and competition between TF antagonists. Here we showcase the approach using a large peptide library (131,027 members), demonstrating that they can be screened within the TBS platform for functional c-Jun antagonism. The selected peptide is validated using a range of biophysical approaches indicating a clear improvement from the parent peptide in target binding and c-Jun/TRE DNA antagonism that is particularly facilitated by a reduction in homodimeric stability. The following methods were used:

### 1.1 Methods

**Plasmid Constructs and Protein Production:** The TRE-mDHFR **(****Figure 2****)** and WT-mDHFR DNA constructs were subcloned into pQE16 derivative plasmid pES300d; the c-Jun LZ and c-Jun bZIP DNA constructs were subcloned into pQE16 derivative plasmid pES230d; and the cFos LZ and A-FosW DNA constructs were subcloned into pET24a. The human c-Jun bZIP domain spans from Arg²⁵² to Leu³⁰⁸ and the LZ domain spans from Ile²⁷⁷ to Leu³⁰⁸. The human cFos bZIP domain spans from Glu¹³⁷ to Leu¹⁹³ and the LZ domain spans from Thr¹⁶² to Leu¹⁹³. A-FosW has the following sequence: LEQRAEELARENEELEKEAEELEQE**LDELQAEIEQLEERNYALRKEIEDLQKQLEKL** (FosW sequence in bold). All constructs are capped at the N-terminus with AS residues and at the C-terminus with GAP residues and are also 6xHis-tagged, other than the WT- or TRE-mDHFR constructs which are only 6xHis-tagged. A full list of sequences is provided in **Table 1.**

**Table 1: Prior art and Hinge W peptide sequences**

| **Peptide** | **Sequence** |
|---|---|
| cJun LZ | MASIARLEEKVKTLKAQNYELASTANMLREQVAQLGAPHHHHHH (SEQ ID NO: 42) |
| cJun bZIP | RIKAERKRMRNRIAASKCRKRKLERIARLEEKVKTLKAQNYELASTANMLREQVAQLGAP (SEQ ID NO: 43) |
| cFos LZ | MASTDTLQAETDQLEDEKYALQTEIANLLKEKEKLGAPHHHHHH (SEQ ID NO: 44) |
| cFos bZIP | EEKRRIRRERNKMAAAKCRNRRRELTDTLQAETDQLEDEKYALQTEIANLLKEKEKLGAP (SEQ ID NO: 45) |
| FosW | MASLDELQAEIEQLEERNYALRKEIEDLQKQLEKLGAPHHHHHH (SEQ ID NO: 46) |
| A-FosW | MASLEQRAEELARENEELEKEAEELEQELDELQAEIEQLEERNYALRKEIEDLQKQLEKL GAPHHHHHH (SEQ ID NO: 47) |
| HingeW | MASLEQRAEELARENEELEKEAEELVVEEDVLEEEIEQLEERNYALRKEIEDLQKQLEKL GAPHHHHHH (SEQ ID NO: 48) |

Proteins were purified by subcloning their DNA sequences into either a pET21-His-SUMO plasmid (cJun bZIP, cFos bZIP) or a pET24a plasmid (HingeW, A-FosW, FosW) using Nhel and Ascl sites. An overnight culture of E. *coli* containing the relevant plasmid was used to inoculate LB media at a dilution factor of 1:1000. This culture was incubated with shaking (37°C, 200 rpm) until the OD₆₀₀ₙₘ reached 0.7. Protein over-expression was induced by the addition of IPTG (1 mM) before incubation with shaking (25°C, 200 rpm) overnight. Cells were then harvested from the culture by centrifugation. Cell pellets were resuspended in Histrap Binding Buffer (20 mM potassium phosphate, 500 mM NaCl, 40 mM imidazole, 5 mM DTT, pH 7.4), sonicated and loaded on a HisTrap HP 5 mL pre-loaded column. The column was washed with Binding Buffer before eluting protein samples on a Binding Buffer:Elution Buffer (20 mM potassium phosphate buffer, 500 mM NaCl, 400 mM imidazole, 5 mM DTT, pH 7.4) gradient. This methodology was also used to produce a ~80% pure sample of His-tagged ULP1 protease for use in the SUMO cleavage step. SUMO-tagged proteins were buffer exchanged into Standard Buffer (20 mM Tris.HCl, 2 mM DTT, pH 8.0). A 10:1 mixture of SUMO-tagged protein:ULP1 was incubated at 30°C for 16h. As the SUMO-tagged construct was N-terminally His-tagged on the SUMO, the cleavage reaction was diluted 1 in 5 in Binding Buffer and then passed through the HisTrap column to remove the cleaved SUMO tag and the His-tagged ULP1. The HisTrap flowthrough was finally purified to >98% purity by using RP-HPLC with a Jupiter Proteo column (4-µm particle size, 90 Å pore size, 250 × 10 mm; Phenomenex) using a water:acetonitrile gradient (0.1% TFA). Peptides without a SUMO tag, were concentrated after Histrap elution and HPLC purified. Peptide purity and identity were verified by SDS-PAGE and electrospray ionisation mass spectrometry.

**DHFR Activity Assay:** A colorimetric assay kit (Sigma CD0340) was used to measure the activity of purified DHFR enzymes. WT- or TRE-mDHFR (100 nM in reaction) and NADPH (60 µM in reaction) were mixed in assay buffer only, or with DHFR inhibitors TMP or Mtx (1 µM in reaction). Reactions were initiated by the addition of DHF (50 µM in reaction plus a blank reaction with no DHF) and the absorbance at 340 nm of samples was measured using a Varian Cary 50 UV-Vis spectrophotometer. The specific activity was calculated using the following equation. $Specific activity = \frac{\left(ΔOD/{min}_{sample}-ΔOD/{min}_{blank}\right)}{12.3 \times mg protein}$

**Library Construction and TBS Assay:** Library inserts were produced using PCR fill-in reactions from synthesised primers (Sigma) with degenerate codons at the desired positions to produce the correct residue options. The library was subcloned using Sacl and Ascl sites into the pET24a plasmid containing A-FosW. The primers used were cJun-Hinge-Lib-F: 5'-GAAGAGCTCSWGSWGSWGSWGSWTSWGCTGSWGGMASWGATTGAACAGCTGGAAGAACGCAAC TATGCC-3' (SEQ ID NO: 49) and cJun-Hinge-Lib_R: 5'-TGAGGCGCGCCCAGTTTCTCCAGCTGTTTCTGGAGGTCTTCGATCTCTTTGCGCAAGGCATAGTTGC GTTC-3' (SEQ ID NO: 50). The library DNA was transformed into NEB 10-beta electrocompetent E. *coli* cells. The following equation was utilised to determine library coverage by the number of single colonies: $E = 100 \times {\left(1-\frac{1}{n}\right)}^{m}$ where E is the percentage of the library missing, m is the number of colonies collected and n is the library size. This showed that from 2155000 library colonies collected, 99.9% of the Hinge library was covered. Library DNA quality was assessed by sequencing both the DNA pool and a number of single colonies to show degenerate codons in the correct positions in the pool and to show a diversity of library members from single colonies. The pool of library DNA was transformed into BL21 Gold cells already containing pES300d-TRE-mDHFR and pES230d-cJun bZIP.

Selective pressure is applied by growing the bacteria in M9 minimal media with TMP (2-4 µM) alongside ampicillin, kanamycin and chloramphenicol to maintain the required plasmids, and IPTG (1 mM) to induce protein expression. The library transformants were first plated out onto selective agar plates (2 µM TMP) and grown at 37°C for 72-96h. Optimisation experiments **(****Figure 5****)** indicate that 4 µM TMP is optimum for selection however a lower stringency is used initially before selection is increased in later steps. Colonies from this first round of selection were pooled and serially grown in liquid culture at starting OD₆₀₀ of 0.05 and grown at 37°C with shaking at 200 rpm until the OD₆₀₀ reached 0.6. TMP concentration was 2 µM in the first liquid culture passage before it was increased to the optimum 4 µM in subsequent passages. Bacteria containing the most effective functional antagonists were expected to produce higher levels of TRE-mDHFR which provides a growth advantage, and these will dominate the culture. At each passage step, a sample of the culture was plated on LB agar (supplemented with antibiotics to maintain plasmids) to select and sequence individual colonies, and a DNA pool was also sequenced. This allows the occurrence of library members to be monitored as winner sequences are selected for. Assay Validation experiments utilised a modified assay methodology whereby overnight cultures from glycerol stocks of the Control Strains **(Table 2)** were diluted to OD₆₀₀=0.5 and 50 µL was plated on a selective M9 minimal medium agar plate.

**Table 2: TBS assay validation E.Coli control strains showing the inserts expressed from each plasmid. Strains for growth experiments are plasmid matched to ensure equal antibiotic pressure, using scrambled protein sequences in unrequired plasmids.**

| | **pES300d** | **pES230d** | **pET24a** |
|---|---|---|---|
| 1 | WT-mDHFR | Scrambled (cJun bZIP) | Scrambled (A-FosW) |
| 2 | WT-mDHFR | cJun bZIP | Scrambled (A-FosW) |
| 3 | TRE-mDHFR | Scrambled (cJun bZIP) | Scrambled (A-FosW) |
| 4 | TRE-mDHFR | cJun LZ | Scrambled (A-FosW) |
| 5 | TRE-mDHFR | cJun bZIP | Scrambled (A-FosW) |
| 6 | TRE-mDHFR | cJun bZIP | cFos LZ |
| 7 | TRE-mDHFR | cJun bZIP | FosW |
| 8 | TRE-mDHFR | cJun bZIP | A-FosW |
| 9 | TRE-mDHFR | cJun bZIP | HingeW |

**Circular Dichroism (CD):** An Applied Photophysics Chirascan was used for CD measurements, with a 200 µL sample in a 1 mm path length CD cell. Protein/DNA samples were suspended in 150 mM potassium phosphate, 150 mM potassium fluoride and 5 mM TCEP at pH 7.4 and were equilibrated for 30 minutes before measurement. For full spectra, three scans between 190 and 260 nm (265-320 nm for DNA binding experiments) were collected with a bandwidth of 1 nm and data sampled at a rate of 0.5 s⁻¹. These scans were averaged and converted to molar residue ellipticities (MRE). Thermal denaturation experiments were performed by measuring the ellipticity at 222 nm over a 1 to 90°C gradient at 1°C increments. Post-melt scans at 20°C confirmed the transitions were reversible as they overlaid within 10% of the pre-melt scan. The resulting thermal denaturation curves were converted to MRE and fitted to a two-state model, derived *via* modification of the Gibbs-Helmholtz equation to determine the melting temperature (*Tₘ*) (Mason et al., 2007).

**Isothermal Titration Calorimetry (ITC):** Peptides were studied in an ITC buffer consisting of 10 mM potassium phosphate, 150 mM potassium fluoride and 5 mM TCEP at pH 7.4. Using a MicroCal VP-ITC instrument (Malvern), 10 µL injections of antagonist peptide (HingeW or A-FosW) at 10 µM were injected into the cell containing cJun at 1 µM. MicroCal Origin software was used to record and analyse the heat change upon addition. Control experiments involved the injection of the antagonist peptide sample into the cell containing ITC buffer alone to determine the heat of dilution which was subtracted. The resulting binding data were fit to a one site binding model to extract the enthalpy change of binding (*ΔH*) and the equilibrium binding constant (*K_{D}*), from which the free energy change of binding (*ΔG*) and the entropy change of binding (Δ*S*) was calculated (Wiseman et al., 1989). Thermodynamic parameters are presented as an average of two independent experiments with errors given as one standard deviation.

**Electrophoretic Mobility Shift Assay (EMSA):** The following double-stranded oligonucleotide sequences were used, TRE: 5'-GTCAGTCAGTGACTCAATCGGTCA (SEQ ID NO: 51), control non-TRE: 5'-CCTGCGTAGTTCCATAAGGATAGC (SEQ ID NO: 52) (Sigma). Complementary single strands of DNA were purchased (Sigma) and mixed at a 1:1 ratio, then heated to 95°C for 20 minutes before cooling slowly to room temperature to form DNA duplexes. Protein/DNA samples for electrophoresis were incubated at 4°C for 30 minutes in binding buffer (150 mM KCI, 1 mM dithiothreitol, 1 mM EDTA, 10 mM Tris, 10 mM MgCl₂, pH 8) before running on a 1.3% agarose gel in 0.5xTBE buffer (supplemented with 10 mM MgCl₂). SYBR^{®} Green stain was included in the gel and running buffer to stain for DNA which was imaged on a transilluminator before SYPRO^{®} Ruby was added and incubated for 3 h to stain for protein. The gel was destained in a 10% methanol, 7% acetic acid solution for 1 h before imaging on a transilluminator.

### 1.2 Creation of an active mDHFR from a TRE-containing gene to facilitate a c-Jun imposed transcriptional block

Transcription block survival (TBS) is an intracellular assay that utilises cell survival as a readout. This allows protein-DNA interaction antagonists to be screened, and the most active identified by their ability to remove a transcriptional block on exogenous murine dihydrofolate reductase (DHFR). This enzyme is absolutely essential for survival since it is required for the production of purines needed for DNA and amino acid synthesis. Endogenous E.coli DHFR (ecDHFR) can be selectively inhibited by trimethoprim (TMP), meaning that cells grown in M9 minimal media are rendered dependent on exogenous murine DHFR (mDHFR) activity for their survival (Matthews et al., 1985). We produced a mDHFR gene **(****Figures 2** **and** **3****)** by rational design to introduce 15 TRE sites into the coding DNA sequence to allow a robust cJun transcriptional block whilst minimising alteration to the expressed protein (TRE-mDHFR). In particular, the resulting TRE-mDHFR construct was produced via two silent and thirteen conservative mutations. Using the WT-mDHFR crystal structure as a design guide (PDB code: 1U72) (Cody et al., 2005), no changes were made in residues deemed important for 7,8-dihydrofolate (DHF) substrate or nicotinamide adenine dinucleotide phosphate (NADPH) cofactor binding (e.g., A10, L23, W25 and R71) (Cody et al. 2005; Thillet et al. 1988). The accessible surface areas of all other amino acid residues were calculated using the 'Accessible Surface Area and Accessibility Calculation for Protein' tool (http://cib.cf.ocha.ac.jp/bitool/ASA/). A cut-off score of 20 Å2 was implemented, below which residues were deemed to be buried from solvent exposure and therefore more likely to cause disruption if changed. Of the remaining non-essential, surface exposed residues, mutations were only permissible where the R group change was relatively conservative. The precise permitted substitutions included to incorporate TRE sites were as follows: F32S, T40Q, S42D, G46S, K64S, R78Q, Q103S, M112S, N127T, R138Q, L154S, Y163S and E169Q.

### 1.3 Establishing a transcription block survival assay

We first sought to confirm whether the new TRE-mDHFR construct could replace the TMP-inhibited ecDHFR by confirming it expresses, folds, and is catalytically active. This was achieved via ***i)*** SDS-PAGE analysis of cell lysate, confirming that the protein is expressed within the soluble fraction upon isopropyl β-D-1-thiogalactopyranoside (IPTG) induction **(****Figure 4****), *ii)*** plating E. *coli* containing the TRE-mDHFR plasmid onto M9 agar supplemented with TMP; no growth was observed (4 µM TMP, optimised in Figure 5), with growth restored upon induction of TRE-mDHFR expression by IPTG **(****Figures 5** **and** **8B****-3),** and ***iii)*** purified recombinant WT- and TRE-mDHFR activity was monitored by following the reduction of NADPH at 340 nm in the presence of the DHF substrate **(****Figure 3****).** The specific activities calculated from these reactions demonstrated a 24-fold reduction in activity for TRE-mDHFR relative to WT. In addition, TRE-mDHFR showed a ~1.8-fold reduction in specific activity in the presence of TMP whereas WT-mDHFR was unaffected. Despite an expected reduction in activity resulting from the thirteen amino acid substitutions, TRE-mDHFR retained its ability to turnover DHF and impart survival (while ecDHFR is compromised), confirming its suitability for TBS.

Having established that TRE-mDHFR is active and absolutely required for cell survival under selective conditions (M9 minimal media + 4 µM Tmp + 1 mM IPTG), we next expressed the cJun bZIP domain in cells containing the TRE-mDHFR plasmid, which resulted in a 21-fold reduction in colony counts (P ≤ 0.0001; **Figure 8B-5****).** Expression of cJun bZIP in the presence of WT-mDHFR (i.e. lacking the requisite TRE binding sites) reduced bacterial proliferation (P ≤0.05, **Figure 8B-1** vs. **8B-2).** This is presumably due to overexpressed cJun binding non-specifically to the plasmid DNA. However, the transcription block is strongly TRE site specific as indicated by a 1.3-fold reduction without TRE sites. As a further control, we also introduced a cJun LZ only construct, in which the 25 residue DBD was deleted. This peptide was unable to initiate DNA binding and, as expected, did not affect bacterial colony formation (P = 0.1, Figure **8B-4** vs. **8B-3).** Taken together, this specifically correlates the interaction between the cJun bZIP and TRE sites with ablation of bacterial growth within the TBS system, validating that any subsequent increase in bacterial growth is due to inhibition of this interaction.

Next, peptides known to bind to cJun were introduced into the system, to establish whether they can impact upon cJun function - i.e. sequester the cJun bZIP as a non-functional heterodimer therefore preventing DNA-binding and rescuing TRE-mDHFR transcription. Here, we used two peptides targeting the cJun LZ domain: cFos LZ and FosW, an optimised sequence identified from a protein-fragment complementation assay (PCA) that readily binds to cJun in the absence of DNA at nM affinity (Mason et al., 2006; Worral et al., 2011). Despite their known interactions with cJun, both peptides were shown to be ineffective in restoring TRE-mDHFR expression and activity, producing no significant increase in colony numbers from the transcriptionally blocked cells (P > 0.05 in both cases, **Figure 8B-6** **or** **8B-7** **vs.** **8B-5****).** This important finding demonstrates that although FosW can outcompete the cJun dimer to form a non-functional heterodimer, it is unable to free DNA-bound cJun from TRE sites within TRE-mDHFR.

To address this we turned to work by Olive et al., in which antagonism of cJun was achieved using Acidic-cFos (A-Fos) whereby a rationally designed acidic extension was appended to the cFos LZ (Olive et al., 1997). Since FosW was shown to improve binding to the cJun LZ relative to the WT cFos LZ sequence in the absence of DNA (Mason et al., 2006), an improved hybrid construct was rationally designed. This blended the two previously published components by appending the rationally designed acidic extension to the N-terminus of the FosW LZ sequence to generate A-FosW **(****Figure 9****).** This protein was designed to act as a template for peptide library design and optimisation using TBS screening. Reassuringly the template peptide was able to successfully antagonise the cJun/TRE DNA interaction, restoring 60% of the colony numbers relative to TRE-mDHFR only **(****Figure 8B-8** vs. **8B-5**). Importantly, all experimental variations above were plasmid-matched with appropriate dummy constructs to control for potential differences in antibiotic stress **(Table 3).** TBS design is summarised in **Figure 8A**.

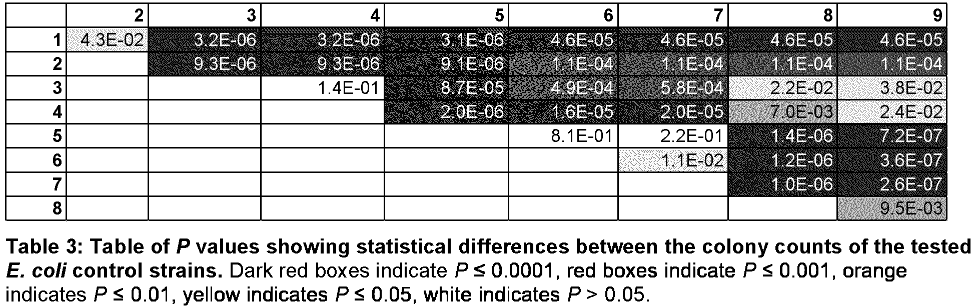

### 1.4 Hinge library design

The acidic extension design principle is the most successful methodology in the literature to target the full bZIP domain of various proteins (Olive et al., 1997; Ahn et al., 1998; Chen et al., 2011). However, incomplete restoration of colonies using A-FosW indicated that transcription remained partially hampered by cJun binding across the 15 TRE sites. This allowed us to employ TBS to screen a peptide library, using A-FosW as a design template, towards further improvement in cJun/TRE DNA antagonism. The library design utilised semi-randomised positions within the hinge region that straddles the acidic extension and LZ domains **(****Figure 9**). It was anticipated that optimisation in the hinge region would induce a significant increase in functional antagonism with high-affinity binding simultaneously disrupting both cJun LZ dimerization and, by scrambling at the area between the two domains would provide a more effect block of the DBD-DNA interaction. Previous studies have generally sought to inhibit binding at either the DBD or LZ, but here we aim to target both regions to generate an improved antagonist versus targeting either domain separately. Of the nine semi-randomised hinge positions, four options were included across eight positions (e4, f4, g4, a4, b4, c4, e5, f5 and g5): two hydrophobic (V/L), one acidic (E or D) and one polar (Q or H). Options of A/E were included at f5 as an f position in the LZ region since these positions were deemed unlikely to be involved in direct target interaction, thus these residues were presented to assist with solubility or higher helical propensity to enhance the PPI by entropic preorganisation. Experimental limitations prevent more amino acid diversity from being incorporated at so many positions, however, these selections will allow testing for general amino acid preference. In all positions, parental residues of A-Fos were incorporated into the library so that this functionally active benchmark protein was included within the screen. Restriction from the genetic methodology used to produce the library result in different mixtures of residues at different positions. Leu at d5, in the middle of this stretch of semi-randomised positions, remained unchanged since alteration was deemed unlikely to be favourable, or may favour other coiled coil architectures than the desired parallel dimer. Altogether, this produced a peptide library of 131,072 members **(****Figure 9****).**

### 1.5 TBS library screening

During the TBS library screening process, *E. coli* were transformed with the pooled DNA plasmid library such that each cell expressed a given member. Cells were plated onto M9 selective media and incubated until colonies expressing TBS active library members had formed. These colonies were pooled and repeated liquid culture passages were undertaken under selective conditions to compete library members against each other, enriching for the most TBS active sequences. At each stage of the assay, DNA sequencing was used to monitor the presence of TBS active sequences in the culture and inform on which residues were being selected at each position until one discrete DNA sequence was detected in the culture, referred to as HingeW **(****Figure 9****).** The residues selected in the winning HingeW peptide were universally found to be either acidic (D/E) or hydrophobic (V). Six of nine residues selected deviated from the parental A-FosW sequence with both V (E-to-V at **e4** and **c4,** Q-to-V at **f4**) and E (L-to-E at **a4,** Q-to-E at **e5,** A-to-E at **f5**) newly selected in three positions. The shift in the proportion of library options at each position during competition rounds provides information on how favoured a particular residue or residue type may be as the selection progresses **(****Figure 10****).** Two residues were selected after the first passage and were therefore considered particularly important for optimisation of this interaction: V at **f4** (Q in the parent sequence) and E at **g4** (unchanged). At position **f5,** there was little preference for either option with E selected over A (A in the parent sequence) at the end of the second passage, indicating less impact on target binding. Although the sequence for A-FosW was included within the library, further confirmation of the TBS selection preference for HingeW over the parental sequence was undertaken by direct competition in liquid culture. In this experiment equal numbers of TBS cells containing either A-FosW or HingeW were mixed in selective M9 media and subjected to competition selection. After three passages only HingeW was observed via DNA sequencing, as the A-FosW-containing cells had been outcompeted. This was further supported in TBS colony counting experiments which showed a 10% increase in colony numbers for HingeW relative to A-FosW (*P* = 0.009, **Figure 8B-9** **vs. 8B-8),** rising to 66% of the theoretical maximum colony numbers observed for TRE-mDHFR alone **(****Figure 8B-3****).**

### 1.6 HingeW binds c-Jun preferentially over A-FosW

Experiments were next undertaken to compare the binding of A-FosW and TBS-optimised HingeW to the cJun bZIP. CD spectroscopy was utilised to measure the global secondary structure of homo- and heterodimeric peptide samples, providing information on overall α-helicity and thermal melting temperatures (Tm). The HingeW/cJun spectrum was 82% higher in α-helical content relative to the average of the two component spectra expected for no interaction **(****Figure 11A****).** The same, though smaller, trend was observed from the A-FosW/cJun spectra where the α-helicity was 39% greater than the average **(****Figure 11B****).** The larger α-helical gain upon binding for HingeW/cJun implies a higher affinity interaction. Of note is that HingeW in isolation is 12.9% less helical relative to A-FosW. The AGADIR helical propensity calculator was used to calculate predicted helicity scores of 14.5 and 13.2 for A-FosW and HingeW respectively (Muñoz et al. 1994). The observed difference in heterodimeric peptide helicity may be partially explained by A-FosW being inherently more helical but the larger scale of the observed effect than this prediction can likely be explained by a homodimeric preference for A-FosW relative to HingeW.

Thermal denaturation analysis of HingeW/cJun, following the loss of signal at 222 nm, displayed a Tm of 71.2°C for the HingeW/cJun heterodimer, representing a clear increase from the Tm of the two component denaturation profiles **(****Figure 11C****).** For A-FosW/cJun, the Tm was observed as 69.9°C **(****Figure 11D****).** Although this represented a negligible 1.3°C increase in heterodimer Tm, there was crucially a much larger ΔTm for HingeW/cJun relative to the component peptide denaturation profiles than for A-FosW/cJun. The low thermal stability of the HingeW homodimer results in no observable lower baseline prior to the transition such that the Tm for this component, and thus the average, cannot be determined. However, this ΔTm can be estimated to be ~40°C, compared to 27.5°C for A-FosW/cJun. The TBS screen has therefore led to an optimised reduction in homodimersation more so than increased heterodimerisation with the target. This ensures that more antagonist is available as free monomer in solution and therefore in a target-dimerisation competent state. Another difference between the two denaturation profiles is the presence of a double transition for the A-FosW/cJun heterodimer, with a smaller initial transition occurring at ~30°C. Jain et al. have previously reported a double transition in similar acidic extension antagonist/bZIP denaturation profiles and suggest that the lower temperature transition occurs due to fraying of the N-terminal acidic extension/DBD interaction, with the higher temperature transition corresponding to dissociation of LZ regions (Jain P et al., 2017). Crucially, these two novel antagonists have significantly higher target heterodimer Tm values than FosW (Tm = 54°C, **Figure 12****).** This demonstrates a clear benefit from the inclusion of the acidic extension which is absent in FosW. Importantly, due to sequence differences between cJun and cFos **(****Figure 9****),** optimisation for cJun binding means that HingeW displays no interaction with the cFos bZIP domain **(****Figure 13****).**

### 1.7 HingeW outcompetes A-FosW for c-Jun binding

Direct competition between HingeW and A-FosW for cJun binding was observed using CD dimer exchange experiments in which a solution containing one antagonist/cJun mixture was combined with the other antagonist to observe potential changes in α-helicity, as an indicator of a change in cJun dimerisation partner. In this case, when HingeW was mixed with the preformed A-FosW/cJun heterodimer a 17% increase in helicity was observed, as measured at 222 nm, relative to the average, indicating a change in binding **(****Figure 11E****).** There was also a clear increase in the peak at 190 nm relative to the average of the two component peptide spectra. Reversing the experiment and mixing A-FosW with a preformed HingeW/cJun heterodimer produced a measured spectrum which overlaid with the average of the components, indicating that no dimer exchange had occurred **(****Figure 11F****).** In combination with TBS growth competition data, the dimer exchange experiments strongly suggest preferential binding of cJun to HingeW relative to A-FosW when in competition.

### 1.8 Isothermal Titration Calorimetry Demonstrates Improved Binding Affinity for HingeW

The binding interactions of cJun with HingeW and A-FosW were further studied by ITC, to provide information on the thermodynamic parameters. The data produced from the injection of HingeW into cJun **(****Figure 14A****)** were fit to a single site binding model (N=1.05 ± 0.05) with a KD of 14.4 ± 3.7 nM and a ΔH of -85.4 ± 4.5 KJ mol-1 (TΔS = -39.2 ± 4.5 KJ mol-1). A-FosW binding to cJun **(****Figure 14B****)** was also fit to a single site model (N=1.04 ± 0.08) with a KD of 88.3 ± 17.6 nM and a ΔH of -152.6 ± 4.1 KJ mol-1 (TΔS = -112.6 ± 4.1 KJ mol-1). This confirms the predicted 1:1 binding stoichiometry of both interactions while demonstrating a 6-fold increase in binding affinity upon TBS optimisation of A-FosW to HingeW. Both interactions are enthalpically driven with negative entropic contributions, with the latter less unfavourable for the HingeW interaction.

### 1.9 HingeW effectively antagonises the c-Jun/TRE DNA interaction

The binding of cJun to TRE DNA can be observed by monitoring a DNA absorbance peak in the CD spectrum centred at ~281 nm (John M et al., 1996). Peptides (cJun, HingeW or A-FosW) in isolation do not absorb at this wavelength meaning that all changes in the spectrum in this region correspond to shifts in DNA conformation. Addition of cJun (20 µM) to TRE DNA (5 µM) decreases this DNA peak by 55% as the cJun engages its target TRE site and alters the DNA structure **(****Figure 15A****).** Subsequent titration of HingeW into this bound cJun/TRE DNA mixture reverses the peak shift, with the peak increasing as DNA is released. This occurs in a dose dependent manner until the signal overlays with the free DNA spectrum at HingeW concentrations of 50 and 100 µM, indicating complete antagonism of the cJun/TRE interaction. Plotting and fitting the relative peak shifts to the Hill equation (OriginPro) yields an IC50 of 13.4 ± 0.6 µM which can be compared to the equivalent data for A-FosW antagonism which produces an IC50 of 16.0 ± 0.4 µM **(****Figure 15B****).** This shows significant improvement in both cases over FosW, which lacks an acidic extension, and displays an IC50 of 119.8 ± 1.1 µM **(****Figure 16****).** In control experiments, both HingeW and A-FosW were shown to have no interaction with DNA **(****Figure 17****).**

To provide further evidence of functional antagonism, an electrophoretic mobility shift assay (EMSA) was employed. Firstly, cJun bZIP (20 µM) was mixed with the TRE DNA construct (2 µM), resulting in a significant reduction in the free DNA band intensity relative to DNA alone **(****Figure 15C****).** No bound cJun/TRE DNA band was observed as the overall charge of this complex prohibited entry into the gel. Antagonism was therefore best observed by monitoring the intensity of the free DNA band. A concentration dependent increase in the free DNA band intensity was observed upon addition of HingeW to cJun/TRE DNA **(****Figure 15D****).** The same trend was observed for increasing concentrations of A-FosW with cJun/TRE DNA **(****Figure 15E****).** In close agreement with the CD DNA peak analysis, the data could be fit to the Hill equation (OriginPro) to determine an IC50 value of 9.6 ± 0.8 µM for HingeW and 12.1 ± 1.9 µM for A-FosW. In close agreement with the CD DNA peak analysis, the data could be fit to the Hill equation (OriginPro) to determine an IC50 value of 9.6 ± 0.8 µM for HingeW and 12.1 ± 1.9 µM for A-FosW **(****Figure 15F****).**

A summary of the thermodynamic parameters for the interactions between c-Jun and either the rationally designed A-FosW template or the TBS library-derived HingeW is provided in **Table 4** as follows:

**Table 4: Thermodynamic parameters for the interactions between c-Jun and either the rationally designed A-FosW template or the TBS library derived HingeW. Errors shown as one standard deviation.**

| | **Fraction helicity at 20°C (CD)** | ***Tₘ* (°C) (CD)** | ***ΔTₘ* from compone nt average (CD)** | ***K_{D}* (nM) (ITC)** | ***N* (ITC)** | ***ΔG* (KJ mol⁻¹) (ITC)** | ***ΔH* (KJ mol⁻¹) (ITC)** | ***TΔS* (KJ mol⁻¹) (ITC)** |
|---|---|---|---|---|---|---|---|---|
| **cJun** | 14.0% | 39.8 ± 1.9 | - | - | - | - | - | - |
| **A-FosW** | 40.1% | 45.1 ± 0.9 | - | - | - | - | - | - |
| **A-FosW/ c-Jun** | 38.7% | 69.9 ± 0.7 | 27.5 | 88.3 ± 17.6 | 1.04 ± 0.08 | -40.0± 0.5 | -152.6 ± 4.1 | -112.6 ± 4.1 |
| **HingeW** | 27.2% | - | - | - | - | - | - | - |
| **HingeW /c-Jun** | 34.7% | 71.2 ± 1.4 | ~40 | 14.4 ± 3.7 | 1.05 ± 0.05 | -46.2 ± 0.6 | -85.4 ± 4.5 | -39.2 ± 4.5 |

### 1.10 Conclusion

There are many screening platforms in place to derive high affinity PPIs but none that guarantee target binding will lead to the desired loss-of-function of the target protein. Using cJun/TRE as an exemplar, we have developed a Transcription Block Survival assay that can be used as a generalised approach for the derivation of peptides capable of ablating TF activity. We have engineered a 'molecular dial' into a bacterial system whereby the cJun/TRE DNA interaction is inversely correlated with cell proliferation. By introducing cJun/TRE antagonists into this system, cellular growth becomes a direct readout for the ability of the antagonist to functionally block the numerous cJun/TRE interactions, turning the molecular dial up. The most effective rationally designed acidic antagonist was next utilised as a parental sequence to design a semi-randomised library that was successfully screened in the TBS platform, to produce the in vitro validated assay hit HingeW.

Establishing the TBS system required the production of a mutant DHFR gene (TRE-mDHFR) which retained its enzymatic activity upon introduction of 15 TRE sites into its DNA sequence, leading to 13 amino acid substitutions. This allowed for a cJun-induced transcriptional block when the TF binds to the TRE sites on the TRE-mDHFR plasmid DNA. For loss of TRE-mDHFR activity to take place there is an absolute requirement for both the TF DBD and the TRE sites within the mDHFR gene, confirming specificity in the TBS system. The phenotype of bacterial growth rate is directly linked to the genotype of the antagonist sequence expressed by virtue of the systems containment in a single cell. The phenotype of bacterial growth rate is directly linked to the genotype of the antagonist sequence expressed by virtue of the system's containment within a single cell. Bacterial cells are ideal for this process owing to their fast growth rate, durability, ease of use and low cost. Crucially, they also allow for the direct measurement of cJun interacting with TRE sites in the absence of any related eukaryotic TFs that might interfere with the assay.

TBS facilitates high-throughput genotype to phenotype screening and competition of peptide libraries to isolate those that result in functional loss of cJun DNA binding activity from those that bind but have little or no effect upon target activity (or those that do not bind at all). The distinction is important since it means that an antagonist must not only bind to the target free in solution but must also be capable of meeting the much more demanding task of liberating the TF from DNA, which is known to be more stable (Seldeen et al., 2011). Lastly, all the above is undertaken within the complex environment of the cytoplasm, removing molecules that are toxic, non-specific, insoluble, or protease susceptible from consideration at the initial screening stage, rather than determining this at later hit validation or clinical trial stages. These factors are particularly important for longer peptides, as required to bind to the large and shallow cJun bZIP surface, which tend to lack these important qualities. TBS improves upon the related protein-fragment complementation assay, as well as *in vitro* screening platforms such as phage display or ribosome display, by the complete removal of any requirement for bulky protein fusions or hydrophobic/aromatic tags, which can interfere with the relevant assay interactions and lead to false readouts.

The central advantage of TBS is the requirement for assay hits to prevent TFs from binding to their consensus DNA sequence as exemplified by the combined design of A-FosW, a hybrid containing domains from both A-Fos (Olive et al., 1997) and the FosW PCA hit (Mason et al., 2006). In A-FosW the LZ targets the antagonist to the cJun bZIP with high affinity and selectivity, with the acidic extension added to assist in functionally antagonising the cJun/TRE DNA interaction by blocking the cJun DBD. The LZ domains of bZIP proteins tend to display more sequence diversity than the DBD, which is useful for therapeutic targeting of specific AP-1 family members, which provides better control and potentially fewer side effects (Eferl et al., 2003). Although it is unclear if A-FosW binds cJun by forming a single continuous LZ interaction as designed, increased binding around the hinge region of cJun was anticipated to propagate increased helicity and therefore affinity in either direction. Further, focusing on the hinge region was supported in the original work of Olive et al., where a point mutation in this region of A-Fos (N26L at position a4 of A-FosW) produced a significant increase in cJun binding affinity and subsequent cJun/TRE antagonism (Olive M et al., 1997). Optimisation of the acidic extension through rational design is hampered by the lack of design rules for guidance, as is the case for the LZ domain which has known structure and predicative tools to produce high affinity interactions (Boysen RI et al., 2002; Kaplan JB et al., 2014). Additionally, no library-based approach had previously been used to optimise binding within this region of cJun. Using A-FosW as a design template and including library options in the hinge region was a clear next step which resulted in the TBS selection of HingeW, with 14 nM affinity for the target cJun protein (a 6-fold improvement over A-FosW). HingeW included one more acidic residue than A-FosW, supporting the Olive et al. methodology of including dominant negative charge throughout the N-terminal domain to interact favourably with positive charge within the cJun DBD. However, the precise selection pattern was more nuanced than simply producing a block of negative charged residues. The nature of HingeW suggests another benefit of the TBS library screening approach, in which directed evolution of the antagonist led to an improvement by reducing homodimerisation. TBS has provided considerable utility in the exploration of novel sequence space by producing a protein sequence which could not have been predicted without the use of this library screening approach.

TBS opens a new capability in semi-rational PPI design where both affinity and activity are co-selected for. This offers significant potential to expand the TBS approach to both new libraries and targets where previous work may have produced potential antagonists which were later found to lack functional activity. In principle, the approach can be fully expanded to any DNA-binding protein that recognises a discrete consensus sequence, or even any dimeric system to which a DBD is appended. The method can be assumed to be generalizable, since any DNA consensus sequence can be incorporated into the DHFR DNA sequence and can be transcriptionally blocked by co-expression of the relevant TFs. This will require the DHFR design process to be iterated and subsequent testing and optimization for each system, however, the central principle has been shown here to be valid. It also potentially permits for the screening of exogenous molecules to allow concomitant profiling of both cell penetrant and functionally active inhibitors. Moreover, libraries with different design principles and expanded options harbour considerable additional promise in producing peptide hits across a broad range of targets in which pathogenic TFs are implicated. Library sizes of 10⁶ - 10⁷ are possible using standard techniques and readily available reagents which may allow the exploitation of a broader range of peptide diversity and further optimisation. Further TBS screening of a range of TF targets will produce both non-genetic tools and probes of disease pathways, but there is also considerable potential for a new generation of optimised functional antagonists and clinical leads.

### EXAMPLE 2 - Optimisation of functional c-Jun antagonist

This example shows the optimisation of the peptide library screen-derived hit of example 1, designed to target the full cJun bZIP domain in an attempt to simultaneously block both cJun dimerisation *and* DNA-binding. TBS screening of a 130,000-member peptide library resulted in the HingeW sequence (HW1). HingeW was developed to be capable of binding across the full cJun bZIP domain for more effective functional antagonism of TRE binding, relative to DBD-only or LZ-only cJun inhibitors. The nature of the broad, shallow helical binding surface supports the use of longer peptides such as Hinge. However it was unclear whether the full length of the sequence was required to achieve functional antagonism. HW1 was recombinantly produced and biophysically characterised as a capped (MAS at the N-terminus, GAP at the C-terminus) and C-terminally 6xHis-tagged protein construct of 69 amino acids in length, with significant negative charge throughout. Optimisation of the peptides was carried out with the aim to improve their drug-like characteristics.

### 2.1 Methods

**Peptide synthesis and purification:** All peptides were synthesised using a Liberty Blue microwave peptide synthesiser (CEM) at a 0.1 mmol scale on ChemMatrix Rink amid resin using standard Fmoc solid-phase methodology. Coupling was performed using 5x amino acid, 4.5x PyBOP and 10x diisopropylethylamine in dimethylformamide (DMF, 5 mL). Deprotection was performed using 20% piperidine in DMF. Peptides were capped at the N-terminus by a final reaction with 3x acetic anhydride, 4.5x diisopropylethylamine in DMF for 5 min at 90°C. For lactamised peptides, the relevant K and D positions were orthogonally protected by the use of Lys(Mtt) and Asp(O-2-PhiPr). The sidechains of these residues were selectively deprotected by washing the resin with dichloromethane (DCM) x3, 2% trifluoroacetic acid (TFA) in DCM x10, DCM 3x then DMF 3x. The newly deprotected sidechains were coupled in PyBOP (1 mL), diisopropylethylamine (1 mL) and DMF (3 mL) for 5 hours at 60°C. The resin was dried, and the same reagents added for a second reaction for 16 hours at 60°C. Incubation in a cleavage mixture (95% TFA, 2.5% triisopropylsilane, 2.5% H2O, 10 mL) for 4 h at room temperature cleaves the peptide from the resin and removes side chain protecting groups. The resin was removed by filtration and cleaved peptides were precipitated in diethyl ether at -80°C and centrifuged. This pellet was washed a further four times with diethyl ether before it was dried overnight at room temperature. Peptides were resuspended in 3:1 water:acetonitrile before purification using RP-HPLC with a Jupiter Proteo column (4-µm particle size, 90 Å pore size, 250 × 10 mm; Phenomenex) using a water:acetonitrile gradient (0.1% TFA). Peptide masses and purity (>95%) were verified by electrospray ionisation mass spectrometry.

**Circular Dichroism (CD):** An Applied Photophysics Chirascan was used for CD measurements, with a 200 µL sample in a 1 mm path length CD cell. Protein/DNA samples were suspended in 150 mM potassium phosphate,150 mM potassium fluoride and 5 mM TCEP at pH 7.4 and were equilibrated for 30 minutes before measurement. For full spectra, three scans between 190 and 260 nm (265-320 nm for DNA binding experiments) were collected with a bandwidth of 1 nm and data sampled at a rate of 0.5 s-1. These scans were averaged and converted to molar residue ellipticities (MRE). Thermal denaturation experiments were performed by measuring the ellipticity at 222 nm over a 1 to 90°C gradient at 1°C increments. Post-melt scans at 20°C confirmed the transitions were reversible as they overlaid within 10% of the pre-melt scan. The resulting thermal denaturation curves were converted to MRE and fitted to a two-state model, derived via modification of the Gibbs-Helmholtz equation to determine the melting temperature (Tm) (Mason et al., 2007).

**Serum Stability:** Peptide stocks (600 µM) were prepared in water and 50 µL added to 950 µL human serum (Merck) before incubation at 37°C. 100 µL aliquots were removed at designated timepoints and added to 300 µL 3:1 acetonitrile:water and centrifuged (18000 xg, 15 minutes). The supernatant was analysed by LC-MS and quantified using the sum of the two largest charge state intensities (1: 9+, 10+; 23,24: 3+, 4+).

**Isothermal Titration Calorimetry (ITC):** Peptides were studied by ITC using a PEAQ-ITC (Malvern Instruments) using an ITC buffer consisting of 10 mM potassium phosphate, 150 mM potassium fluoride and 5 mM TCEP at pH 7.4. 2 µL injections of antagonist peptide at 25-200 µM were injected into the cell containing cJun at 2.5-20 µM. Software was used to record and analyse the heat change upon addition. Control experiments involved the injection of the antagonist peptide sample into the cell containing ITC buffer alone to determine the heat of dilution which was subtracted. The resulting binding data were fit to a one site binding model to extract the enthalpy change of binding (ΔH) and the equilibrium binding constant (KD), from which the free energy change of binding (ΔG) and the entropy change of binding (ΔS) was calculated (Wiseman et al., 1989). Thermodynamic parameters are presented as an average of two independent experiments with errors given as one standard deviation.

### 2.2 Acidic/N-terminal truncation

A summary of the various peptides used and described in this section is provided in the Table 5 as follows:

**Table 5: Summary of peptides tested**

| **Peptid e #** | **acidic-ZIP Sequence** |
|---|---|
| | ***defg**abcdefg**abcdefg**abcdefg**abcdefg**abcdefg**abcdefg**abcdefg**abcd*** |
| 1 | MAS***LEQR***AEELARE***NEELEKE***AEELVVE***EDVLEEE***IEQLEER***NYALRKE***IEDLQKQ***LEKL***GAPHHHHHH |
| 2 | Ac-LARE***NEELEKE***AEELVVE***EDVLEEE***IEQLEER***NYALRKE***IEDLQKQ***LEKL***-NH₂ |
| 3 | Ac-***LEKE***AEELVVE***EDVLEEE***IEQLEER***NYALRKE***IEDLQKQ***LEKL***-NH₂ |
| 4 | Ac-***E***AEELVVE***EDVLEEE***IEQLEER***NYALRKE***IEDLQKQ***LEKL***-NH₂ |
| 5 | Ac-LVVE***EDVLEEE***IEQLEER***NYALRKE***IEDLQKQ***LEKL***-NH₂ |
| 6 | ***EDVLEEE***IEQLEER***NYALRKE***IEDLQKQ***LEKL***GAP-NH₂ |
| 7/8 | Ac-LVVE***EDVLEEE***IEQLEEK***NKALKDE***IEDLQKQ***LEKL***Y-NH₂ |
| 9/10 | Ac-LVVE***EDVLEEE***IEQLEER***NYALRKE***IEDLQKQ***LEDL***-NH₂ |
| 11 | Ac-***E***AEELVVE***EDVLEEE***IEQLEER***NYALRKE***IEDLQKQ-NH₂ |
| 12/13 | Ac-***KE***AEDLVVE***EDVLEEE***IEQLEER***NYALRKE***IKDLQDQ-NH₂ |
| 14/15 | Ac-***E***AKELVDE***EDVLEEE***IEQLEER***NYALRKE***IEDLQKQ-NH₂ |
| 16/17 | Ac-***E***AEELVVE***EKVLEDE***IEQLEER***NYALRKE***IEDLQKQ-NH₂ |
| 18/19 | Ac-***E***AEELVVE***EDVLEEE***IKQLEDR***NYALRKE***IEDLQKQ-NH₂ |
| 20/21 | Ac-***E***AEELVVE***EDVLEEE***IEQLEEK***NKALKDE***IEDLQKQY-NH₂ |
| 22/23 | Ac-***E***AEELVVE***EDVLEEE***IEQLEER***NYALRKE***IKDLQDQ-NH₂ |
| 24 | Ac-***E***AEELVVE***EKVLEDE***IEQLEER***NYALRKE***IKDLQDQ-NH₂ |
| 25 | Ac-***E***AEELVVE***EDVLEEE***IEQLEER***NYALRKE***IEDL-NH₂ |
| 26/27 | Ac-***E***AEELVVE***EDVLEEE***IEQLEEK***NKALKDE***IEDLY-NH₂ |
| 28/29 | Ac-***E***AEELVVE***EDVLEEE***IEQLEER***NYALRKE***IEDLQ-NH₂ |

The precise nature of the interaction between the c-Jun DBD and the rationally designed acidic domain of **1** is unknown. Therefore, the acidic domain was the initial focus for optimisation. 1 was iteratively truncated **(2-6)** to investigate the effect on c-Jun binding and c-Jun/TRE DNA antagonism. Peptide helicity was determined by quantifying the CD signal at 222 nm of peptide only samples. Thermal denaturation experiments were then used to determine the *Tₘ* of peptide-c-Jun heterodimers, which was used as an approximate measure of target engagement. CD was also used to investigate the ability of the functional activity of peptides in antagonising the c-Jun/TRE DNA interaction. The TRE-DNA construct used produces a positive CD peak at ~281nm (no c-Jun absorbance at this wavelength allows for direct measurement of DNA upon binding) which decreases in intensity upon c-Jun binding. This provided a clear and direct measurement of the proportion of DNA bound. As increasing concentrations of antagonist peptides were added to the sample, the peak shifts back to overlay with the free TRE-DNA. This allowed for the calculation of an *IC₅₀* value by fitting the titration data to a Hill equation. Due to the higher concentrations required in these experiments (for signal to be detected in the CD) the *IC₅₀* values are corresponding higher, as there is 20 µM cJun in the experimental conditions the lowest the IC50 could drop is 10 µM. Therefore, these values should be taken in context and not compared with values produced by different methodologies but instead used here for comparison amongst antagonists tested. The same 60 amino acid c-Jun bZIP construct was used for all experiments, regardless of antagonist peptide length. Through these experiments, the target binding and subsequent target antagonism were both rapidly characterised *in vitro.*

A summary of the thermal denaturation (*Tₘ*), functional activity (IC₅₀ CD (µM)), helicity (fH (%)) results obtained for each of the peptides tested is provided in **Table 6** as follows:

**Table 6: Summary of thermal denaturation, functional activity, and helicity results**

| **Peptide** # | **Tₘ (°C)** | **IC₅₀ CD (µM)** | **fH (%)** | **Truncation** |
|---|---|---|---|---|
| 1 | 71.2±1.4 | 13.4±0.6 | 27.2 | - |
| 2 | 68.5±1.1 | 16.6±0.5 | 32.8 | NΔ10 CΔ3 |
| 3 | 67.9±0.9 | 33.7±2.8 | 38.3 | NΔ17 CΔ3 |
| 4 | 64.1±1.5 | 45.4±3.6 | 45.5 | NΔ20 CΔ3 |
| 5 | 62.8±1.0 | 78.2±4.1 | 46.7 | NΔ24 CΔ3 |
| 6 | 55.1±1.1 | 129.8±13.0 | 38.0 | NΔ28 |
| 7 | 64.3±1.3 | 83.9±6.2 | 45.6 | NΔ24 CΔ3 |
| 8 | 67.2±0.8 | 73.5±5.2 | 54.2 | NΔ24 CΔ3 |
| 9 | 62.1±1.4 | 94.6±7.4 | 43.0 | NΔ24 CΔ3 |
| 10 | 68.3±0.9 | 69±4.1 | 46.8 | NΔ24 CΔ3 |
| 11 | 57.1±0.9 | 82.1±6.0 | 37.3 | NΔ20 CΔ7 |
| 12 | 56.5±1.6 | 115.0±13.4 | 38.7 | NΔ19 CΔ7 |
| 13 | 58.5±1.0 | 92.5±3.4 | 40.1 | NΔ19 CΔ7 |
| 14 | 57.5±1.1 | 100.4±3.9 | 36.9 | NΔ20 CΔ7 |
| 15 | 59.4±1.0 | 81.1±5.7 | 39.2 | NΔ20 CΔ7 |
| 16 | 54.9±1.2 | 113.6±10.8 | 37.2 | NΔ20 CΔ7 |
| 17 | 64.0±0.8 | 74.9±4.1 | 42.6 | NΔ20 CΔ7 |
| 18 | 55.5±1.7 | 98.9±5.1 | 37.0 | NΔ20 CΔ7 |
| 19 | 60.9±3.6 | 94.4±4.5 | 38.7 | NΔ20 CΔ7 |
| 20 | 56.2±1.3 | 120.8±17.3 | 36.0 | NΔ20 CΔ7 |
| 21 | 58.9±1.0 | 94.1±6.0 | 38.5 | NΔ20 CΔ7 |
| 22 | 55.6±1.2 | 100.8±3.7 | 35.0 | NΔ20 CΔ7 |
| 23 | 65.0±1.4 | 52.7±2.0 | 43.4 | NΔ20 CΔ7 |
| 24 | 63.2±1.3 | 44.9±2.1 | 47.1 | NΔ20 CΔ7 |
| 25 | 44.8±1.2 | 1212.9±118.4 | 32.8 | NΔ20 CΔ10 |
| 26 | N.D. | 1559.5±143.9 | 30.1 | NΔ20 CΔ10 |
| 27 | 52.5±1.4 | 118.5±10.9 | 35.0 | NΔ20 CΔ10 |
| 28 | 53.8±5.3 | 725±62.6 | 31.2 | NΔ20 CΔ9 |
| 29 | 57.2±0.4 | 140±14.3 | 36.4 | NΔ20 CΔ9 |

All N-terminal truncations in the series **(2-6)** reduced peptide/target binding and antagonism efficacy, indicating that the full length of the acidic extension contributes to antagonism of the c-Jun/TRE interaction **(****Figure 18****).** However, each truncation in the series resulted in an increasingly large effect on the antagonism per residue removed. Inspection of the truncations from **1** to **2,** from **2** to **3** and from **3** to **5,** representing the three full heptad N-terminal deletions, reveals antagonism decreases of 1.2-fold, 2-fold and 2.3-fold respectively. As expected, the percentage lost, as each subsequent heptad is deleted, increases and therefore becomes more important to antagonism. Further, the final N-terminal truncation investigated, from **5** to **6,** reduces the antagonism 1.7-fold despite only removing four residues. Peptide **6** (NΔ28) represents the LZ-only portion of the HingeW molecule. To test for the possibility that the acidic-domain alone can antagonise c-Jun, this portion alone was also tested and shown not to bind (Data not shown). This indicates that the acidic extension and LZ domains of HingeW operate synergistically to bind and antagonise c-Jun.

Truncating the N-terminus also leads to sequential increases in fraction helicity (fH), rising from ~27% for **1** to ~47% for **5,** indicating that deleted regions are of lower helicity relative to the LZ region. The LZ domain of **1** was mostly unchanged from its parent sequence, FosW, which is known to homodimerize (Mason et al., 2006). The negative charge of the acidic extension produces electrostatic repulsion and therefore decreases the propensity to homodimerse, with their removal increasing homodimerisation-induced helicity. However, further truncation from **5** to **6** reverses this trend, reducing fH to ~38.0% while removing one acidic and three hydrophobic residues, to leave the LZ -only domain. There is little further electrostatic repulsion to be reduced but this region likely contributes to homodimerisation through direct binding or helix induction.

Overall, removal of the acidic extension domain **(1** to **6)** reduced cJun/TRE DNA antagonism 9.7-fold. Previously, we observed a highly significant reduction in peptide activity during TBS screening for peptides with IC₅₀ values similar to that observed for peptide **6** (130 uM), meaning that **6** may be unable to fully outcompete cJun/TRE DNA binding. This further supported the design rationale of the acidic extension and suggested that **5** (IC50 = 78uM, 5.8x lower antagonism than **1,** and 1.7x better than **6)** may be considered the maximum viable truncation from the N-terminus to be taken forward.

### 2.3 Lactamisation of the N-terminal truncation

Peptide **5** was next optimised by incorporating *i→i+4* (K-to-D) lactam bridges. Lactam bridges can be incorporated through the use of orthogonal-protecting groups (Lys(Mtt) and Asp(O-2-PhiPr)), which can be selectively deprotected (2% trifluoroacetic acid in DCM) and reacted using typical solid phase chemistry while the peptide is still attached to the resin. The success of the reaction can be confirmed using mass spectrometry (MS) to observe the decreased mass from the loss of a water molecule, compared with the linear unreacted peptide. In this example lactams were only introduced at solvent exposed **b-to-f** or **f**-to-c heptad positions to prevent disruption of the binding surface of the helix. Point mutations to the sequence were required to incorporate the bridging K and D residues, with both linear **(7,9)** and cyclised **(8,10)** versions of each sequence produced by split batch synthesis.

Cyclised peptides **8** and **10** increase antagonism relative to the linear counterpart **5,** 1.8-fold and 1.9-fold respectively. The side chain lactamisation reactions lead to increased helicity which translate to higher affinity binding. It is interesting to note in these peptides that the binding indicated by *Tₘ* values of ~67 and ~68°C do not produce correspondingly low IC₅₀ values. *Tₘ* can be seen to inversely correlate with *IC₅₀* for each peptide as predicted, however there is a level of variability in the trend **(****Figure 19****).** Peptides **2** and **10,** for example, have similar *Tₘ* values but **2** antagonises the cJun/TRE interaction 4x more effectively. **10** has been truncated by a further 14 N-terminal residues than **2.** This supports the importance of the rational design principle used for HingeW whereby inhibition of both domains of the cJun bZIP produces the most effective antagonism as the majority of the acidic extension has been removed in peptides **5** and **7-10** and all of it has been removed in peptide **6.** This results in peptides which can be optimised to bind tightly to the cJun LZ but are limited in their ability to functionally antagonise cJun by also blocking the DBD.

Truncation from **4** to **5** removed a block of negative charge (EAEE) and resulted in a 1.7x reduction in antagonism. This suggests the regions importance for interacting with the positively charged cJun DBD surface, as well as inducing helicity and potentially stabilising the dipole of the molecule (Pace et al., 1998; Sali et al., 1988). This correlates with work on the interaction of the cJun DBD with TRE DNA that has shown the particular residues which interact directly with the DNA. The central two residues of this added block (EAEE - moving from **10** to **11)** occur at positions corresponding to interaction with DNA on cJun i.e. these assist in form a direct block between cJun and DNA. The NΔ20 truncation may therefore be considered as an optimal balance between downsizing and retaining functional activity.

### 2.4 Truncation of the C-terminus

Peptide **11** was therefore the next step in optimisation which utilises the NΔ20 truncation whilst also truncating at the C-terminus. The removal of the four C-terminal residues from **4** to **11** reduced antagonism 1.8x but further truncation at the C-terminus to produce **25** vastly reduced antagonism 14.8x compared to **11,** and 26.7x compared to **4.** Attempts to optimise **25** by lactamisation to produce **27** and **29** were effective in that they significantly improved antagonism however they still produce 8.8x and 10.4x reductions in antagonism relative to **1.** Although these lactamisations produced a much larger impact on the peptides, they were still considered to be too ineffective for further study.

### 2.5 Optimisation of 11

Peptide **11** was considered as a scaffold for further optimisation which has almost half the number of residues compared with **1** whilst retaining a high level of functional activity. K-to-D lactam bridges at *i* to *i+4* positions were systematically incorporated at different sites to investigate which regions were most amenable to the helix constraint, and which produced improvements in affinity and inhibition. Again, due to point mutations to accommodate the bridging K and D residues, both linear and cyclised peptides were produced. The heterodimer *ΔTₘ* from lactamisation ranges from ~2°C for **14/15** to ~9°C for **22/23.** It is also useful to compare to the original parent sequence where the heterodimer *ΔTₘ* ranges from ~1°C for **11/13** to ~8°C for **11/23.** C-Jun/TRE antagonism is the most important measure in this work however and only **23** produced a significant improvement in *IC₅₀*, shown to be 1.6x lower than for **11** (P=0.003). By the use of a lactam, **23** restores the reduction in antagonism caused by truncating the four C-terminal residues of **4.**

Information regarding the suitability of each site for lactamisation can be gathered from this data. As the change in fraction helicity of the peptides ranges from ~1% for **12/13** to ~8% for **22/23.** There is a correlation between the increase in peptide helicity induced by lactamisation at a particular site and the increase in cJun/TRE antagonism observed.

Although the lactamisation within **17** did not lead to a significant increase in antagonism (P=0.24), it did produce ~5% increase in peptide helicity and ~9°C increase in cJun heterodimer *Tₘ* from its linear form **16.** Due to the distance between this site and the lactamisation site of **23,** a double lactamised peptide was produced, **24.** The double lactamisation produces a peptide that is ~4% more helical, no significant change in heterodimer *Tₘ* but a significant decrease in *IC₅₀* value (P=0.02), compared to the best single lactamised peptide.

A selection of the most effective peptides from this work were investigated by ITC to quantify the thermodynamic parameters of their interaction with target cJun **(****Figure 20****, Table 7).** The two single lactam peptides which were most effective in CD experiments and the double lactam which utilised both of these were tested and compared to the parent sequence. 1/cJun has been investigated via ITC previously but at a higher temperature resulting in the lower *K_{D}* reported here. All interactions investigated were dominated by the enthalpic component, with a smaller unfavourable entropic contribution. This unfavourable entropic component was largest for the **24**/cJun interaction and smallest for **17**/cJun.

**Table 7: ITC derived thermodynamic parameters c-Jun-peptide interactions. Thermodymanic parameters are presented as an average of two independent experiments with errors given as one one SD.**

| | **Stoichiometry, n** | ***K_{D}* (nM)** | ***ΔH* (kJ mol⁻¹)** | ***ΔG* (kJ mol⁻¹)** | ***-TΔS* (kJ mol⁻¹)** |
|---|---|---|---|---|---|
| **1** | 0.93±0.01 | 0.21±0.03 | -129.70±3.65 | -56.27±0.75 | 73.43±3.70 |
| **4** | 0.91±0.04 | 91.1±24.80 | -189.95±8.09 | -40.50±0.48 | 149.5±8.10 |
| **11** | 0.95±0.09 | 1099.50±181 .5 | -25.06±3.40 | -34.10±0.41 | -8.95±3.42 |
| **17** | 1.20±0.02 | 69.55±7.83 | -59.00±1.68 | -40.86±0.28 | 18.14±1.70 |
| **23** | 0.97±0.02 | 86.55±16.97 | -98.74±2.54 | -40.42±0.49 | 58.37±2.59 |
| **24** | 1.05±0.01 | 9.69±3.95 | -127.82±2.61 | -45.81±1.01 | 82.01±2.80 |

**1, 23** and **24** were also tested for their serum stability to illustrate the effect of truncation and lactamisation **(****Figure 21****).** At 24 hours, **1** is 67% degraded, **23** is 21% degraded and **24** is 7% degraded. Only **1** shows the exponential decay required to fit to a model of exponential decay, which indicates a half-life of 18.9 hours. After 96 hours, no peaks corresponding to **1** are observed in the MS. At this end point, the peak intensities of **23** are still 17% of their starting values and **24** is at 35%. This clearly illustrates the increase in stability provided by the non-natural lactam bridges, and that the effect is cumulative as **24** is more stable than **23** (P=0.038).

### 2.5 Conclusion

A rationally designed series of peptides was synthesised to investigate and optimise HingeW **(1),** through the systematic study of truncation and helix-inducing lactamisation. **1** proved to be inaccessible via SPPS using standard techniques (i.e. without native chemical ligation), with **2** and **3** only produced at strikingly low yields. The remaining smaller peptides were all produced to a similar and high yield via SPPS. The increased production efficiency derived through moving from recombinant expression to SPPS should not be understated. The process of iterative truncation and characterisation elucidates the efficacy of different portions of the parent sequence and allowed careful consideration of how much peptide to truncate to derive a minimal effective sequence. In this example, the important consideration is whether a peptide binding to the target c-Jun will be able to outcompete dimeric c-Jun bound to TRE. This interaction has been variously established as having a *K_{D}* ~100-200 nM so maintaining a stronger interaction than this was considered an important benchmark (Seldeen et al., 2011). Comparing the CD antagonism data **of 1** and **6 (****Figure 18B****)** illustrates this important consideration as 1 is able to restore the TRE DNA peak to its unbound intensity, indicating that no DNA is bound to cJun at high peptide concentration. However, whilst **6** is inhibiting the interaction, it may be that the line does not tend towards 1 and that regardless of the amount of **6** present in the sample some c-Jun/TRE DNA complex will remain. The balance between this loss of efficacy and the gains in drug-like characteristics and synthesis efficiency are difficult to quantify but the data suggested the truncation from **1** to **4,** which removed 20 residues from the N-terminus. Truncation at the C-terminus from 4 to **11** was also supported as in more truncation weakened the peptides efficacy to a degree whereby even lactamisation could not restore binding to the point where the peptide can outcompete the c-Jun/TRE DNA interaction.

The acceptable degree of truncation at the N-terminus appears to go beyond binding affinity considerations as this acidic domain is crucial for blocking the c-Jun DBD. Peptides **5-10** are no longer able to effectively prevent DNA binding to the c-Jun DBD, i.e. cJun may still bind to TRE sites as a monomer whilst these antagonist peptides are bound to its LZ domain. However, effective antagonism might not require binding to the full length of the c-Jun DBD. Specific contacts between particular c-Jun DBD amino acid side chains and TRE DNA bases are known so it can be assumed that an antagonist that binds any of these c-Jun residues will significantly reduce c-Jun/TRE binding, so binding to the full DBD may not be required to maintain high levels of inhibition (Glover et al., 1995; Seldeen et al., 2011). So peptide **4** appears to be the maximum acceptable truncation as it extends N-terminally to a point where it can directly block the c-Jun DBD from interacting with DNA, and crucially presents a block of negatively charged residues in this region to attract the DBD and repel DNA. **4,** by virtue of the addition of EAEE residues, has slightly reduced homodimerisation compared to **5** and better antagonism.

Almost half of the length of 1 has been removed to produce **24,** with a high level of efficacy retained. Due to the nature of the α-helical coiled coil structure and hydrogen bond networks, residues which are not involved in binding to the peptide's dimer partner may still affect binding by the induction of helicity. Further, the helicity of peptides studied here must be considered in their various dimerisation equilibria e.g. between homodimer, monomer and heterodimer, with increases in either dimerisation event tending to lead to increases in helicity. Reducing the length of an α-helix will tend to reduce its helicity unless the removed residues disrupt the structure (e.g. if it contains proline or glycine) or repel either dimer binding partner (e.g. electrostatic repulsion).

The 20% increase in peptide helicity from **1** to **24** resulted in increased peptide serum stability. The N-terminal regions of the full length **1** in particular appear to be non-helical and don't improve target antagonism so their removal increases stability without a significant impact on peptide efficacy. Cyclisation increases peptide helicity, which reduces protease recognition and increases peptide serum stability. Adding the second lactam in **24** does increase this stability further though the increase in stability is smaller than for the addition of the first lactam. Peptide degradation for the lactamised peptides cannot be fit to an exponential decay function as it occurs too slowly.

Particular lactam flanking residues, at any given position, may be better suited to accommodating the lactam and adopting a helix structure than others. How the peptide folds to adopt the helical structure may also influence the effect of a lactam, as for example if the helix fold is propagated from one end of the peptide to the other then the effect of a lactam will likely vary depending on how close to this locus of folding they are located.

The thermodynamic parameters of binding observed by ITC show a slightly unexpected result in terms of the entropic component. It is usually asserted that introduction of lactam bridges increases binding affinity by preorganising the peptide molecule into its helical structure which can bind to the target with an entropic penalty. All of the peptides investigated by ITC have an unfavourable entropic component however **17** has a significantly lower contribution from this component. In this case it does appear that the entropic penalty of binding is being reduced by preorganising the peptide into a helical fold that is complimentary to the cJun binding surface. However, for **23** and particularly **24** there is large unfavourable contribution from the entropic component. This illustrates that increased target binding affinity from sidechain cyclisation can also occur due to improved enthalpic interactions.

### EXAMPLE 3 - HingeW bisalkylation

To identify further peptide HingeW variants that can be cyclised using bisalkylation, as opposed to lactamisation, variants of the c-Jun antagonist sequence EAEELVVEEDVLEEEIEQLEERNYALRKEICDLQCQ (SEQ ID NO: 28) were screened using the TBS library screen described in Example 1. The screen identified the sequences set out in Table 8 below.

**Table 8 - HingeW variants suitable for bisalkylation with metaDBM B.**

| **Peptide variant** | **Sequence** |
|---|---|
| 0W | EAEELVVEEDVLEEEIEQLEERNYALRSEICSLQCQ (SEQ ID NO: 37) |
| orthoDBMBW | EAEELVVEEDVLEEEIEQLEERNYALRKEICELSCQ (SEQ ID NO: 37) |
| metaDBMBW | EAEELVVEEDVLEEEIEQLEERNYALRAEICNLSCQ (SEQ ID NO: 39) |
| paraDBMBW | EAEELVVEEDVLEEEIEQLEERNYALRTEICSLMCK (SEQ ID NO: 40) |
| tdbbDBMBW | EAEELVVEEDVLEEEIEQLEERNYALRAEICSLQCQ (SEQ ID NO: 41) |

Out of the five sequences listed in Table 8, 0W and metaDBMBW were cyclised using bisalkylation with mDBMB and the functional activity of the linear and cyclised forms were tested in a CD assay and results and the CD curves are shown in **Figure 23. Figure 23** shows that cyclised metaDBMBW and 0W improves c-Jun target binding compared to their linear counterparts and this is reflected in lower IC50 for the cyclised variants as shown in **Table 9.**

**Table 9: Functional activity (IC₅₀ CD) of listed peptide variants. Errors are standard deviation (SD).**

| **Peptide variant** | **IC₅₀ (µM)** |
|---|---|
| 0W linear | 86.7±4.4 |
| 0W cyclised | 77.6±6.5 |
| metaDBMBW Linear | 91.1±8.6 |
| metaDBMBW Cyclised | 61.5±1.9 |

### References

A number of publications are cited above in order to more fully describe and disclose the invention and the state of the art to which the invention pertains. Full citations for these references are provided below.
Ahn et al., A dominant-negative inhibitor of CREB reveals that it is a general mediator of stimulus-dependent transcription of c-fos. Mol Cell Biol 18, 967-977 (1998).
Alani et al., The transactivating domain of the c-Jun proto-oncoprotein is required for cotransformation of rat embryo cells. Mol Cell Biol 11, 6286-6295 (1991).
de Araujo, et al., Comparative α-helicity of cyclic pentapeptides in water. Angew Chem Int Ed Engl,. 53(27): p. 6965-9. (2014)
Azzarito, V. et al., Inhibition of α-helix-mediated protein-protein interactions using designed molecules. Nature chemistry, 5(3), 161-173. (2013)
Baxter et al., Library construction, selection and modification strategies to generate therapeutic peptide-based modulators of protein-protein interactions. Future Med Chem 6, 2073-2092 (2014).
Baxter et al., Exploiting Overlapping Advantages of In Vitro and In Cellulo Selection Systems to Isolate a Novel High-Affinity cJun Antagonist. ACS Chem Biol 12, 2579-2588 (2017).
Boysen et al., Role of interfacial hydrophobic residues in the stabilization of the leucine zipper structures of the transcription factors c-Fos and c-Jun. J Biol Chem 277, 23-31 (2002).
Brennan, et al., Selective antagonism of cJun for cancer therapy. J Exp Clin Cancer Res 39, 184 (2020).
Cabezas, E.; Satterthwait, A. C. J. Am. Chem. Soc., 121 , 3862. (1999)
Chen et al., Design of peptide inhibitors that bind the bZIP domain of Epstein-Barr virus protein BZLF1. J Mol Biol 408, 304-320 (2011).
Cody et al., Understanding the role of Leu22 variants in methotrexate resistance: comparison of wild-type and Leu22Arg variant mouse and human dihydrofolate reductase ternary crystal complexes with methotrexate and NADPH. Acta Crystallogr D Biol Crystallogr 61, 147-155 (2005).R. L.
Dai et al., Novel DNA bis-intercalation by MLN944, a potent clinical bisphenazine anticancer drug. J Biol Chem 279, 46096-46103 (2004).
Eckert et al., AP1 transcription factors in epidermal differentiation and skin cancer. J Skin Cancer 2013, 537028 (2013).
Eferl et al., AP-1: a double-edged sword in tumorigenesis. Nat Rev Cancer 3, 859-868 (2003).
Fanjul et al., A new class of retinoids with selective inhibition of AP-1 inhibits proliferation. Nature 372, 107-111 (1994).
Fairlie, D. P., & Dantas de Araujo, A.. Review stapling peptides using cysteine crosslinking. Biopolymers, 106(6), 843-852. (2016)
Fujimoto, K. et al. (2008) Development of a series of cross-linking agents that effectively stabilize alpha-helical structures in various short peptides. Chemistry 14(3):857-63.
Glover, J.N. and S.C. Harrison, Crystal structure of the heterodimeric bZIP transcription factor c-Fos-c-Jun bound to DNA. Nature, 1995. 373(6511): p. 257-61.
Haney, C.M. et al. Promoting peptide α-helix formation with dynamic covalent oxime side-chain crosslinks. Chem Commun Camb).47(39):10915-7. (2011)
Heitz et al. Twenty years of cell-penetrating peptides: from molecular mechanisms to therapeutics. Br J Pharmacol.157(2):195-206. (2009)
Holland-Nell, K.; Meldal, M. Maintaining biological activity by using triazoles as disulfide bond mimetics. Angew Chem Int Ed Engl. 50(22):5204-6. (2011)
Jain et al., A-ZIP53, a dominant negative reveals the molecular mechanism of heterodimerization between bZIP53, bZIP10 and bZIP25 involved in Arabidopsis seed maturation. Sci Rep 7, 14343 (2017).
Jo, H et al., Development of α-helical calpain probes by mimicking a natural protein-protein interaction. Journal of the American Chemical Society, 134(42), 17704-17713 (2012)
Jochim, A. L., & Arora, P. S.. Systematic analysis of helical protein interfaces reveals targets for synthetic inhibitors. ACS chemical biology, 5(10), 919-923. (2010)
John, R. et al., DNA binding of Jun and Fos bZip domains: homodimers and heterodimers induce a DNA conformational change in solution. Nucleic Acids Res 24, 4487-4494 (1996).
Kaplan et al., Increasing the affinity of selective bZIP-binding peptides through surface residue redesign. Protein Sci 23, 940-953 (2014).
Kowalczyk et al., Peptide Lipidation - A Synthetic Strategy to Afford Peptide Based Therapeutics. Peptides and Peptide-based Biomaterials and their Biomedical Applications. 1030: 185-227.(2017)
Lambert S. A. et al., The Human Transcription Factors. Cell 175, 598-599 (2018).
Lathbridge et al., Computational Competitive and Negative Design To Derive a Specific cJun Antagonist. Biochemistry 57, 6108-6118 (2018).
Leduc, A.M. et al. Helix-stabilized cyclic peptides as selective inhibitors of steroid receptor- coactivator interactions. Proc Natl Acad Sci U S A. 100(20):11273-8 (2003)
Lee T. I., Young R. A., Transcriptional regulation and its misregulation in disease. Cell 152, 1237-1251 (2013)
Mason et al., Semirational design of Jun-Fos coiled coils with increased affinity: Universal implications for leucine zipper prediction and design. Proc Natl Acad Sci U S A 103, 8989-8994 (2006).
Mason et al., Improved stability of the Jun-Fos Activator Protein-1 coiled coil motif: A stopped-flow circular dichroism kinetic analysis. J Biol Chem,. 282(32): p. 23015-24. (2007)
Matthews et al., Dihydrofolate reductase. The stereochemistry of inhibitor selectivity. J Biol Chem 260, 392-399 (1985).
Muppidi, A. et al. Achieving cell penetration with distance-matching cysteine cross-linkers: a facile route to cell-permeable peptide dual inhibitors of Mdm2/Mdmx. Chem Commun (Camb). 47(33):9396-8. (2011)
Muñoz et al., Elucidating the folding problem of helical peptides using empirical parameters. Nat Struct Biol 1, 399-409 (1994).
Olive et al., A dominant negative to activation protein-1 (AP1) that abolishes DNA binding and inhibits oncogenesis. J Biol Chem 272, 18586-18594 (1997).
Pace et al., A helix propensity scale based on experimental studies of peptides and proteins. Biophys J,. 75(1): p. 422-7. (1998)
Perry, Samuel R et al. "Contiguous hydrophobic and charged surface patches in short helix-constrained peptides drive cell permeability." Organic & biomolecular chemistry vol. 16,3 (2018): 367-371.
Raj, M., Bullock, B. N., & Arora, P. S.. Plucking the high hanging fruit: a systematic approach for targeting protein-protein interactions. Bioorganic & medicinal chemistry, 21(14), 4051-4057 (2013)
Risse et al., Asymmetrical recognition of the palindromic AP1 binding site (TRE) by Fos protein complexes. EMBO J 8, 3825-3832 (1989).
Robertson, N. S., & Spring, D. R., Using Peptidomimetics and Constrained Peptides as Valuable Tools for Inhibiting Protein-Protein Interactions. Molecules (Basel, Switzerland), 23(4), 959. (2018)
Rodriguez-Martinez et al., Combinatorial bZIP dimers display complex DNA-binding specificity landscapes. Elife 6, e19272 (2017).
Ruan, F. et al. Metal ion-enhanced helicity in synthetic peptides containing unnatural, metal- ligating residues J. Am. Chem. Soc., 112 (25): 9403-9404 (1990)
Sali, D., M. Bycroft, and A.R. Fersht, Stabilization of protein structure by interaction of alpha-helix dipole with a charged side chain. Nature,. 335(6192): p. 740-3. (1988)
Seldeen et al, Evidence that the bZIP domains of the Jun transcription factor bind to DNA as monomers prior to folding and homodimerization. Arch Biochem Biophys 480, 75-84 (2008).
Seldeen et al., Energetic coupling along an allosteric communication channel drives the binding of Jun-Fos heterodimeric transcription factor to DNA. FEBS J 278, 2090-2104 (2011).
Szalóki et al., Evidence for Homodimerization of the c-Fos Transcription Factor in Live Cells Revealed by Fluorescence Microscopy and Computer Modeling. Mol Cell Biol 35, 3785-3798 (2015).
Shaulian et al., AP-1 in cell proliferation and survival. Oncogene 20, 2390-2400 (2001).
Shiozawa, S. and K. Tsumiyama, Pathogenesis of rheumatoid arthritis and c-Fos/AP-1. Cell Cycle, 2009. 8(10): p. 1539-43.
Smith, Betsy A et al. "Minimally cationic cell-permeable miniature proteins via alpha-helical arginine display." Journal of the American Chemical Society vol. 130,10 (2008): 2948-9.
Thillet et al., Site-directed mutagenesis of mouse dihydrofolate reductase. Mutants with increased resistance to methotrexate and trimethoprim. J Biol Chem 263, 12500-12508 (1988).
Timmerman et al., Rapid and quantitative cyclization of multiple peptide loops onto synthetic scaffolds for structural mimicry of protein surfaces. Chembiochem : a European journal of chemical biology, 6(5), 821-824. (2005)
Tsuchida et al., Design, synthesis, and biological evaluation of new cyclic disulfide decapeptides that inhibit the binding of AP-1 to DNA. J Med Chem 47, 4239-4246 (2004).
Tyndall, et al., Proteases universally recognize beta strands in their active sites. Chem Rev,. 105(3): p. 973-99 (2005)
Walensky, L.D. et al. Activation of apoptosis in vivo by a hydrocarbon-stapled BH3 helix. Science. 305(5689):1466-70. (2004)
Wang, D. et al. Enhanced metabolic stability and protein-binding properties of artificial alpha helices derived from a hydrogen-bond surrogate: application to Bcl-xL. Angew Chem Int Ed Engl. 44(40):6525-9. (2005)
Wiseman, T., et al., Rapid measurement of binding constants and heats of binding using a new titration calorimeter. Anal Biochem,. 179(1): p. 131-7. (1989)
Worrall, J. M. Mason, Thermodynamic analysis of Jun-Fos coiled coil peptide antagonists. FEBS J 278, 663-672 (2011).
Yung et al., Role of c-Jun N-terminal Kinase (JNK) in Obesity and Type 2 Diabetes. Cells, 2020. 9(3).

## Claims

1. A c-Jun antagonist, comprising:
an extended hinge region having an amino acid sequence of LV[X₁]EE[X₂][X₃]LE[X₄]E (SEQ ID NO: 1); and
a leucine zipper (LZ) region C-terminal to the extended hinge region,
wherein
X₁ is selected from V, D, K, C and R,
X₂ is selected from D, K, C and R,
X₃ is selected from V, D, C, K and R, and
X₄ is selected from E, D, C, K and R.

2. The c-Jun antagonist of claim 1, wherein:
(a) the extended hinge region further comprises an N-terminal acidic extension having an amino acid sequence of EA[X₅][X₆] (SEQ ID NO: 2),
wherein
X₅ is selected from E, K and C, and
X₆ is selected from E and D;
optionally wherein the acidic extension has an amino acid sequence of EAEE (SEQ ID NO: 3); and/or
(b)
(i) X₁ is V, and/or wherein X₃ is V;
(ii) X₁ is V, X₂ is D, X₃ is V and X₄ is E;
(iii) X₁ is V, X₂ is K, X₃ is V and X₄ is D; or
(iv) X₁ is V, X₂ is C, X₃ is V and X₄ is C.

3. The c-Jun antagonist of claim 1 or claim 2, wherein the LZ region comprises an amino acid sequence of IEQLEERNYALRKEIKDLQDQ (SEQ ID NO: 7), or a variant thereof comprising 1, 2, 3, 4 or 5 amino acid modifications;
optionally wherein:
(i) the LZ region comprises an amino acid sequence of IEQLEERNYALRKEIKDLQDQ (SEQ ID NO: 7), or a variant thereof comprising 1, 2 or 3 amino acid modifications,
optionally wherein amino acid residues at positions corresponding to position 16 and position 20 of SEQ ID NO: 7 in the variant are K and D amino acid residues, respectively; or
(ii) the LZ region comprises an amino acid sequence of IEQLEERNYALRKEICDLQCQ (SEQ ID NO: 27), or a variant thereof comprising 1, 2 or 3 amino acid modifications,
optionally wherein amino acid residues at positions corresponding to position 16 (position b in the heptad) and position 20 (position *f* in the heptad) of SEQ ID NO: 27 in the variant are both C amino acid residues.

4. The c-Jun antagonist according to any one of claims 1-3, wherein the LZ region comprises an amino acid sequence of IEQLEERNYALR[X₇]E[X₈]K[X₉]L[X₁₀]D[X₁₁] (SEQ ID NO: 29) or IEQLEERNYALR[X₇]E[X₈]C[X₉]L[X₁₀]C[X₁₁] (SEQ ID NO: 30) wherein
[X₇] is K, L, S, W, P, Q, R, M, T, V, A, E, or G;
[X₈] is I, V, or L;
[X₉] and [X₁₀] are any amino acid residue; and
[X₁₁] is Q, E, or K;
optionally wherein the LZ region comprises an amino acid sequence selected from the group consisting of:
IEQLEERNYALRSEICSLQCQ (SEQ ID NO: 66); or
IEQLEERNYALRKEICELSCQ (SEQ ID NO: 67); or
IEQLEERNYALRAEICNLSCQ (SEQ ID NO: 68); or
IEQLEERNYALRTEICSLMCK (SEQ ID NO: 69); or
IEQLEERNYALRAEICSLQCQ (SEQ ID NO: 70),
or a variant thereof comprising 1, 2, or 3 amino acid modifications.

5. The c-Jun antagonist of any one of the preceding claims, wherein the antagonist has the amino acid sequence of:
(i) EAEELVVEEDVLEEEIEQLEERNYALRKEIKDLQDQ (SEQ ID NO:10), or a variant thereof comprising 1, 2 or 3 amino acid modifications, optionally wherein amino acid residues at positions corresponding to position 31 and position 35 of SEQ ID NO: 10 in the variant are K and D amino acid residues, respectively;
(ii) EAEELVVEEKVLEDEIEQLEERNYALRKEIKDLQDQ (SEQ ID NO: 11), or a variant thereof comprising 1, 2 or 3 amino acid modifications, optionally wherein amino acid residues at positions corresponding to position 10 and position 14 of SEQ ID NO: 11 in the variant are K and D amino acid residues, respectively, and wherein amino acid residues at positions corresponding to position 31 and position 35 of SEQ ID NO: 11 in the variant are K and D amino acid residues, respectively;
(iii) EAEELVVEEDVLEEEIEQLEERNYALRKEICDLQCQ (SEQ ID NO: 28), or a variant thereof comprising 1, 2 or 3 amino acid modifications, optionally wherein amino acid residues at positions corresponding to position 31 and position 35 of SEQ ID NO: 28 in the variant are both C amino acid residues;
(iv) EAEELVVEEDVLEEEIEQLEERNYALRSEICSLQCQ (SEQ ID NO: 37), or a variant thereof comprising 1, 2 or 3 amino acid modifications, optionally wherein amino acid residues at positions corresponding to position 31 and position 35 of SEQ ID NO: 37 in the variant are both C amino acid residues; or
(v) EAEELVVEEDVLEEEIEQLEERNYALRAEICNLSCQ (SEQ ID NO: 39), or a variant thereof comprising 1, 2 or 3 amino acid modifications, optionally wherein amino acid residues at positions corresponding to position 31 and position 35 of SEQ ID NO: 39 in the variant are both C amino acid residues.

6. The c-Jun antagonist of any one of the preceding claims, wherein the antagonist:
(a) has between 30 and 70 amino acids, between 30 and 60 amino acids, between 30 and 50 amino acids, or between 30 and 40 amino acids; and/or
(b) is able to inhibit the DNA-binding activity of c-Jun within 10-fold of the ability of HingeW (SEQ ID NO: 48) to inhibit the DNA-binding activity of c-Jun.

7. The c-Jun antagonist of any one of claims 1 to 3 and 6, wherein the antagonist comprises or consists of the amino acid sequence of LARENEELEKEAEELVVEEDVLEEEIEQLEERNYALRKEIEDLQKQLEKL (SEQ ID NO: 13), or a variant thereof comprising 1, 2 or 3 amino acid modifications,
optionally wherein the antagonist comprises the amino acid sequence of LARENEELEKEAEELVVEEDVLEEEIEQLEERNYALRKEIEDLQKQLEKL (SEQ ID NO: 13).

8. The c-Jun antagonist of any one of the preceding claims, wherein the antagonist is in the D- or L-form.

9. The c-Jun antagonist of any one of the preceding claims, comprising:
(i) at least one amino acid residue cross-linker; and/or
(ii) at least one covalent *i to i+4* or *i to i+7* amino acid residue cross-linker,
optionally wherein:
(a) the antagonist comprises at least one covalent *i to i+4* amino acid residue cross-linker, optionally wherein the peptide comprises two covalent *i to i+4* amino acid residue cross-linkers; and/or
(b) the covalent *i to i+4* amino acid cross-linker(s) are present at heptad locations b-to-f or f-to-c; and/or
(c) the covalent *i to i+4* amino acid residue cross-linker(s) are K to D lactam bridge(s), or alkyl cross-link(s) formed between pair(s) of C residues;
and optionally wherein the alkyl cross-link formed between two C residues is formed by 1,3 dibromomethylbenzene (DBMB).

10. A nucleic acid encoding the amino acid sequence of the c-Jun antagonist of any one of the preceding claims.

11. A conjugate comprising the c-Jun antagonist of any one of claims 1 to 9 conjugated to a lipid, a polymer, or a second peptide,
optionally wherein the second peptide is a cell penetrating peptide.

12. A pharmaceutical composition comprising the c-Jun antagonist of any one of claims 1 to 9, or the nucleic acid according to claim 10, or the conjugate according to claim 11, in combination with a pharmaceutically acceptable excipient or carrier.

13. The c-Jun antagonist of any one of claims 1 to 9, nucleic acid of claim 10, conjugate of claim 11, or pharmaceutical composition of claim 12, for use as a medicament.

14. The c-Jun antagonist of any one of claims 1 to 9, nucleic acid of claim 10, conjugate of claim 11, or pharmaceutical composition of claim 12, for use in a method of treating a disease selected from the group consisting of cancer, diabetes, cardiovascular diseases, autoimmune diseases, arthritis, and neurodegenerative disorders.

15. A method of inhibiting c-Jun comprising administering a peptide according to any one of claims 1 to 9, nucleic acid according to claim 10, or conjugate according to claim 11, *in vitro,* to a cell comprising or expressing c-Jun.

## Patentansprüche

1. c-Jun-Antagonist, der Folgendes umfasst:
eine verlängerte Gelenkregion mit der Aminosäuresequenz LV[X₁]EE[X₂][X₃]LE[X₄]E (SEQ ID NO: 1); und
eine Leucin-Zipper(LZ)-Region C-terminal zur verlängerten Gelenkregion,
wobei
X₁ aus V, D, K, C und R ausgewählt ist,
X₂ aus D, K, C und R ausgewählt ist,
X₃ aus V, D, C, K und R ausgewählt ist und
X₄ aus E, D, C, K und R ausgewählt ist.

2. c-Jun-Antagonist nach Anspruch 1, wobei:
(a) die verlängerte Gelenkregion weiters eine N-terminale saure Verlängerung mit der Aminosäuresequenz EA[X₅][X₆] (SEQ ID NO: 2) aufweist,
wobei
X₅ aus E, K und C ausgewählt ist und
X₆ aus E und D ausgewählt ist;
wobei die saure Verlängerung gegebenenfalls die Aminosäuresequenz EAEE (SEQ ID NO: 3) aufweist; und/oder wobei
(b)
(i) X₁ V ist und/oder X₃ V ist,
(ii) X₁ V ist, X₂ D ist, X₃ V ist und X₄ E ist,
(iii) X₁ V ist, X₂ K ist, X₃ V ist und X₄ D ist oder
(iv) X₁ V ist, X₂ C ist, X₃ V ist und X₄ C ist.

3. c-Jun-Antagonist nach Anspruch 1 oder Anspruch 2, wobei die LZ-Region die Aminosäuresequenz IEQLEERNYALRKEIKDLQDQ (SEQ ID NO: 7) oder eine Variante davon mit 1, 2, 3, 4 oder 5 Aminosäure-Modifikationen aufweist;
wobei gegebenenfalls:
(i) die LZ-Region eine Aminosäuresequenz IEQLEERNYALRKEIKDLQDQ (SEQ ID NO: 7) oder eine Variante davon mit 1, 2 oder 3 Aminosäure-Modifikationen aufweist,
wobei gegebenenfalls in der Variante Aminosäurereste an Positionen, die der Position 16 und der Position 20 von SEQ ID NO: 7 entsprechen, die Aminosäurereste K bzw. D sind; oder
(ii) die LZ-Region die Aminosäuresequenz IEQLEERNYALRKEICDLQCQ (SEQ ID NO: 27) oder eine Variante davon mit 1, 2 oder 3 Aminosäure-Modifikationen aufweist,
wobei gegebenenfalls in der Variante Aminosäurereste an Positionen, die der Position 16 (Position b im Heptad) und der Position 20 (Position *f* im Heptad) von SEQ ID NO: 27 entsprechen, beide Aminosäurereste C sind.

4. c-Jun-Antagonist nach einem der Ansprüche 1 bis 3, wobei die LZ-Region die Aminosäuresequenz IEQLEERNYALR[X₇]E[X₈]K[X₉]L[X₄₀]D[X₁₁] (SEQ ID NO: 29) oder IEQLEERNYALR[X₇]E[X₈]C[X₉]L[X₁₀]C[X₁₁] (SEQ ID NO: 30) umfasst, wobei
[X₇] K, L, S, W, P, Q, R, M, T, V, A, E oder G ist,
[X₈] I, V oder L ist,
[X₉] und [X₁₀] beliebige Aminosäurereste sind und
[X₁₁] Q, E oder K ist;
wobei die LZ-Region gegebenenfalls eine Aminosäuresequenz umfasst, die aus der aus den folgenden bestehenden Gruppe ausgewählt ist:
IEQLEERNYALRSEICSLQCQ (SEQ ID NO: 66), oder
IEQLEERNYALRKEICELSCQ (SEQ ID NO: 67), oder
IEQLEERNYALRAEICNLSCQ (SEQ ID NO: 68), oder
IEQLEERNYALRTEICSLMCK (SEQ ID NO: 69) oder
IEQLEERNYALRAEICSLQCQ (SEQ ID NO: 70),
oder eine Variante davon mit 1, 2 oder 3 Aminosäure-Modifikationen umfasst.

5. c-Jun-Antagonist nach einem der vorangegangenen Ansprüche, wobei der Antagonist die folgende Aminosäuresequenz aufweist:
(i) EAEELVVEEDVLEEEIEQLEERNYALRKEIKDLQDQ (SEQ ID NO:10) oder eine Variante davon mit 1, 2 oder 3 Aminosäure-Modifikationen, wobei gegebenenfalls in der Variante Aminosäurereste an Positionen, die der Position 31 und der Position 35 von SEQ ID NO: 10 entsprechen, die Aminosäurereste K bzw. D sind;
(ii) EAEELVVEEKVLEDEIEQLEERNYALRKEIKDLQDQ (SEQ ID NO: 11) oder eine Variante davon mit 1, 2 oder 3 Aminosäure-Modifikationen, wobei gegebenenfalls in der Variante Aminosäurereste an Positionen, die der Position 10 und der Position 14 von SEQ ID NO: 11 entsprechen, die Aminosäurereste K bzw. D sind und in der Variante Aminosäurereste an Positionen, die der Position 31 und der Position 35 von SEQ ID NO: 11 entsprechen, die Aminosäurereste K bzw. D sind;
(iii) EAEELVVEEDVLEEEIEQLEERNYALRKEICDLQCQ (SEQ ID NO: 28) oder eine Variante davon mit 1, 2 oder 3 Aminosäure-Modifikationen, wobei gegebenenfalls in der Variante Aminosäurereste an Positionen, die der Position 31 und der Position 35 von SEQ ID NO: 28 entsprechen, beide Aminosäurereste C sind;
(iv) EAEELVVEEDVLEEEIEQLEERNYALRSEICSLQCQ (SEQ ID NO: 37) oder eine Variante davon mit 1, 2 oder 3 Aminosäure-Modifikationen, wobei gegebenenfalls in der Variante Aminosäurereste an Positionen, die der Position 31 und der Position 35 von SEQ ID NO: 28 entsprechen, beide Aminosäurereste C sind; oder
(v) EAEELVVEEDVLEEEIEQLEERNYALRAEICNLSCQ (SEQ ID NO: 39) oder eine Variante davon mit 1, 2 oder 3 Aminosäure-Modifikationen, wobei gegebenenfalls in der Variante Aminosäurereste an Positionen, die der Position 31 und der Position 35 von SEQ ID NO: 28 entsprechen, beide Aminosäurereste C sind.

6. c-Jun-Antagonist nach einem der vorangegangenen Ansprüche, wobei der Antagonist:
(a) zwischen 30 und 70 Aminosäuren, zwischen 30 und 60 Aminosäuren, zwischen 30 und 50 Aminosäuren oder zwischen 30 und 40 Aminosäuren aufweist; und/oder
(b) in der Lage ist, die DNA-Bindungsaktivität von c-Jun innerhalb des 10-Fachen der Fähigkeit von HingeW (SEQ ID NO: 48) zur Hemmung der DNA-Bindungsaktivität von c-Jun zu hemmen.

7. c-Jun-Antagonist nach einem der Ansprüche 1 bis 3 und 6, wobei der Antagonist die Aminosäuresequenz LARENEELEKEAEELVVEEDVLEEEIEQLEERNYALRKEIEDLQKQLEKL (SEQ ID NO: 13) oder eine Variante davon mit 1, 2 oder 3 Aminosäure-Modifikationen umfasst oder daraus besteht,
wobei der Antagonist gegebenenfalls die Aminosäuresequenz LARENEELEKEAEELVVEEDVLEEEIEQLEERNYALRKEIEDLQKQLEKL (SEQ ID NO: 13) umfasst.

8. c-Jun-Antagonist nach einem der vorangegangenen Ansprüche, wobei der Antagonist in der D- oder der L-Form vorliegt.

9. c-Jun-Antagonist nach einem der vorangegangenen Ansprüche, der Folgendes umfasst:
(i) zumindest einen Aminosäurerest-Vernetzer und/oder
(ii) zumindest einen kovalenten *i-zu-(i+4)-* oder *i*-zu-(*i*+7)-Aminosäurerest-Vernetzer;
wobei gegebenenfalls:
(a) der Antagonist zumindest einen kovalenten *i*-zu-(*i*+4)-Aminosäurerest-Vernetzer umfasst, wobei das Peptid gegebenenfalls zwei kovalente *i*-zu-(*i*+4)-Aminosäurerest-Vernetzer umfasst; und/oder
(b) der/die kovalente(n) *i*-zu-(*i*+4)-Aminosäurerest-Vernetzer an Heptad-Positionen b-zu-f oder f-zu-c vorhanden ist/sind; und/oder
(c) der/die kovalente(n) *i*-zu-(*i*+4)-Aminosäurerest-Vernetzer (eine) K-zu-D-Lactam-Brücke(n) oder (ein) Alkylvernetzer zwischen einem oder mehreren Paaren von C-Resten ist/sind;
und wobei gegebenenfalls die zwischen zwei C-Resten gebildete Vernetzung durch 1,3-Dibrommethylbenzol (DBMB) gebildet wird.

10. Nukleinsäure, die für die Aminosäuresequenz eines c-Jun-Antagonisten nach einem der vorangegangenen Ansprüche kodiert.

11. Konjugat, das einen c-Jun-Antagonisten nach einem der Ansprüche 1 bis 9 an ein Lipid, ein Polymer oder ein zweites Peptid konjugiert umfasst,
wobei das zweite Peptid gegebenenfalls ein zellpenetrierendes Peptid ist.

12. Pharmazeutische Zusammensetzung, die einen c-Jun-Antagonisten nach einem der Ansprüche 1 bis 9 oder eine Nukleinsäure nach Anspruch 10 oder ein Konjugat nach Anspruch 11 in Kombination mit einem pharmazeutisch annehmbaren Hilfsstoff oder Träger umfasst.

13. c-Jun-Antagonist nach einem der Ansprüche 1 bis 9, Nukleinsäure nach Anspruch 10, Konjugat nach Anspruch 11 oder pharmazeutische Zusammensetzung nach Anspruch 12 zur Verwendung als Medikament.

14. c-Jun-Antagonist nach einem der Ansprüche 1 bis 9, Nukleinsäure nach Anspruch 10, Konjugat nach Anspruch 11 oder pharmazeutische Zusammensetzung nach Anspruch 12 zur Verwendung in einem Verfahren zur Behandlung einer Erkrankung, die aus der aus Krebs, Diabetes, kardiovaskulären Erkrankungen, Autoimmunerkrankungen, Arthritis und neurodegenerativen Störungen bestehenden Gruppe ausgewählt ist.

15. Verfahren zur Hemmung von c-Jun, das die In-vitro-Verabreichung eines Peptids nach einem der Ansprüche 1 bis 9, einer Nukleinsäure nach Anspruch 10, eines Konjugats nach Anspruch 11 an eine Zelle umfasst, die c-Jun umfasst oder exprimiert.

## Revendications

1. Antagoniste de c-Jun, comprenant :
une région charnière étendue présentant une séquence d'acides aminés de LV[X₁]EE[X₂][X₃]LE[X₄]E (SEQ ID NO : 1) ; et
une région de fermeture à glissière à leucine (LZ) C-terminale par rapport à la région de charnière étendue,
dans lequel
X₁ est choisi parmi V, D, K, C et R,
X₂ est choisi parmi D, K, C et R,
X₃ est choisi parmi V, D, C, K et R, et
X₄ est sélectionné parmi E, D, C, K et R.

2. Antagoniste de c-Jun selon la revendication 1, dans lequel :
(a) la région charnière étendue comprend en outre une extension acide N-terminale présentant une séquence d'acides aminés de EA[X₅][X₆] (SEQ ID NO : 2),
dans lequel
X₅ est choisi parmi E, K et C, et
X₆ est choisi parmi E et D ;
facultativement dans lequel l'extension acide présente une séquence d'acides aminés d'EAEE (SEQ ID NO : 3) ; et/ou
(b)
(i) X₁ est V, et/ou dans lequel X₃ est V ;
(ii) X₁ est V, X₂ est D, X₃ est V et X₄ est E ;
(iii) X₁ est V, X₂ est K, X₃ est V et X₄ est D ; ou
(iv) X₁ est V, X₂ est C, X₃ est V et X₄ est C.

3. Antagoniste de c-Jun selon la revendication 1 ou la revendication 2, dans lequel la région LZ comprend une séquence d'acides aminés de IEQLEERNYALRKEIKDLQDQ (SEQ ID NO : 7), ou un variant de celle-ci comprenant 1, 2, 3, 4 ou 5 modifications d'acides aminés ;
facultativement dans lequel :
(i) la région LZ comprend une séquence d'acides aminés de IEQLEERNYALRKEIKDLQDQ (SEQ ID NO : 7), ou un variant de celle-ci comprenant 1, 2 ou 3 modifications d'acides aminés,
facultativement dans lequel les résidus d'acides aminés aux positions correspondant à la position 16 et à la position 20 de SEQ ID NO : 7 dans le variant sont des résidus d'acides aminés K et D, respectivement ; ou
(ii) la région LZ comprend une séquence d'acides aminés de IEQLEERNYALRKEICDLQCQ (SEQ ID NO : 27), ou un variant de celle-ci comprenant 1, 2 ou 3 modifications d'acides aminés,
facultativement dans lequel les résidus d'acides aminés aux positions correspondant à la position 16 (position b dans l'heptade) et à la position 20 (position f dans l'heptade) de SEQ ID NO : 27 dans le variant sont tous deux des résidus d'acides aminés C.

4. Antagoniste de c-Jun selon l'une quelconque des revendications 1 à 3, dans lequel la région LZ comprend une séquence d'acides aminés de IEQLEERNYALR [X₇]E[X₈]K[X₉][X₁₀]D[X₁₁] (SEQ ID NO : 29) ou de IEQLEERNYALR [X₇]E[X₈]C[X₉]L[X₁₀]C[X₁₁] (SEQ ID NO : 30) dans lequel
[X₇] est K, L, S, W, P, Q, R, M, T, V, A, E ou G ;
[X₈] est I, V ou L ;
[X₉] et [X₁₀] sont tout résidu d'acide aminé ; et
[X₁₁] est Q, E ou K ;
facultativement dans lequel la région LZ comprend une séquence d'acides aminés choisie dans le groupe constitué de :
IEQLEERNYALRSEICSLQCQ (SEQ ID NO: 66) ; ou
IEQLEERNYALRKEICELSCQ (SEQ ID NO: 67); ou
IEQLEERNYALRAEICNLSCQ (SEQ ID NO: 68); ou
IEQLEERNYALRTEICSLMCK (SEQ ID NO: 69); ou
IEQLEERNYALRAEICSLQCQ (SEQ ID NO: 70),
ou un variant de celles-ci comprenant 1, 2 ou 3 modifications d'acides aminés.

5. Antagoniste de c-Jun selon l'une quelconque des revendications précédentes, dans lequel l'antagoniste présente la séquence d'acides aminés de :
(i) EAEELVVEEDVLEEEIEQLEERNYALRKEIKDLQDQ (SEQ ID NO :10), ou un variant de celle-ci comprenant 1, 2 ou 3 modifications d'acides aminés, facultativement dans lequel les résidus d'acides aminés aux positions correspondant à la position 31 et à la position 35 de SEQ ID NO : 10 dans le variant sont des résidus d'acides aminés K et D, respectivement ;
(ii) EAEELVVEEKVLEDEIEQLEERNYALRKEIKDLQDQ (SEQ ID NO : 11), ou un variant de celle-ci comprenant 1, 2 ou 3 modifications d'acides aminés, facultativement dans lequel les résidus d'acides aminés aux positions correspondant à la position 10 et à la position 14 de SEQ ID NO : 11 dans le variant sont des résidus d'acides aminés K et D, respectivement, et dans lequel les résidus d'acides aminés aux positions correspondant à la position 31 et à la position 35 de SEQ ID NO : 11 dans le variant sont des résidus d'acides aminés K et D, respectivement ;
(iii) EAEELVVEEDVLEEEIEQLEERNYALRKEICDLQCQ (SEQ ID NO : 28), ou un variant de celle-ci comprenant 1, 2 ou 3 modifications d'acides aminés, facultativement dans lequel les résidus d'acides aminés aux positions correspondant à la position 31 et à la position 35 de SEQ ID NO : 28 dans le variant sont tous deux des résidus d'acides aminés C ;
(iv) EAEELVVEEDVLEEEIEQLEERNYALRSEICSLQCQ (SEQ ID NO : 37), ou un variant de celle-ci comprenant 1, 2 ou 3 modifications d'acides aminés, facultativement dans lequel les résidus d'acides aminés aux positions correspondant à la position 31 et à la position 35 de SEQ ID NO : 37 dans le variant sont tous deux des résidus d'acides aminés C ; ou
(v) EAEELVVEEDVLEEEIEQLEERNYALRAEICNLSCQ (SEQ ID NO : 39), ou un variant de celle-ci comprenant 1, 2 ou 3 modifications d'acides aminés, facultativement dans lequel les résidus d'acides aminés aux positions correspondant à la position 31 et à la position 35 de SEQ ID NO : 39 dans le variant sont tous deux des résidus d'acides aminés C.

6. Antagoniste de c-Jun selon l'une quelconque des revendications précédentes, dans lequel l'antagoniste :
(a) présente entre 30 et 70 acides aminés, entre 30 et 60 acides aminés, entre 30 et 50 acides aminés, ou entre 30 et 40 acides aminés ; et/ou
(b) est capable d'inhiber l'activité de liaison à l'ADN de c-Jun dans les 10 fois la capacité de HingeW (SEQ ID NO : 48) à inhiber l'activité de liaison à l'ADN de c-Jun.

7. Antagoniste de c-Jun selon l'une quelconque des revendications 1 à 3 et 6, dans lequel l'antagoniste comprend ou consiste en la séquence d'acides aminés de LARENEELEKEAEELVVEEDVLEEEIEQLEERNYALRKEIEDLQKQLEKL (SEQ ID NO : 13), ou un variant de celle-ci comprenant 1, 2 ou 3 modifications d'acides aminés,
facultativement dans lequel l'antagoniste comprend la séquence d'acides aminés de LARENEELEKEAEELVVEEDVLEEEIEQLEERNYALRKEIEDLQKQLEKL (SEQ ID NO : 13).

8. Antagoniste de c-Jun selon l'une quelconque des revendications précédentes, dans lequel l'antagoniste est sous la forme D- ou L-.

9. Antagoniste de c-Jun selon l'une quelconque des revendications précédentes, comprenant :
(i) au moins un réticulant de résidu d'acide aminé ; et/ou
(ii) au moins un agent de réticulation de résidu d'acide aminé covalent *i* à *i* +4 ou *i* à *i+7,*
facultativement dans lequel :
(a) l'antagoniste comprend au moins un agent de réticulation de résidu d'acide aminé covalent *i* à *i+4,* facultativement dans lequel le peptide comprend deux agents de réticulation de résidu d'acide aminé covalent *i à i+4 ;* et/ou
(b) le(s) agent(s) de réticulation d'acides aminés covalent *i à i+4* sont présents aux emplacements heptadiques b-à-f ou *f-à-c* ; et/ou
(c) le(s) agent(s) de réticulation du ou des résidus d'acides aminés covalents *i à i+4* sont un ou des ponts lactame K à D ou une ou des liaisons alkyle réticulées formées entre une ou plusieurs paires de résidus C ;
et facultativement dans lequel la réticulation alkyle formée entre deux résidus C est formée par du 1,3 dibromométhylbenzène (DBMB).

10. Acide nucléique codant pour la séquence d'acides aminés de l'antagoniste de c-Jun selon l'une quelconque des revendications précédentes.

11. Conjugué comprenant l'antagoniste de c-Jun selon l'une quelconque des revendications 1 à 9 conjugué à un lipide, un polymère ou un second peptide,
facultativement dans lequel le second peptide est un peptide pénétrant dans les cellules.

12. Composition pharmaceutique comprenant l'antagoniste de c-Jun selon l'une quelconque des revendications 1 à 9, ou l'acide nucléique selon la revendication 10, ou le conjugué selon la revendication 11, en combinaison avec un excipient ou support pharmaceutiquement acceptable.

13. Antagoniste de c-Jun selon l'une quelconque des revendications 1 à 9, acide nucléique selon la revendication 10, conjugué selon la revendication 11 ou composition pharmaceutique selon la revendication 12, à utiliser comme médicament.

14. Antagoniste de c-Jun selon l'une quelconque des revendications 1 à 9, acide nucléique selon la revendication 10, conjugué selon la revendication 11 ou composition pharmaceutique selon la revendication 12, à utiliser dans un procédé de traitement d'une maladie choisie dans le groupe constitué par le cancer, le diabète, les maladies cardiovasculaires, les maladies auto-immunes, l'arthrite et les troubles neurodégénératifs.

15. Procédé d'inhibition de c-Jun comprenant l'administration d'un peptide selon l'une quelconque des revendications 1 à 9, d'un acide nucléique selon la revendication 10 ou d'un conjugué selon la revendication 11, in vitro, à une cellule comprenant ou exprimant c-Jun.
